# DEMANDE DE BREVET EUROPEEN

(11) **EP 2 348 107 A2**
(43) Date de publication de la demande: **27.07.2011**
(21) Numéro de dépôt: 10183261.6
(22) Date de dépôt: 17.02.2004
(51) Int. Cl.: C12N 15/01, C12N 15/10, C12N 9/02, C12P 7/18, C12P 7/20, C12P 7/42, C12P 7/52, C12P 13/12, C12P 33/00

(54) **Procédé de préparation de microorganismes évolués permettant la création ou la modification de voies métaboliques**

(30) Priorité: 06.11.2003 FR 0313054; 18.02.2003 FR 0301924; 14.05.2003 FR 0305768; 14.05.2003 FR 0305769
(62) Demande divisionnaire de: 04711626.4
(71) Demandeur: Metabolic Explorer, 63360 Saint Beauzire (FR)
(72) Inventeur: Chateau, Michel, 63200 Riom (FR); Gonzalez, Benjamin, 63200 Riom (FR); Meynial-Salles, Isabelle, 31450 Fourquevaux (FR); Soucaille, Philippe Noel Paul, 31450 Deyme (FR); Zink, Olivier, 68100 Mulhouse (FR)
(74) Mandataire: Tetaz, Franck Claude Edouard

(57) **Abrégé**

La présente invention concerne un procédé de préparation de microorganismes évolués, permettant une modification des voies métaboliques consommatrices de NADPH, caractérisé en ce qu'il comprend les étapes suivantes :
a) Modification génétique des cellules d'un microorganisme initial de telle sorte que la vitesse de production du NADPH soit supérieure à sa vitesse d'oxydation en NADP, ce microorganisme comprenant un ou plusieurs gènes codant pour des enzymes NADPH dépendantes impliquées dans la biotransformation d'une molécule d'intérêt,
b) Culture des microorganismes modifiés précédemment obtenus sur un milieu défini pour faire évoluer ce ou ces gènes codant pour des enzymes NADPH dépendantes impliquées dans la biotransformation d'une molécule d'intérêt,
c) Sélection des cellules de microorganismes modifiés capables de se développer sur le milieu défini.

L'invention se rapporte également à un microorganisme évolué susceptible d'être obtenu par le procédé décrit ci-dessus, ainsi qu'à son utilisation dans un procédé de biotransformation consommateur de NADPH.

## Description

La présente invention concerne un nouveau procédé de préparation de microorganismes évolués permettant la création ou la modification de voies métaboliques, les souches de microorganismes évolués ainsi obtenus, les gènes évolués codant pour des protéines évoluées susceptibles d'être obtenues par le procédé selon l'invention et l'utilisation desdits microorganismes, gènes ou protéines évolués dans un procédé de biotransformation.

La préparation de microorganismes à propriétés modifiées est un processus largement répandu. Il s'agit soit de faire évoluer des microorganismes en les faisant croître sur un milieu de croissance avec un élément de pression de sélection, de manière à sélectionner les microorganismes capables de résister à cette pression, soit à introduire un ou plusieurs gènes hétérologues par des techniques aujourd'hui largement répandues de génie génétique, afin de conférer aux microorganismes des nouveaux caractères phénotypiques associés à l'expression du ou desdits gènes hétérologues. L'évolution peut être favorisée par l'utilisation d'agents mutagènes bien connus de l'homme du métier.

Des techniques d'évolution par croissance sous pression de sélection en supprimant un gène nécessaire à la transformation d'un composant présent dans le milieu de culture et au moyen d'un agent mutagène sont décrites notamment dans FR 2 823 219 ou WO 03/004656.

### Brève description de l'invention

La présente invention concerne un nouveau procédé de préparation de microorganismes évolués, permettant une modification des voies métaboliques, caractérisé en ce qu'il comprend les étapes suivantes :
a) Modification des cellules d'un microorganisme initial de manière à inhiber la production ou la consommation d'un métabolite lorsque le microorganisme est cultivé sur un milieu défini, affectant ainsi la capacité de croissance du microorganisme. En l'absence de modification des cellules, le microorganisme est capable de produire ou de consommer ce métabolite et présente une croissance normale lorsque cultivé sur ce même milieu défini.
b) Culture des microorganismes modifiés précédemment obtenus sur ledit milieu défini pour le faire évoluer, le milieu défini pouvant également comprendre un co-substrat nécessaire à cette évolution,
c) Sélection des cellules de microorganismes modifiés capables de se développer sur le milieu défini, éventuellement avec un co-substrat.

Le microorganisme évolué comprend préférentiellement au moins un gène évolué, codant pour une protéine évoluée, dont l'évolution permet de remplacer la voie métabolique inhibée par une nouvelle voie métabolique.

La présente invention concerne également un procédé comprenant une étape supplémentaire a1) d'introduction d'au moins un gène hétérologue codant pour une protéine hétérologue, ledit gène hétérologue étant destiné à faire évoluer une nouvelle voie métabolique, préalable à l'étape b) de culture des microorganismes modifiés.

La présente invention concerne aussi un procédé comprenant une étape d) d'isolation du gène évolué codant pour ladite protéine évoluée.

La présente invention concerne aussi un procédé selon l'invention dans lequel on introduit le gène évolué précédemment obtenu, sous une forme appropriée, dans un microorganisme de production destiné à la production de protéine évoluée.

L'invention concerne également un microorganisme évolué susceptible d'être obtenu par un procédé selon l'invention tel que défini ci-dessus et ci-après.

L'invention concerne aussi un procédé de préparation d'une protéine évoluée, caractérisé en ce que l'on cultive un microorganisme évolué selon l'invention dans un milieu de culture approprié pour la production de la protéine évoluée, la protéine évoluée produite étant éventuellement purifiée.

L'invention concerne aussi un gène évolué codant pour une protéine évoluée susceptible d'être obtenu par un procédé selon l'invention tel que défini ci-dessus et ci-après.

L'invention concerne également une protéine évoluée susceptible d'être obtenue par un procédé selon l'invention tel que défini ci-dessus et ci-après.

L'invention concerne également l'utilisation d'un microorganisme évolué ou une protéine évoluée tels que définis ci-dessus et ci-après dans un procédé de biotransformation.

### Définitions

Par « microorganisme évolué », on entend selon l'invention un microorganisme obtenu par sélection d'un microorganisme modifié. Le microorganisme évolué présente au moins une différence par rapport au microorganisme modifié, cette différence correspond par exemple à une amélioration d'une caractéristique enzymatique ou encore à la création d'une nouvelle voie métabolique.

Par « voie métabolique », on entend selon l'invention une ou plusieurs réactions enzymatiques dont la succession permet de produire une molécule (produit) différente de la molécule initiale (substrat).

Par « modification », on entend selon l'invention une altération, en particulier une délétion, d'au moins un gène et/ou de sa séquence promotrice, le gène codant pour une enzyme.

Par « métabolite », on entend selon l'invention une molécule synthétisée et/ou transformée par le microorganisme.

Par « milieu défini », on entend selon l'invention un milieu de composition moléculaire connue, adapté à la croissance du microorganisme. Le milieu défini est substantiellement exempt du ou des métabolites dont on inhibe la production en réalisant la modification.

Par « co-substrat », on entend selon l'invention une molécule carbonée ou non, différente du substrat, qui est impliqué dans une réaction et donnant un ou plusieurs atomes au substrat afin de former le produit. Le co-substrat n'a pas de propriété mutagène reconnue.

Par « sélection », on entend selon l'invention un procédé de culture permettant de sélectionner les microorganismes ayant évolué de telle sorte que la modification n'affecte plus la croissance. Une application préférée est un procédé de culture en continu, conduit en appliquant des taux de dilution croissants de telle sorte de ne conserver dans le milieu de culture que les microorganismes ayant un taux de croissance égal ou supérieur au taux de dilution imposé.

Par « gène évolué », on entend selon l'invention une succession d'acides nucléiques (pris parmi A,T,G ou C) délimité par un codon stop (TAA, TAG, TGA) en phase et ayant, à l'issue de la sélection, au moins un acide nucléique différent par rapport à la séquence initiale de telle sorte que la protéine codée par ce gène évolué présente au moins un acide aminé différent par rapport à la protéine codée par le gène initial.

Par « gène hétérologue », on entend selon l'invention une succession d'acides nucléiques, délimitée par un codon start (ATG ou GTG) et un codon stop (TAA, TAG, TGA) en phase, dénommée séquence codante et issue d'un organisme différent de celui utilisé pour réaliser l'évolution et/ou la production.

Par « protéine évoluée », on entend selon l'invention une succession d'acides aminés (séquence protéique) ayant, à l'issue de la sélection, au moins un acide aminé différent par rapport à la séquence protéique initiale.

Par « protéine hétérologue », on entend selon l'invention une protéine issue de la traduction d'un gène hétérologue.

Les gènes et protéines peuvent être identifiées par leurs séquences primaires, mais également par homologies de séquences ou alignements qui définissent des groupes de protéines.

Les PFAM (Protein families database of alignments and Hidden Markov Models ; http://www.sanger.ac.uk/Software/Pfaml) représentent une large collection d'alignements de sequences protéiques. Chaque PFAM permet de visualiser des alignements multiples, de voir des domaines protéiques, d'évaluer la répartition entre les organismes, d'avoir accès à d'autres bases de données, de visualiser des structures connues de protéines.

Les COGs (Clusters of Orthologous Groups of proteins ; http://www.ncbi.nlm.nih.gov/COG/) sont obtenus en comparant les séquences protéiques issus de 43 génomes complètement séquencés représentant 30 lignées phylogénétiques majeurs. Chaque COG est défini à partir d'au moins trois lignées ce qui permet ainsi d'identifier des domaines conservés anciens.

Les moyens d'identification des séquences homologues et de leurs pourcentages d'homologie sont bien connus de l'homme du métier, comprenant notamment les programmes BLAST qui peuvent être utilisé à partir du site http://www.ncbi.n1m.nih.gov/BLAST/avec les paramètres indiqués par défaut sur ce site. Les séquences obtenues peuvent alors être exploitées (e.g. alignées) en utilisant par exemple les programmes CLUSTALW (http://www.ebi.ac.uk/clustalw/) ou MULTALIN (http://prodes.toulouse.inra.fr/multalin/cgi-bin/multalin.pl), avec les paramètres indiqués par défaut sur ces sites.

En utilisant les références données sur GenBank pour les gènes qui sont connus, l'homme du métier est capable de déterminer les gènes équivalents dans d'autres organismes, souches bactériennes, levures, champignons, mammifères, plantes, etc. Ce travail de routine est avantageusement effectué en utilisant les séquences consensus pouvant être déterminées en réalisant des alignements de séquences avec des gènes issus d'autres microorganismes, et en dessinant des sondes dégénérées permettant de cloner le gène correspondant dans un autre organisme. Ces techniques de routine de biologie moléculaire sont bien connues dans l'art et sont décrites par exemple dans Sambrook et al. (1989 Molecular cloning : a laboratory manual. 2nd Ed. Cold Spring Harbor Lab., Cold Spring Harbor, New York.).

Les gènes susceptibles d'être délétés ou surexprimés pour les souches évoluées selon l'invention sont définis principalement par l'emploi de la dénomination du gène de *E. coli.* Cependant, cet emploi a une signification plus générale selon l'invention et englobe les gènes correspondants d'autres microorganismes. En effet en utilisant les références GenBank des gènes d'*E*. *coli,* l'homme du métier est capable de déterminer les gènes équivalents dans d'autres souches bactériennes qu'*E*. *coli.*

Par « nouvelle voie métabolique », on entend selon l'invention une ou plusieurs réactions enzymatiques, dont la succession permet de produire une molécule, qui, après l'étape de sélection du microorganisme évolué, se révèlent différentes des activités enzymatiques du microorganisme non évolué. La différence pouvant résider dans le type de réaction catalysée ou bien dans ses caractéristiques cinétiques (Km, Vmax, Ki, ...). Une nouvelle voie enzymatique permet de produire une molécule, différente ou non du produit initial, à partir d'un substrat différent ou non du substrat initial.

Par « forme appropriée », on entend selon l'invention une succession d'acide nucléique, délimitée par un codon start (ATG ou GTG) et un codon stop (TAA, TAG, TGA) en phase, dénommée séquence codante, ou une partie de cette séquence codante, sous le contrôle d'éléments de régulation nécessaires à son expression dans le microorganisme dans lequel le gène hétérologue sera exprimé. Ces éléments de régulations sont bien connus de l'homme du métier, et comprennent des séquences de régulation promotrice, ou promoteurs, en particulier des promoteurs dits promoteurs forts constitutifs chez les microorganismes. De préférence, le promoteur constitutif est choisi parmi pTAC-O, pLAC-O, pTRC-O, des promoteurs forts pour lesquels l'opérateur lac a été délété pour les rendre constitutifs, pTHLA.

Par « microorganisme initial » on entend un microorganisme avant réalisation de toute modification, mutation ou évolution.

Par « microorganisme de production », on entend selon l'invention un microorganisme évolué ou un microorganisme optimisé dans le lequel a été introduit la nouvelle voie métabolique issue d'un microorganisme évolué.

Par « microorganisme modifié » on entend un microorganisme obtenu après réalisation de modifications maîtrisées par l'homme et ne résultant pas d'un processus d'évolution. On citera à titre d'exemple de modification, la mutation dirigée ou la délétion d'un gène, la modification dirigée d'un promoteur.

Par « milieu de culture approprié pour la production de la protéine évoluée », on entend selon l'invention soit un milieu de composition défini, soit un milieu complexe, soit un milieu partiellement défini. Le milieu complexe est réalisé à partir soit d'un hydrolysat végétal, de microorganisme ou animal ; sa composition peut-être connue en réalisant des analyses, il est cependant rare de pouvoir réaliser une analyse exhaustive de ce type de milieu. Un milieu partiellement défini est un milieu défini auquel on rajoute un milieu complexe.

Par « procédé de biotransformation » on entend selon l'invention un procédé de transformation d'une molécule A en une molécule B en utilisant une ou plusieurs enzymes, contenues ou non dans un ou plusieurs microorganismes. On distingue trois types de procédés de biotransformation : la bioconversion, la fermentation et la biocatalyse. Dans le cas de la bioconversion, la ou les enzymes sont contenues dans un ou plusieurs microorganismes, ceux-ci sont cultivés sur un milieu approprié puis la molécule A et éventuellement un ou plusieurs co-substrats sont apportés pour être transformés en B. Dans le cas de la fermentation, la ou les enzymes sont contenues dans un ou plusieurs microorganismes,-ceux-ci sont cultivés sur un milieu approprié permettant au(x) microorganisme de synthétiser la molécule A ; le milieu approprié peut contenir des co-substrats. Dans le cas de la biocatalyse, la ou les enzymes ne sont pas contenues dans des cellules mais sont entourées par un milieu adéquat apportant la molécule A ainsi que les co-substrats nécessaires à la biotransformation.

Les méthodes d'isolation de gènes sont bien connues de l'homme du métier, décrites notamment dans Sambrook et al.(1989 Molecular cloning : a laboratory manual. 2nd Ed. Cold Spring Harbor Lab., Cold Spring Harbor, New York), Ausubel et al., 1987 (Current Protocols in Molecular Biology, John Willey and Sons, New York); Maniatis et al., 1982, (Molecular Cloning : a laboratory manual. Cold Spring Harbor Laboratory, Cold Sring Harbor, New York). Ces méthodes permettent de localiser, de copier ou d'extraire le gène, afin de l'introduire dans un nouvel organisme. Cette dernière étape pouvant être précédée d'une étape d'incorporation du gène dans un polynucléotides préalablement à son introduction dans le microorganisme.

Les méthodes de purification d'une protéine sont bien connues de l'homme du métier, décrites notamment dans Coligan et al., 1997 (Current Protocols in Protein Science, John Wiley & Sons, Inc). Elles permettent d'identifier la protéine recherchée dans un extrait protéique, fractionné ou non. La protéine peut ensuite être purifiée, ce qui se traduit par une augmentation de son activité spécifique, dans le cas particulier d'une enzyme. Enfin ces techniques permettent éventuellement d'immobiliser la protéine sur un support (e.g. résine).

Par « délétion », on entend selon l'invention une suppression de l'activité du gène « délété ». Cette suppression peut être une inactivation du produit d'expression du gène concerné par un moyen approprié, ou bien l'inhibition de l'expression du gène concerné, ou encore la délétion d'au moins une partie du gène concerné de manière soit que son expression n'ait pas lieu (par exemple délétion d'une partie ou de l'ensemble de la région promotrice nécessaire à son expression) soit que le produit d'expression ait perdu sa fonction (par exemple délétion dans la partie codante du gène concerné). De manière préférentielle, la délétion d'un gène comprend la suppression de l'essentiel dudit gène, et le cas échéant son remplacement par un gène marqueur de sélection permettant de faciliter l'identification, l'isolement et la purification des souches évoluées selon l'invention.

Par « substrat » on entend un métabolite qui peut être transformé par l'action d'une enzyme, éventuellement en présence d'un co-substrat.

### Description détaillée de l'invention

### A. Microorganismes modifiés

Les souches de microorganismes modifiés selon l'invention peuvent être procaryotiques ou eucaryotiques.

Par souche de microorganismes, on entend selon l'invention un ensemble de microorganismes d'une même espèce comprenant au moins un microorganisme de ladite espèce. Ainsi, les caractéristiques décrites pour la souche s'appliquent à chacun des microorganismes de ladite souche. De même, les caractéristiques décrites pour l'un des microorganismes de la souche s'appliqueront à l'ensemble desdits microorganismes la composant.

Les microorganismes optimisés selon l'invention sont choisis parmi les bactéries, les levures et les champignons, et notamment ceux des espèces suivantes : *Aspergillus sp., Bacillus sp., Brevibacterium sp., Clostridium sp., Corynebacterium sp., Escherichia sp., Gluconobacter sp., Pseudomonas sp., Rhodococcus sp., Saccharomyces sp., Streptomyces sp., Xanthomonas sp., Candida sp.*

Dans un mode de réalisation préféré, la souche bactérienne est une souche *d'Escherichia,* en particulier d'*E*. *coli.* Dans un autre mode de réalisation, la souche bactérienne est une souche de *Corynebacterium,* en particulier *C. glutamicum.*

Dans un autre mode de réalisation, la souche de levure est une souche de *Saccharomyces,* en particulier *S. cerevisiae*

Pour préparer lesdit microorganismes modifiés, il peut être avantageux d'atténuer, en particulier de déléter, d'autres gènes associés ou non à la voie métabolique à modifier afin de favoriser l'évolution du microorganisme.

Pour préparer lesdit microorganismes modifiés, il peut être également avantageux de favoriser, en particulier de surexprimer, d'autres gènes, hétérologues ou non, associés ou non à la voie métabolique à modifier afin de favoriser l'évolution du microorganisme.

La surexpression d'un gène peut être effectuée par changement du promoteur de ce gène *in situ,* par un promoteur fort ou inductible. De façon alternative, on introduit, dans la cellule, un plasmide réplicatif (simple ou multicopies) dans lequel le gène que l'on désire surexprimer est sous le contrôle du promoteur adéquat.

De telles modifications seront décidées au cas par cas selon le choix de la voie métabolique à modifier. Elles sont notamment décrites au cas par cas pour les voies métaboliques particulières exposées ci-après.

L'homme du métier connaît les protocoles permettant de modifier le caractère génétique de microorganismes.

L'inactivation d'un gène se fait préférentiellement par recombinaison homologue. (Datsenko, K.A. ; Wanner, B.L. (2000) One-step inactivation of chromosomal genes in Escherichia coli K-12 using PCR products. Proc. Natl. Acad. Sci. USA 97: 6640-6645). Le principe d'un protocole en est rappelé brièvement : on introduit dans la cellule un fragment linéaire, obtenu *in vitro,* comprenant les deux régions flanquant le gène, et au moins un gène de sélection entre ces deux régions (généralement un gène de résistance à un antibiotique), ledit fragment linéaire présentant donc un gène inactivé. On sélectionne les cellules ayant subi un événement de recombinaison et ayant intégré le fragment introduit par étalement sur milieu sélectif. On sélectionne ensuite les cellules ayant subi un événement de double recombinaison, dans lesquelles le gène natif a été remplacé par le gène inactivé. Ce protocole peut être amélioré en utilisant des systèmes de sélections positive et négative, afin d'accélérer la détection des événements de double recombinaisons.

L'inactivation d'un gène chez S. *cerevisiae* se fait préférentiellement par recombinaison homologue (Baudin et al., Nucl. Acids Res. 21, 3329-3330, 1993; Wach et al., Yeast 10, 1793-1808, 1994; Brachmann et al., Yeast. 14 :115-32, 1998).

### B. Microorganismes de production

Les microorganismes de productions sont choisis également parmi les bactéries, les levures et les champignons définis précédemment. Il peut s'agir des microorganismes évolués obtenus par le procédé d'évolution selon l'invention, ou encore de microorganismes optimisés pour la production du métabolite recherché, dans lesquels on aura introduit au moins un gène évolué selon l'invention.

### C. Culture des Microorganismes

Selon l'invention, les termes « culture » et « fermentation » sont employés indifféremment pour désigner la croissance du microorganisme sur un milieu de culture approprié comprenant une source de carbone simple.

Par source de carbone simple, selon la présente invention, on entend des sources de carbone utilisables par l'homme du métier pour la croissance normale d'un microorganisme, d'une bactérie en particulier. On entend désigner notamment les différents sucres assimilables, tels le glucose, le galactose, le saccharose, le lactose ou les mélasses, ou les sous-produits de ces sucres. Une source de carbone simple tout particulièrement préférée est le glucose. Une autre source de carbone simple préférée est le saccharose.

La définition des conditions de culture des microorganismes selon l'invention est à la portée de l'homme du métier. On fermente notamment les bactéries à une température comprise entre 20°C et 55°C, de préférence entre 25°C et 40°C, plus particulièrement d'environ 30°C pour *C. glutamicum* et d'environ 37°C pour *E. coli.*

La fermentation est généralement conduite en fermenteurs avec un milieu minéral de culture de composition connu défini et adapté en fonction des bactéries utilisées, contenant au moins une source de carbone simple et le cas échéant un co-substrat nécessaire à la production du métabolite.

En particulier, le milieu minéral de culture pour *E. coli* pourra ainsi être de composition identique ou similaire à un milieu M9 (Anderson, 1946, Proc. Natl. Acad. Sci. USA 32:120-128), un milieu M63 (Miller, 1992; A Short Course in Bacterial Genetics: A Laboratory Manual and Handbook for Escherichia coli and Related Bacteria, Cold Spring Harbor Laboratory Press, Cold Spring Harbor, New York) ou un milieu tel que défini par Schaefer et al. (1999, Anal. Biochem. 270 : 88-96).

De manière analogue, le milieu minéral de culture pour C. *glutamicum* pourra ainsi être de composition identique ou similaire au milieu BMCG (Liebl et al., 1989, Appl. Microbiol. Biotechnol. 32: 205-210) à un milieu tel que défini par Riedel et al. (2001, J. Mol. Microbiol. Biotechnol. 3: 573-583).

### D. Voies Métaboliques

Les voies métaboliques destinées à être évoluées sont généralement choisies parmi les voies de biosynthèse des acides aminés, les voies de synthèse des acides nucléiques, les voies de synthèse des lipides, les voies du métabolisme des sucres.

Selon un premier mode préférentiel de réalisation de l'invention, la voie métabolique évoluée est une voie de biosynthèse des acides aminés, en particulier une voie de biosynthèse d'un acide aminé choisi parmi la méthionine, la cystéine, la thréonine, la lysine, ou l'isoleucine.

Selon un deuxième mode préférentiel de réalisation de l'invention, la voie métabolique modifiée est une voie permettant la régénération du NADP à partir du NADPH. On citera en particulier la voie de biosynthèse de la cystéine, de l'hydroxypropionate et de biosynthèse du xylitol.

### D.I. Voie de biosynthèse de la méthionine

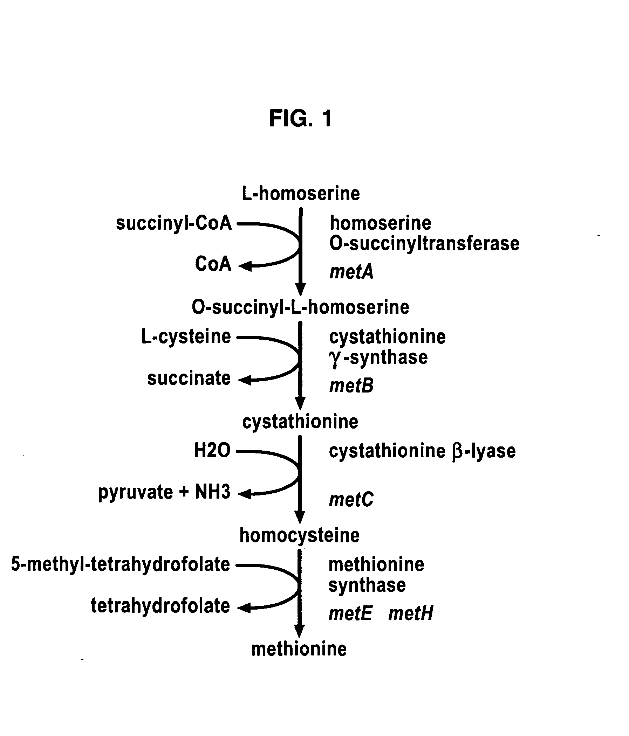

L'application de l'invention peut se faire par exemple sur la voie de biosynthèse de la méthionine (figure 1) et permet d'obtenir des souches de microorganismes, en particulier des bactéries, notamment *E. coli* et les corynébactéries, produisant de l'acide 2-Amino-4-(alkylmercapto)butyrique, en particulier de la L-méthionine (acide 2-Amino-4-(méthylmercapto)butyrique) par métabolisme d'une source de carbone simple et d'une source de soufre, en particulier le methylmercaptan (CH3SH), le sulfure d'hydrogène (H2S) ou leurs sels. La source de soufre H2S peut aussi être introduite dans le milieu de culture sous la forme de sulfate. L'invention concerne également la souche de microorganisme, les enzymes améliorées et à leurs séquences codantes. L'invention concerne enfin un procédé de préparation de la méthionine par culture de ladite souche de microorganisme.

La DL-Methionine est produite industriellement par synthèse chimique. Le méthyl-mercaptan réagit avec l'acroléine pour produire le β-méthyl thiopropionaldéhyde, qui réagit avec l'hydrogène cyanide pour produire l'α-hydroxy-γ-méthyl-thio-butyro-nitrile. Après traitement avec l'ammoniac, et une hydrolyse, on obtient la méthionine.

Tous les producteurs industriels de la DL-methionine utilisent les mêmes matières premières à savoir l'acroléine, le méthane thiol (méthyl-mercaptan), l'hydrogène cyanide et l'ammoniac ou l'ammonium carbonate. Le procédé de synthèse du mélange racémique peut être conduit en batch ou en continu.

Un procédé industriel combine à la synthèse chimique de la biocatalyse par l'utilisant l'amino acylase, enzyme produite par *Aspergillus oryzas* pour obtenir uniquement la L-méthionine à partir de la DL-méthionine.

Les brevets US 6,379,934 et EP 1 055 730 se rapportent à la production d'acides aminés en utilisant une souche de bactéries corynéformes, dans laquelle le gène *accBC* est amplifié. La méthionine est mentionnée, mais seule la préparation de L-lysine est exemplifiée.

Toutefois, la synthèse d'acides aminés soufrés en culture de microorganisme reste difficile à mettre en oeuvre à des niveaux susceptibles de conduire à une exploitation industrielle, notamment du fait de la complexité de leurs voies de biosynthèse et de nombreux mécanismes de régulation.

En effet, le métabolisme de la méthionine, est fortement régulé, la régulation de son métabolisme se faisant à plusieurs niveaux (Weissbach et al., 1991, Mol. Microbiol., 5, 1593-1597) :
- métabolisme carboné pour la fabrication de la L-sérine à partir du glycerate3P, de la L-homosérine à partir de l'aspartate et de l'acétyl-CoA
- métabolisme du soufre pour la fabrication de la L-cystéine à partir de la L-sérine, de l'acétyl-CoA et du sulfate du milieu de culture
- synthèse de la méthionine (Fig. 1) à partir de la L-homosérine, de la cystéine et d'acétyl-CoA ou succinyl-CoA.

La demande WO 93/17112 décrit les différentes enzymes impliquées dans la biosynthèse de la méthionine à partir de l'acide L-aspartique dans divers organismes. Cette demande de brevet décrit également l'introduction dans un microorganisme de plusieurs gènes exogènes agissant de manière séquentielle pour la synthèse de la méthionine, employant du méthyl-mercaptan ou du sulfure d'hydrogène comme source de soufre.

La présente invention se rapporte à des souches de microorganismes en particulier des bactéries, notamment *E. coli* et les corynébactéries, produisant de l'acide 2-amino-4-(alkylmercapto)butyrique de formule générale (I)

R-S-(CH₂)₂-CHNH₂-COOH (I)

dans laquelle R représente un radical alkyle linéaire ou ramifié comprenant de 1 à 18 atomes de carbones, éventuellement substitué par un ou des groupe(s) hydroxy, ou un radical aryle ou hétéroaryle comprenant un ou plusieurs atomes d'azote ou de soufre dans le cycle hétéroaromatique, sélectionné parmi les groupes phényle, pyridyle, pyrolyle, pyrazolyle, triazolyle, tetrazolyle, thiazolyle, ou thienyle,
par métabolisme d'une source de carbone simple et d'une source de soufre comprenant un composé de formule générale (II) :

R'-SH (II)

dans laquelle R' représente un atome d'hydrogène ou R, R étant défini précédemment, et ses sels physiologiquement acceptables,
les dites souches présentant au moins un gène codant pour une enzyme à activité « méthionine synthase » modifiée.

Par enzyme à activité «méthionine synthase » modifiée, on entend selon l'invention toute enzyme impliquée dans la biosynthèse des acides 2-amino-4-(alkylmercapto)butyriques de formule générale (I) par conversion d'un substrat dérivé de formule générale (III)

R"-O-(CH₂)₂-CHNH₂-COOH (III)

dans laquelle R" représente un radical acyle de préférence choisi parmi le radical succinyle ou le radical acétyle,
soit par conversion directe du substrat en acide de formule générale (I),
soit par conversion du substrat en homocystéine de formule générale (IV)

HS-(CH₂)₂-CHNH2-COOH (IV)

laquelle est ensuite transformée en acide de formule générale (I) par une enzyme appropriée.

Les enzymes à activité « méthionine synthase » modifiée sont des enzymes modifiées par rapport aux enzymes natives pour leur permettre de réaliser de manière préférentielle la conversion directe du substrat de formule générale (III) en acide de formule générale (I) ou en homocystéine de formule générale (IV) au lieu de la réaction catalysée par l'enzyme native. Cette modification, appelée encore mutation, consiste essentiellement en ce que l'enzyme modifiée ait une plus grande affinité pour le composé soufré de formule générale (II) que pour son co-substrat naturel.

Par source de carbone simple, selon la présente invention, on entend des sources de carbone utilisables par l'homme du métier pour la croissance normale d'un microorganisme, d'une bactérie en particulier. On entend désigner notamment les différents sucres assimilables, tels le glucose, le galactose, le saccharose, le lactose ou les mélasses, ou les sous-produits de ces sucres. Une source de carbone simple tout particulièrement préférée est le glucose. Une autre source de carbone simple préférée est le saccharose.

Par sel physiologiquement acceptable, on entend selon l'invention les sels du composé de formule générale (I) qui n'affectent pas le métabolisme et la capacité de croissance de la souche de microorganisme selon l'invention, notamment les sels de métaux alcalins comme le sodium.

Selon un mode préférentiel de réalisation de l'invention, R représente un radical alkyle linéaire ou ramifié comprenant de 1 à 4 atomes de carbone, en particulier choisi parmi les radicaux méthyle, éthyle, n-propyle, i-propyle, n-butyle, i-butyle ou t-butyle. De manière plus préférentielle, R représente le radical méthyle.

L'acide 2-amino-4-(alkylmercapto)butyrique de formule générale (I) obtenu est de préférence la L-méthionine.

L'utilisation d'une source de carbone simple et d'un composé soufré de formule (II) pour la production d'acide de formule (I) par bioconversion présente *a priori* plusieurs avantages, notamment :
- la synthèse de l'acide (I), comme la méthionine, en une ou deux étapes à partie à partir d'O-acyl-L-homosérine, devient indépendante de la synthèse de la cystéine voire également du cycle du tétrahydrofolate ;
- les composés soufrés de formule (II), comme le méthyl-mercaptan, qui sont des matières premières généralement toxiques issues de la pétrochimie, peuvent être valorisés dans la synthèse d'acides aminés à haute valeur ajoutée.

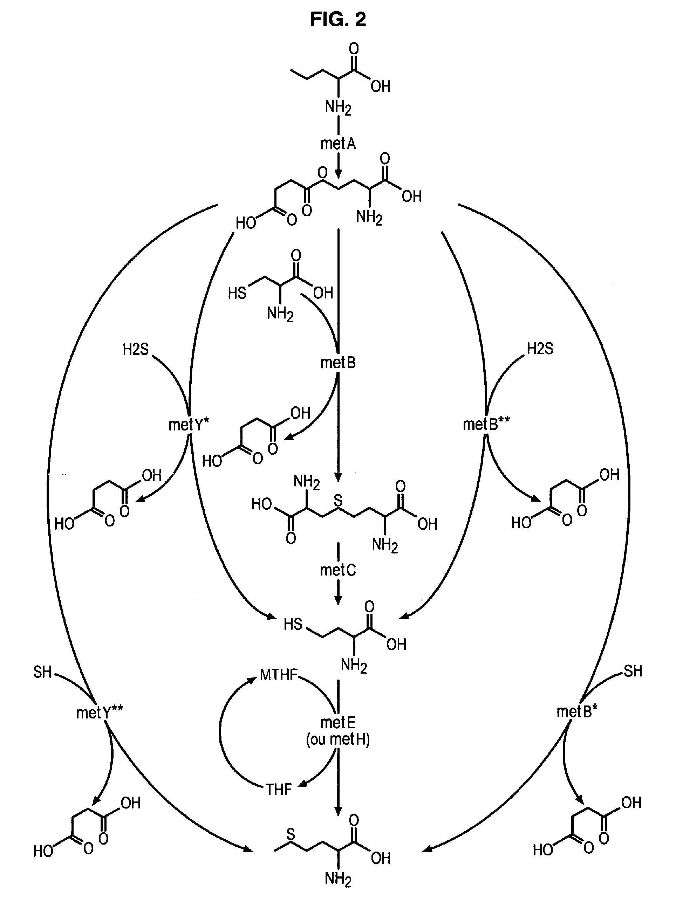

L'invention est basée sur le fait que l'on peut obtenir, de façon dirigée, une modification de l'activité « méthionine-synthase » de la cystationine-γ-synthase (EC 4.2.99.9 ; GenBank AAN83320, ou AAA24167) en présence de méthyl-mercaptan. Cette enzyme de la voie de biosynthèse de méthionine, codée par le gène *metB* chez *E. coli* (Fig. 2) et *C. glutamicum,* présente une activité pour un large spectre de substrats (Elavin, M. ; Slaughter, C. (1967) Enzymatic synthesis of homoserine or methionine directly from O-succinyl-homoserine.Biochim. Biophys. Acta 132: 400-405).

L'invention est aussi basée sur le fait que l'on peut obtenir, de façon dirigée, une modification de l'activité « méthionine-synthase » de l'O-acétyl-L-homosérine sulfhydrolase (ou O-acétyl-L-homosérine sulfhydrylase, C 4.2.99.10) en présence de méthylmercaptan. Cette enzyme de la voie de biosynthèse de méthionine, codée par le gène *metY* chez *C. glutamicum* (Genbank AF220150), présente une activité pour un large spectre de substrats (Smith IK, Thompson JF. (1969) Utilization of S-methylcysteine and methylmercaptan by methionineless mutants of Neurospora and the pathway of their conversion to methionine. II. Enzyme studies. Biochim Biophys Acta 184(1):130-8).

L'invention concerne donc également un procédé de préparation des souches selon l'invention et leur utilisation dans un procédé de préparation d'un acide 2-amino-4-(alkylmercapto)butyrique de formule générale (I), de préférence de la L-méthionine.

Le procédé de préparation de souches selon l'invention consiste à obtenir, à partir d'une souche bactérienne initiale, une souche bactérienne génétiquement modifiée présentant au moins une modification dans un gène codant pour une enzyme à activité « méthionine synthase », ledit procédé comprenant une étape consistant à soumettre ladite souche bactérienne initiale à une pression de sélection en présence du composé de formule (II) défini ci-dessus, afin de diriger une évolution dudit gène codant pour ladite enzyme à activité « méthionine synthase » dans ladite souche bactérienne, vers un gène codant pour une enzyme à activité «méthionine synthase » modifiée par rapport à ladite souche bactérienne initiale.

Une activité « méthionine synthase » est améliorée dans la souche (A) de microorganisme par rapport à la souche (I) initiale lorsque la production de méthionine dans les mêmes conditions de culture (dans un milieu contenant une quantité efficace de dérivé soufré de formule (II)) est supérieure pour la souche (A) que pour la souche (I). Cette amélioration est préférentiellement observée par étude de la quantité de méthionine produite. Dans certains cas, on peut observer cette amélioration par l'augmentation du taux de croissance de la bactérie (A) par rapport au taux de croissance de la bactérie (I), dans un milieu minimum ne contenant pas de méthionine.

La présente invention concerne également les souches à activité « méthionine synthase » améliorée susceptibles d'être obtenues par le procédé de sélection selon l'invention et comprenant au moins un gène codant pour une enzyme à activité « méthionine synthase » modifiée telle que définie précédemment et ci-après.

Selon un premier mode de réalisation de l'invention, l'enzyme à activité « méthionine synthase » modifiée permet la conversion directe du substrat de formule générale (III) en acide de formule générale (I). Dans ce cas, la source de soufre est un composé de formule générale (II) pour laquelle R' représente le radical R définit précédemment.

L'enzyme à activité « méthionine synthase » modifiée est choisie parmi les cystathionine-γ-synthases et les acylhomosérine sulfhydrylases.

Selon un deuxième mode de réalisation de l'invention, l'enzyme à activité « méthionine synthase » modifiée permet la conversion du substrat de formule générale (III) en homocystéine de formule générale (IV)

HS-(CH₂)₂-CHNH₂-COOH (IV)

laquelle est ensuite transformée en acide de formule générale (I) par une enzyme appropriée. Dans ce cas, la source de source de soufre est un composé de formule générale (II) pour laquelle R' représente un atome d'hydrogène, préférentiellement du sulfure d'hydrogène. La source de soufre H2S peut-être introduite dans le milieu de culture ou bien être produite par la bactérie à partir d'une source de soufre simple, par exemples un sulfate.

L'enzyme à activité « méthionine synthase » modifiée est avantageusement choisie parmi les cystathionine-γ-synthases et les acylhomosérine sulfhydrylases.

L'homme de métier saura identifier d'autres gènes codant des activités enzymatiques pouvant évoluer en activité « méthionine synthase » ou en « homocystéine synthase » et saura adapter en conséquence les modifications à apporter au microorganisme initial avant de le sélectionner. A titre d'exemple on citera les cystéines synthases A et B codées par les gènes *cysK* et *cysM* chez les bactéries.

Pour les cystathionine-γ-synthases modifiées définies précédemment et ci-dessous, le substrat est avantageusement la O-acétyl-L-homosérine ou la O-succinyl-L-homosérine , de préférence la O-succinyl-L-homosérine.

Pour les acylhomosérine sulfhydrylases modifiées définies précédemment et ci-dessous, le substrat est avantageusement la O-succinyl-L-homosérine ou la O-acétyl-L-homosérine, de préférence la O-acétyl-L-homosérine.

Pour ces deux enzymes, la modification consiste essentiellement en une mutation de manière à ce que la transformation du substrat de formule générale (III) se fasse de manière préférentielle avec le composé de formule générale (II) plutôt qu'avec la L-cystéine.

La mutation des enzymes peut être obtenue par la mise en oeuvre du procédé de préparation de souches à activité « méthionine synthase » améliorée selon l'invention par culture sous pression de sélection en présence du composé de formule générale (II).

Dans ce cas, le gène comprenant la séquence codant pour la cystathionine-γ-synthase ou l'acylhomosérine sulfhydrylase est
- soit un gène natif, présent dans le génome de la souche initiale (I) où il est exprimé pour permettre la traduction de l'enzyme correspondante,
- soit un gène hétérologue comprenant une séquence codant pour une cystathionine-γ-synthase ou une acylhomosérine sulfhydrylase, sous le contrôle d'éléments de régulation permettant son expression et sa traduction dans la souche initiale (I) où il aura été introduit.

La mutation peut également être obtenue par mutagénèse dirigée,
- soit directement sur le gène natif présent naturellement dans la souche initiale (I), notamment par recombinaison homologue,
- soit par des techniques usuelles de mutagenèse dirigée sur des séquences codant pour une cystathionine-γ-synthase ou une acylhomosérine sulfhydrylase, introduite ensuite dans la souche initiale (I) sous le contrôle d'éléments de régulation permettant son expression et sa traduction dans ladite souche initiale (I) où il aura été introduit.

De tels éléments de régulation sont bien connus de l'homme du métier, et comprennent des séquences de régulation promotrice, ou promoteurs, en particulier des promoteurs dits promoteurs forts constitutifs chez les microorganismes. De préférence, le promoteur fort constitutif est choisi parmi pTAC-O, pLAC-O, pTRC-O, pTHLA, promoteurs forts pour lesquels l'opérateur lac a été délété pour les rendre constitutifs.

De manière avantageuse, les cystathionine-γ-synthases « initiales » ou à activité « méthionine synthase » non modifiée sont sélectionnées parmi les cystathionine-γ-synthases correspondent au PFAM référence PF01053 et au COG référence CPG0626.

De manière préférentielle, la cystathionine-γ-synthase est la cystathionine-γ-synthase de E. coli K12, représentée sur la SEQ ID NO: 6, et les séquences homologues de cette séquence présentant une activité cystathionine-γ-synthase et comprenant au moins 80% d'homologie, préférentiellement 90% d'homologie, plus préférentiellement 95% d'homologie avec la séquence d'acides aminés de la SEQ ID NO 6.

Les moyens d'identification des séquences homologues et de leur pourcentages d'homologie sont bien connus de l'homme du métier, comprenant notamment le programme BLAST, et notamment le programme BLASTP, qui peut être utilisé à partir du site http://www.ncbi.nlm.nih.gov/BLAST/ avec les paramètres indiqués par défaut sur ce site.

Selon un mode préférentiel de réalisation de l'invention, la cystathionine-γ synthase initiale, avant modification, comprend une séquence d'acide aminés ci-dessous dans sa partie C-terminale (zone conservée 1)

X1-X2-X3-**L-G**-X4-X5-X6-X7-X8-X9

dans laquelle
X1 représente A,G,S, de preference A
X2 représente E,V,P,T, de preference E
X3 représente S,T,N, de preference S
X4 représente G,D,A,H,T, de preference G
X5 représente V,A,T,H,N, de preference V
X6 représente E,R,K,F, de preference E
X7 représente S,T, de preference S
X8 représente L,I,V,A, de preference L et
X9 représente I,V,A,T, de preference I.

De manière préférentielle, la cystathionine-γ-synthase à activité « méthionine synthase » modifiée, selon l'invention comprend la séquence d'acides aminés suivante dans sa partie C-terminale :

A-E-S-**L-G**-G-V-E-S

De manière avantageuse, la cystathionine γ-synthase initiale, avant modification, comprend également au moins une séquence d'acide aminés ci-dessous dans sa partie N-terminale (zone conservée 2) :

X10-X11-**Y**-X12-**R-**X13-X14-X15-X16-X17-X18

dans laquelle
X10 représente A, H,Y,F,L,K, de preference A
X11 représente Y, E,D,K,R,V,I, de preference Y
X12 représente S,A,T,P,G, de preference S
X13 représente I,S,T,R,E,F,W,D, de preference S
X14 représente S,G,A,I,E,N,K,P, de preference G
X15 représente N,H,Q,S, de preference N
X16 représente P,D,L, de preference P
X17 représente T,M,N,G,S, de preference T et
X18 représente R,L,V,S,W,E, de preference R.

Les acides aminés X1 à X9 correspondent aux résidus 324 à 334 de la séquence de cystathionine γ-synthase de *E. coli* K12, représenté sur la SEQ ID NO 6.

Les acides aminés X10 à X18 correspondent aux résidus 44 à 54 de la séquence de cystathionine γ-synthase de *E. coli* K12, représenté sur la SEQ ID NO 6.

Les positions des acides aminés employées ci-dessus et ci-après sont entendues comme étant des positions relatives faites par référence à la séquence de cystathionine-γ-synthase de *E. coli* K12. L'homme du métier saura bien entendu retrouver les acides aminés correspondants sur d'autres séquences de cystathionine-γ-synthases, par l'emploi d'outils usuels d'alignement de séquences. On citera notamment le programme BLAST, qui peut être utilisé à partir du site http://www.ncbi.nlm.nih.gov/BLAST/ avec les paramètres indiqués par défaut sur ce site. L'alignement de séquence pourra être effectué tant au niveau de la protéine (SEQ ID NO 6) que de sa séquence codante, comme par exemple la séquence codante représentée sur la SEQ ID NO 5.

On peut aussi avantageusement utiliser la recherche avancée de BlastP en affinant la recherche avec un motif (PHI-BLAST). Dans ce cas on pourra prendre le motif [**A**GS]-[**E**VPT]-[**S**TN]-**L-G**-[**G**DAHT]-[VATHN]-[ERKF]-[ST]-[**L**IVA]-[IVAT] pour une première zone conservée et le motif x(2)-**Y**-[**S**ATPG]-**R**-x(2)-[**N**HQS]-[PDL]-[**T**MNGS]-[RLVSWE] pour une deuxième zone conservée où les lettres indiquées en gras correspondent aux acides aminés majoritairement présent à cette position dans la séquence, et où x correspond à n'importe quel acide aminé. Le chiffre 2 entre parenthèse signifie qu'il y a deux acides aminés indéterminés.

Pour l'alignement des séquences, on peut utiliser les programmes CLUSTALW (http://www.ebi.ac.uk/clustalw) ou MULTALIN ((http://prodes.toulouse.inra.fr/multalin/cgi-bin/multalin.pl), avec les paramètres indiqués par défaut sur ces sites.

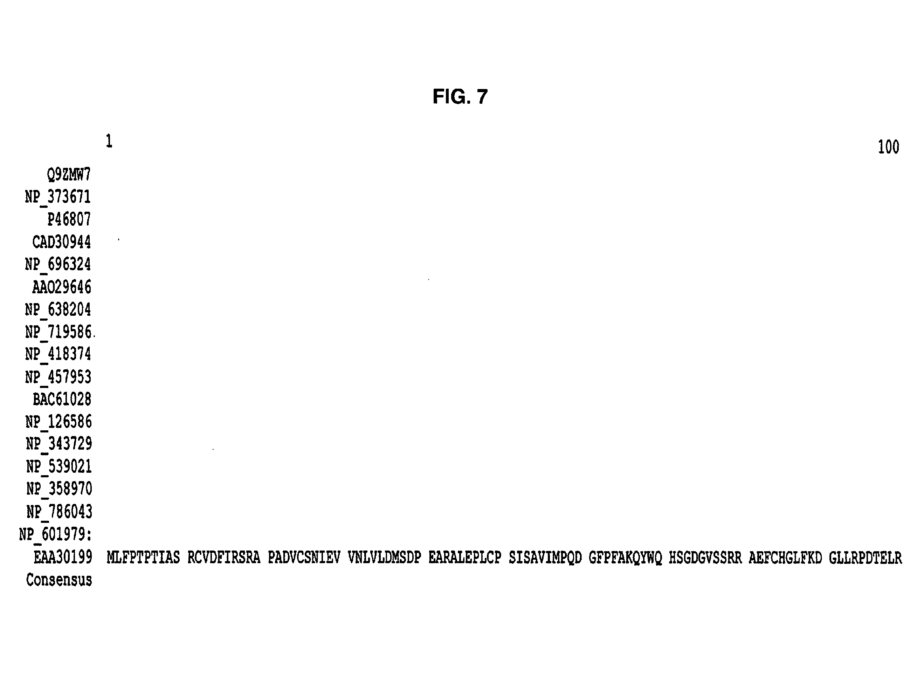

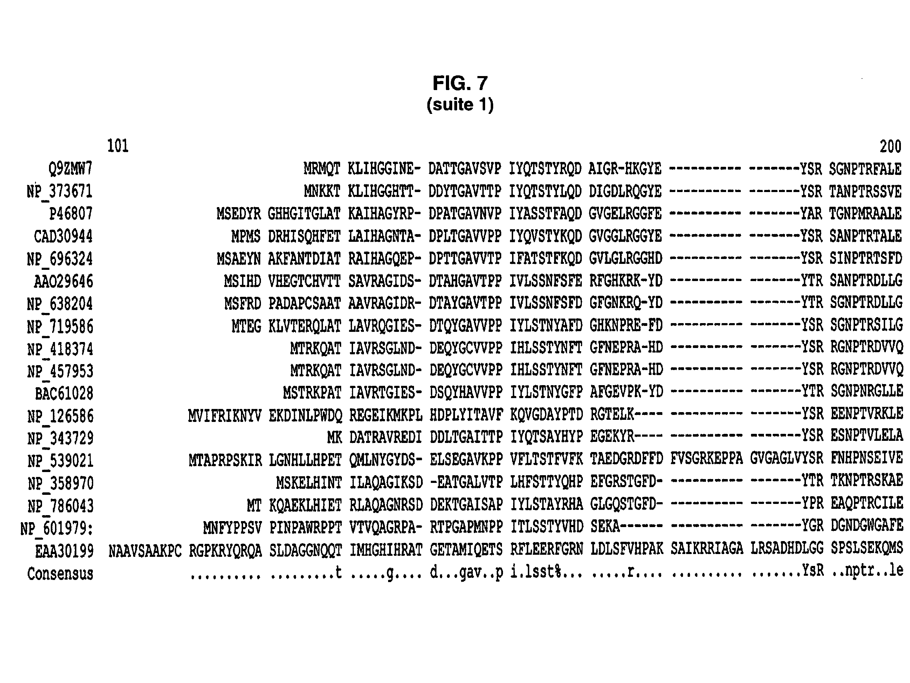

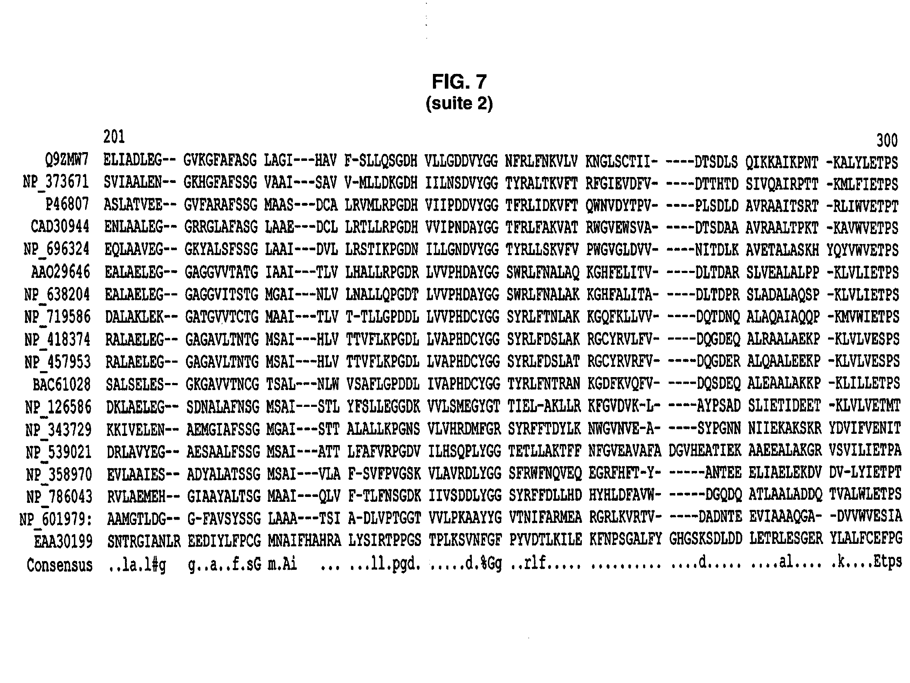

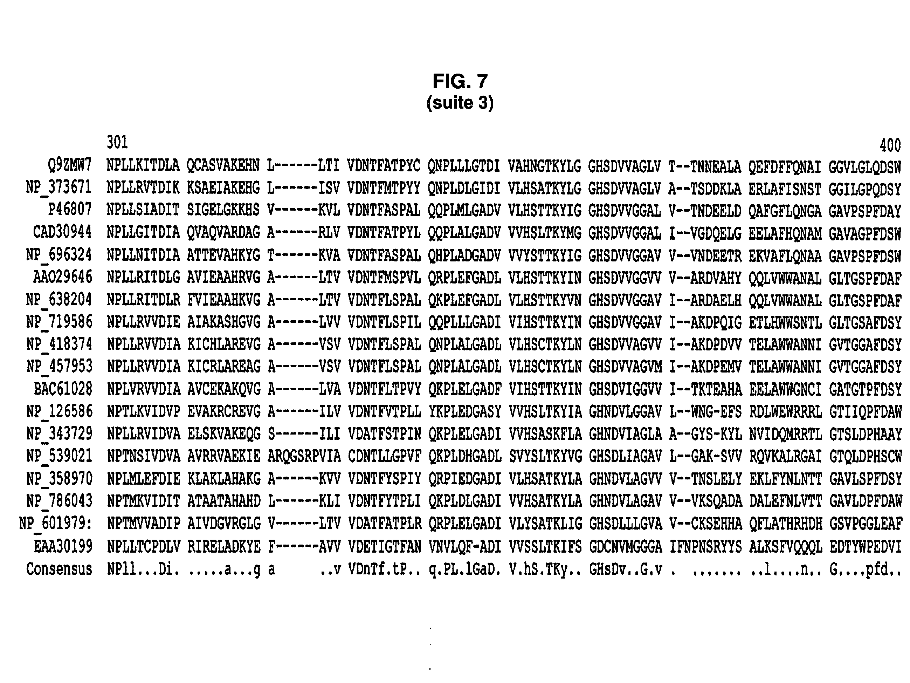

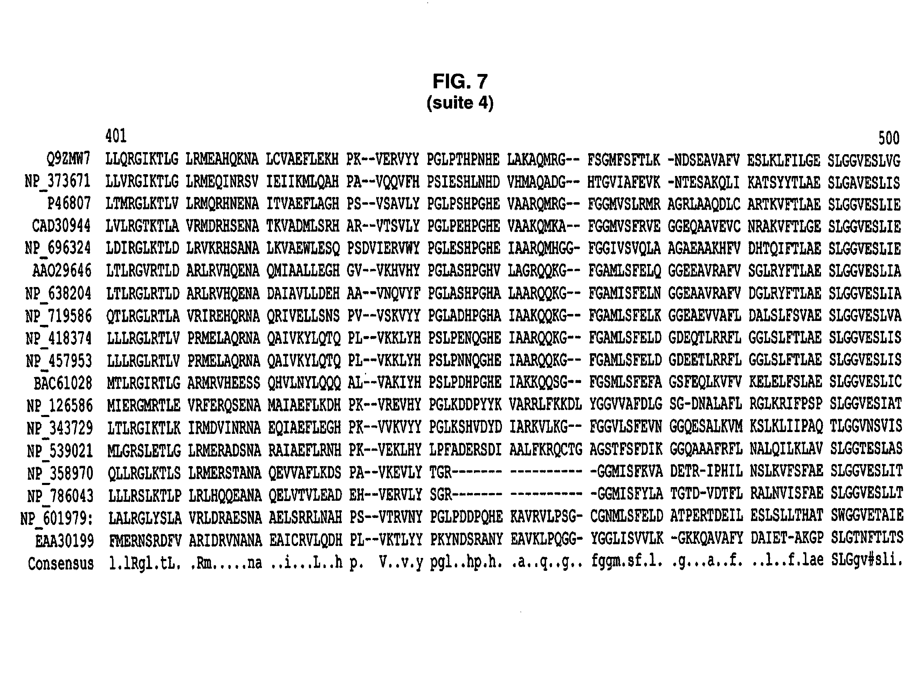

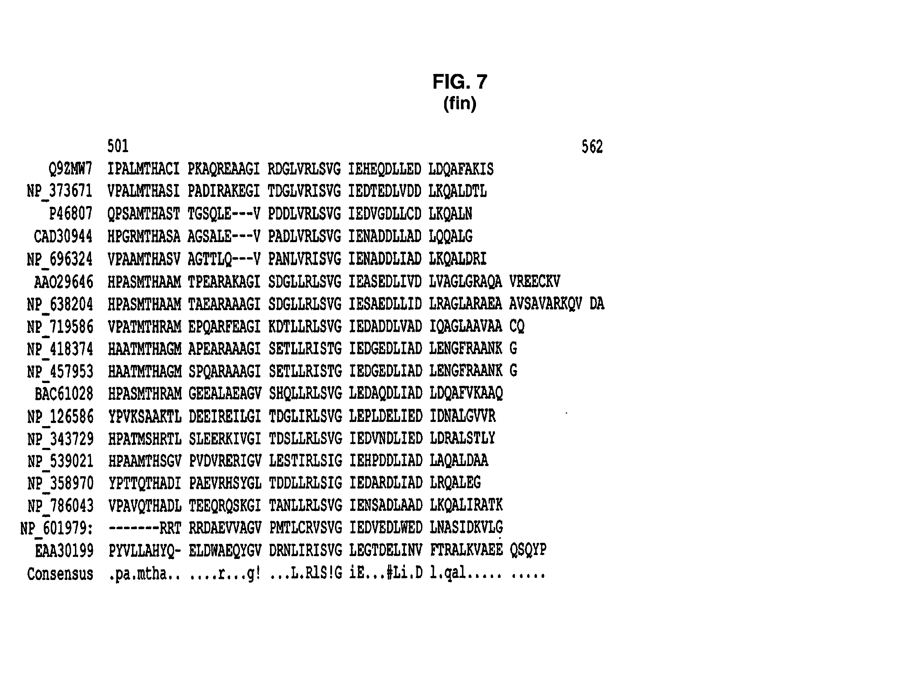

Un tel alignement de séquences est représenté sur la figure 7 pour une sélection de différentes cystathionine-γ-synthases.

Elles sont de préférence choisies parmi les cystathionine-γ-synthase (CGS) suivantes :
Q9ZMW7 O-succinylhomoserine (Thiol)-lyase, Helicobacter pylori
P46807 O-succinylhomoserine (Thiol)-lyase, Mycobacterium leprae
AA029646 Xylella fastidiosa Temeculal
NP_638204 Xanthomonas campestris pv. campestris str. ATCC 33913
NP_358970 Streptococcus pneumoniae R6
NP_126586 O-succinylhomoserine (Thiol)-lyase, Pyrococcus abyssi
NP_373671 Staphylococcus aureus subsp. aureus N315
NP_418374 [Escherichia coli K12
NP_601979 Corynebacterium glutamicum ATCC 13032
NP_343729 O-succinylhomoserine (thiol)-lyase, Sulfolobus solfataricus
NP_786043 O-succinylhomoserine (thiol)-lyase, Lactobacillus plantarum WCFS1
NP_719586 Shewanella oneidensis MR-1
CAD30944 Streptomyces coelicolor A3(2)
NP_696324 Bifidobacterium longum NCC2705
NP_457953 Salmonella enterica subsp. enterica serovar Typhi
NP 539021 Brucella melitensis
EAA30199 O-succinylhomoserine (Thiol)-lyase, Neurospora crassa
BAC61028 Vibrio parahaemolyticus.

De préférence, la cystathionine-γ-synthase modifiée telle que définie précédemment, comprend au moins une mutation dans sa partie C-terminale, et/ou au moins une mutation dans sa partie N-terminale.

Par mutation, on entend selon l'invention la substitution d'un acide aminé de la séquence native par un acide aminé différent.

De préférence, la mutation consiste à remplacer un acide aminé acide, lequel interagit avec le co-substrat cystéine pour l'enzyme non modifiée, par un acide aminé apolaire, sélectionné parmi les résidus glycine, alanine, leucine, isoleucine, valine, phénylalanine ou méthionine.

Ces acides aminés peuvent être identifiés par référence à la structure cristalline de la cystathionine-γ-synthases de E. *coli,* décrite par Clausen & al. (EMBOJ, Vol. 17, No. 23, pp 6827-6838, 1998).

De manière avantageuse, la mutation dans la partie C-terminale est introduite parmi les acides aminés acides de la « zone conservée 1 » telle que définie ci-dessus, en particulier au niveau du résidu X2.

La cystathionine-γ-synthase à activité « méthionine synthase » modifiée selon l'invention comprend avantageusement la séquence d'acides aminés suivante dans sa partie C-terminale :

X1-X2-X3-**L**-**G**-X4-X5-X6-X7-X8-X9

dans laquelle
X1, X3, X4, X5, X6, X7, X8 et X9 sont définis ci-dessus, et
X2 représente G,A,L,I,V,F,M, de préférence A.

De manière avantageuse, la mutation dans la partie N-terminale est introduite parmi les acides aminés acides de la « zone conservée 2 » telle que définie ci-dessus, en particulier au niveau du résidu X11 et/ou R et/ou X13.

De manière préférentielle, la cystathionine-γ-synthase à activité « méthionine synthase » modifiée, selon l'invention comprend la séquence d'acides aminés suivante dans sa partie C-terminale :

A-A-S-**L-G**-G-V-E-S

La cystathionine-γ-synthase à activité « méthionine synthase » modifiée selon l'invention peut comprendre avantageusement la séquence d'acide aminés suivante dans sa partie N-terminale :

X10-X11-Y-X12-X19-XI3-XI4-X15-X16-XI7-X18

dans laquelle
X7, X9, X12, X14, X15, X16, X,17 et X,18 sont définis ci-dessus,
X11 est défini ci-dessus ou représente un acide aminé apolaire,
X13 est défini ci-dessus ou représente un acide aminé apolaire,
X19 est défini ci-dessus ou représente un acide aminé apolaire, et
l'un au moins de X11, X13 et X19 représente un acide aminé apolaire tel que défini précédemment.

Selon un mode préférentiel de réalisation de l'invention, la cystathionine-γ-synthase à activité « méthionine synthase » modifiée est une cystathionine-γ-synthase telle que définie précédemment, modifiée pour permettre la conversion directe de la O-succinyl-L-homosérine en L-methionine avec le méthyl-mercaptan comme source de soufre (composé de formule générale (II) dans lasquelle R représente le méthyle).

Selon un mode de réalisation préférentiel de l'invention, la cystathionine-γ-synthase à activité « méthionine synthase » modifiée comprend la séquence d'acides aminés représentée sur la SEQ ID NO 8. Une séquence d'ADN codant pour cette enzyme modifiée est représentée sur la SEQ ID NO 7.

La présente invention concerne également les cystathionine-γ-synthases modifiées telles que définies ci-dessus ainsi que les séquences d'acide nucléique codant pour ces cystathionine-γ-synthases modifiées, notamment les séquences isolées, en particulier les séquences d'ADN et notamment la séquence représentée sur la SEQ ID NO 7, les vecteur de clonage et/ou d'expression comprenant les dites séquences, en particulier les vecteurs comprenant lesdites séquences d'acide nucléique sous le contrôle d'éléments de régulation nécessaires à l'expression et la transcription de la cystathionine-γ-synthase modifiée dans un organisme hôte et les organismes hôtes transformés avec lesdits vecteurs.

De manière avantageuse, les acylhomosérine sulfhydrylases « initiales » ou à activité « méthionine synthase » non modifiée sont chosies parmi les acylhomosérine sulfhydrylases correspondant au PFAM référence PF01053 et au COG référence COG2873.

Elles sont de préférence choisies parmi les acylhomosérine sulfhydrylases suivantes :
NP_785969 O-acetylhomoserine (thiol)-1yase, Lactobacillus plantarum WCFS1
AAN68137 O-acetylhomoserine sulfhydrylase, Pseudomonas putida KT2440
NP_599886 O-acetylhomoserine -sulfhydrylase, Corynebacterium glutamicum ATCC 13032
NP_712243 acetylhromoserine sulfhydrylase, Leptospira interrogans serovar lai str. 56601
BAC46370 O-succinylhomoserine sulfhydrylase, Bradyrhizobium japonicum USDA110
AAO57279 O-succinylhomoserine sulfhydrylase, Pseudomonas syringae pv. tomato str. DC3000
NP_284520 O-succinylhomoserine sulfhydrolase [Neisseria meningitidis Z2491
AAA83435 O-succinylhomoserine sulfhydrylase (P. aeruginosa)

Selon un mode préférentiel de réalisation de l'invention, la modification de la voie de biosynthèse de la méthionine nécessite dans un premier temps de modifier génétiquement la bactérie initiale. La modification implique nécessairement l'atténuation d'au moins un gène et éventuellement le clonage d'au moins un gène hétérologue. L'atténuation du gène doit rendre la bactérie dépendante de la restauration d'une voie métabolique équivalente afin de permettre sa croissance. La bactérie génétiquement modifiée est cultivée et l'on sélectionne la ou les souches bactériennes dont le taux de croissance s'améliore en présence ou non d'un co-substrat exogène.

Le procédé de préparation de souches selon l'invention consiste à obtenir, à partir d'une souche bactérienne initiale, une souche bactérienne génétiquement modifiée présentant au moins une modification dans un gène codant pour une enzyme à activité , « méthionine synthase » (*e.g*. MetE chez *E. coli*) ou « homocystéine synthase » (*e.g*. MetC chez *E. coli*), ledit procédé comprenant une étape consistant à soumettre ladite souche bactérienne initiale à une pression de sélection en présence de la source de soufre définie ci-dessus, afin de diriger une évolution d'au moins un gène (*e.g. met*B chez *E. coli*) dans ladite souche bactérienne, afin de restaurer une activité « méthionine synthase » ou « homocystéine synthase » dans la souche évoluée ; cette restauration d'activité n'étant pas liée à une réversion de la modification réalisée.

L'invention concerne donc également une souche à activité « méthionine synthase » améliorée qui comprend une inactivation d'au moins un gène endogène impliqué dans la voie de biosynthèse habituelle de la méthionine.

Dans un mode de réalisation préféré, la souche bactérienne comprend une inactivation d'au moins un gène endogène choisi parmi *met*B, *met*J, *met*C, *met*E*, met*H*.*

Dans un mode préféré d'application de l'invention, ladite modification de la souche initiale, que l'on retrouve dans la souche à activité « méthionine synthase améliorée », notamment *E. coli,* consiste à inactiver l'activité méthionine-synthase native (codée par le gène *met*E chez *E. coli*) puis à cultiver ledit microorganisme modifié obtenu sur un milieu défini dépourvu de méthionine, S-adenosylméthionine, homocystéine et cystathionine mais en présence de methylmercaptan (co-substrat exogène), ou de l'un de ses sels, et de sélectionner un microorganisme évolué caractérisé en ce que l'activité méthionine-synthase de la cystathionine γ-synthase se soit fortement améliorée, en présence de methylmercaptan, au point de remplacer l'activité initialement inactivée. Dans ce mode d'application de l'invention il est important de noter que le milieu utilisé pour sélectionner le microorganisme évolué est identique au milieu sur lequel pousse le microorganisme initial (c'est à dire non modifié) ; seul un co-substrat (*e.g*. alkylmercaptan) est ajouté. Selon un mode préférentiel de réalisation de l'invention, la souche bactérienne sélectionnée comprenant le microorganisme évolué selon l'invention, est une souche de *E. coli,* plus préférentiellement la souche *E. coli* K183, déposée sous le numéro I-3005, le 2 avril 2003 à la Collection Nationale de Culture et Microorganismes (CNCM), 25 rue du Docteur Roux, 75724 "Paris Cedex15, France, selon les dispositions du traité de Budapest. Cette souche comprend un gène exprimant une cystathionine-γ-synthase modifiée, l'enzyme comprenant la mutation E325A, et une inactivation du gène *met*E*.*

Dans un mode particulier d'application de cette invention, il est aussi possible d'utiliser des souches ayant en plus les délétions *met*C et/ou *met*H et/ou *met*J pour obtenir les souches à activité « méthionine synthase » améliorée correspondante.

Dans un second mode tout aussi préféré d'application de l'invention, ladite modification de la souche initiale, notamment *E. coli,* consiste à inactiver l'activité cystathionine-β-lyase (codée par le gène *met*C chez *E. coli*) puis à cultiver ledit microorganisme modifié obtenu sur un milieu défini dépourvu de méthionine, S-adenosylmethionine, homocystéine et cystathionine puis de sélectionner un microorganisme évolué caractérisé en ce que l'activité homocystéine-synthase de la cystathionine γ-synthase se soit fortement améliorée, en présence d'H2S endogène, au point de remplacer l'activité initialement inactivée. Dans ce mode d'application de l'invention il est important de noter que le milieu utilisé pour sélectionner le microorganisme évolué est identique au milieu sur lequel pousse le microorganisme initial (c'est à dire non modifié). Dans un mode particulier de cette application, il est aussi possible d'ajouter dans le milieu du NaSH. Dans un autre mode particulier de cette application il est possible d'utiliser une souche qui présente en plus au moins une mutation prise parmi *met*H et/ou *met*J*.*

Dans un troisième mode tout aussi préféré d'application de l'invention, ladite modification de la souche initiale, notamment *E. coli,* consiste à inactiver les activités succinyl-homosérine (codée par le gène *met*B) et méthionine-synthase native (codée par le gène *met*E chez *E. coli*) puis à introduire l'activité acetyl-homoserine sulfhydrylase (codée par le gène *met*Y de *C. glutamicum*) et à cultiver ledit microorganisme modifié obtenu sur un milieu défini dépourvu de méthionine, S-adenosylmethionine, homocystéine et cystathionine mais supplémenté en methylmercaptide de sodium afin de sélectionner un microorganisme évolué caractérisé en ce que l'activité méthionine-synthase portée par l'acetyl-homosérine sulfhydrylase (METY) se soit fortement améliorée, en présence de methylmercaptide de sodium, au point de remplacer l'activité methionine-synthase initialement inactivée. Dans ce mode d'application de l'invention il est important de noter que le milieu utilisé pour sélectionner le microorganisme évolué est identique au milieu sur lequel pousse le microorganisme initial (c'est à dire non modifié) ; seul un co-substrat (e.g. methylmercaptan) est ajouté. Dans un mode particulier de cette application il est possible d'utiliser une souche qui présente en plus au moins une mutation prise parmi *met*H et/ou *met*J et/ou *met*C.

Une mutation du gène *met*J a été proposée dans JP 2000157267-A/3, pour produire une quantité supérieure de méthionine (voir aussi GenBank E35587). Ce gène code pour une protéine de répression des gènes *met* B, E, L, J et R (chez *Salmonella typhimurium*). Son inactivation ou sa modification permet de diminuer le rétrocontrôle par la méthionine.

Le gène *met*C (GenBank M12858), code la cystathionine-β-lyase (EC 4.4.1.8), les gènes *met*E (GenBank AE000458) et *met*H (GenBank J04975) codent la méthionine synthase (EC 2.1.1.13). La méthionine est un acide aminé essentiel à la vie cellulaire. L'inactivation d'un ou plusieurs de ces gènes revient à supprimer la voie habituelle de biosynthèse de la méthionine.

En utilisant les références données sur GenBank pour ces gènes qui sont bien connus, l'homme du métier est capable de déterminer les gènes équivalents dans d'autres souches bactériennes qu*'E. coli.* Ce travail de routine est avantageusement effectué en utilisant les séquences consensus pouvant être déterminées du fait de la synthèse de ces gènes pour d'autres microorganismes, et en dessinant des sondes dégénérées permettant de cloner le gène correspondant dans un autre organisme. Ces techniques de routine de biologie moléculaire sont bien connues dans l'art et sont décrites par exemple dans Sambrook et al. (1989 Molecular cloning: a laboratory manual. 2nd Ed. Cold Spring Harbor Lab., Cold Spring Harbor, New York.).

La souche à activité « methionine synthase » modifiée selon l'invention comprenant une enzyme à activité « méthionine synthase » modifiée définie ci-dessus comprend de préférence au moins une inactivation du gène *met*E et/ou metH, et/ou du gène *met*C*,* et/ou du gène *met*B*.*

Lorsque l'enzyme à activité « méthionine synthase » modifiée définie ci-dessus permet d'effectuer la conversion directe du substrat de formule générale (III) en acide de formule générale (I), la souche selon l'invention comprend avantageusement au mois une inactivation du gène *met*E et/ou *met*H et/ou *met*B*,* de préférence au moins une inactivation du gène *met*E*.*

Lorsque l'enzyme à activité « méthionine synthase » modifiée définie ci-dessus permet d'effectuer la conversion du substrat de formule générale (III) en homocystéine de formule générale (IV)

HS-(CH₂)₂-CHNH₂-COOH (IV)

laquelle est ensuite transformée en acide de formule générale (I) par une enzyme appropriée, la souche selon l'invention comprend au moins une inactivation du gène *met*C et/ou *met*B*.* Elle peut également comprendre une inactivation du gène *met*E et/ou *met*H endogène. Dans ce cas, l'activité méthylase associée aux gènes *met*E et/ou *met*H est restaurée par l'introduction d'un gène codant pour une enzyme ayant la même activité. Cette enzyme peut avoir été sélectionnée et/ou modifiée pour permettre une amélioration des rendements-de-synthèse des acides aminés de formule générale (I).

Dans un mode de réalisation, ladite souche bactérienne peut comprendre également une modification de l'activité homosérine O-acyltransférase porté par le gène *met*A afin de lui conférer au choix une activité homosérine O-succinyltransférase (EC 2.3.1.46) ou homosérine O-acétyltransférase (EC 2.3.1.11).

Dans un mode particulier, on pourra remplacer ou modifier le gène *met*A de *E. coli,* codant l'enzyme possédant l'activité homosérine O-succinyltransférase (Genbank AAN83396), afin d'obtenir une activité homosérine O-acétyltransférase. II est connu de l'homme du métier que cette activité est codée par le gène *metA* de *C. glutamicum* (Genbank AF052652). Les protocoles permettant de remplacer le gène *met*A de *E. coli* par le gène *met*A de *C. glutamicum,* ou de modifier la séquence de *met*A de *E. coli* afin d'obtenir une activité homosérine O-acetyltransferase au lieu d'une activité homosérine O-succinyltransférase sont connus de l'homme du métier.

De manière similaire on peut remplacer ou modifier le gène *met*A de *C. glutamicum,* codant une activité homosérine O-acetyltransférase, afin d'obtenir une activité homosérine O-succinyltransférase.

Toutes les modifications mentionnées ci-dessus peuvent être effectuées directement sur la souche objet de la pression de sélection, lorsque le procédé selon l'invention est mis en oeuvre. Alternativement, il est préférable de mettre en oeuvre le procédé de criblage selon l'invention sur une souche ne présentant qu'un nombre restreint de modifications, d'obtenir une souche présentant une activité « méthionine synthase » en présence du composé de formule (II), en particulier de méthyl-mercaptan, et d'effectuer alors d'autres modifications telles que mentionnées, afin d'augmenter le 'bypass' de la voie classique de synthèse de la méthionine.

L'homme du métier connaît les protocoles permettant de modifier le caractère génétique de microorganismes. La surexpression d'un gène peut être effectuée par changement du promoteur de ce gène *in situ,* par un promoteur fort ou inductible. De façon alternative, on introduit, dans la cellule, un plasmide réplicatif (simple ou multicopies) dans lequel le gène que l'on désire surexprimer est sous le contrôle du promoteur adéquat.

L'inactivation d'un gène se fait préférentiellement par recombinaison homologue. Le principe d'un protocole en est rappelé brièvement : on introduit dans la cellule un fragment linéaire, obtenu *in vitro,* comprenant les deux régions flanquant le gène, et au moins un gène de sélection entre ces deux régions (généralement un gène de résistance à un antibiotique), ledit fragment linéaire présentant donc un gène inactivé. On sélectionne les cellules ayant subi un événement de recombinaison et ayant intégré le fragment introduit par étalement sur milieu sélectif. On sélectionne ensuite les cellules ayant subi un événement de double recombinaison, dans lesquelles le gène natif a été remplacé par le gène inactivé. Ce protocole peut être amélioré en utilisant des systèmes de sélections positive et négative, afin d'accélérer la détection des événements de double recombinaison.

Dans un mode de réalisation préféré, ladite souche bactérienne est une souche *d'E. coli.*

Dans un autre mode de réalisation, ladite souche bactérienne est une souche de *Corynebacterium,* en particulier *C. glutamicum.*

Selon un mode préférentiel de réalisation de l'invention, la souche bactérienne est la souche *E. coli* K183, déposée à la CNCM le 2 avril 2003 sous le numéro 1-3005. Cette souche comprend un gène exprimant une cystathionine-γ-synthase modifiée, l'enzyme comprenant la mutation E325A, décrite précédemment et une inactivation du gène *met*E*.*

Les souches de microorganismes selon l'invention possèdent une enzyme cystathionine-γ-synthase et/ou l'acylhomosérine sulfhydrylase ; elles sont de préférence sélectionnées et améliorées par un procédé de criblage et d'évolution, qui est aussi un objet de l'invention. Les souches selon l'invention peuvent également être génétiquement modifiées (c'est-à-dire présenter une inactivation, une mutation et/ou la suractivation d'au moins un gène endogène), la modification étant effectuée préalablement ou non à la mise en oeuvre du procédé de criblage.

Afin d'accélérer la sélection et l'évolution dirigée des souches pour la production de méthionine en présence de composé de formule (II), en particulier de méthyl-mercaptan, on peut effectuer les opérations ci-dessous. Le procédé est décrit pour le méthyl-mercaptan. Toutefois, l'homme du métier saura l'adapter avec tout autre composé de formule (II), en particulier l'H2S.

### a. Coupler la biosynthèse de la molécule d'intérêt à la croissance du microorganisme de telle sorte que la production de cette molécule est nécessaire pour une bonne croissance du microorganisme

Pour cette raison on peut faire le choix de détruire le gène *met*E codant la méthionine-synthase qui produit la méthionine à partir d'homocystéine. Ce faisant la souche devient auxotrophe pour la méthionine.

Le microorganisme, pour vivre en milieu minimum contenant une source de carbone simple et du méthylmercaptan ou méthylmercaptide de sodium, doit donc optimiser la voie de synthèse de la L-méthionine à partir de l'O-acyl-L-homosérine et du méthylmercaptan ou méthylmercaptide de sodium. Une modélisation informatique montre que dans ces conditions il est possible de doubler les rendements théoriques en méthionine (Tableau 1).

**Tableau 1 : rendements théoriques maximums pour la production de méthionine (g de produit/g de glucose) par E. coli dans le cas d'une fermentation sur glucose (a) et d'une fermentation sur glucose et méthyl-mercaptan (b) avec un rendement en biomasse constant (cultures continues).**

| Rendements biomasse | Rendements méthionine^{a} | Rendements méthionine^{b} |
|---|---|---|
| **Y**x/s (g.g⁻¹) | **Y**_{P/S} (g_{.}g⁻¹) | **Y**_{P/S} (g.g⁻¹) |
| 0 | 0,36 | 0,74 |
| 0,11 | 0,30 | 0,62 |
| 0,28 | 0,21 | 0,42 |
| 0,44 | 0,12 | 0,24 |
| 0,61 | 0 | 0 |

Cependant, lorsque l'on souhaite produire un acide 2-amino-4-(alkylmercapto)butyrique différent de la L-méthionine, il est nécessaire de supplémenter le milieu en méthionine pour permettre la croissance du microorganisme.

### b. Supprimer les régulations, notamment les rétro-inhibitions soit au niveau des enzymes, soit au niveau des gènes afin que la voie de biosynthèse principale soit potentialisée

On peut ainsi supprimer le gène *met*J codant une protéine répresseur. Par ailleurs, il a été montré que l'homosérine trans-succinylase, codée par le gène *metA,* était rétro-inhibée par la méthionine et la S-adénosylméthionine (Taylor et al., 1966, J. Biol. Chem., 241 : 3641-3642). Il est donc souhaitable de remplacer cette enzyme par une enzyme insensible à la rétro-inhibition (Chater et al, 1970, J. Gen. Microbiol. 63: 121-l31).

Un autre objet de l'invention est un test de criblage-identification permettant d'obtenir un microorganisme produisant de l'acide 2-amino-4-(alkylmercapto)butyrique, notamment la L-méthionine, en métabolisant un composé soufré de formule générale (II), en particulier un alkyl-mercaptan ou l'H2S, notamment le méthyl-mercaptan.

Ainsi, la présente invention permet d'identifier des souches présentant des mutations dans leur génome, lesdites mutations permettant l'assimilation du composé de formule (II) par ladite souche, et la production dudit acide aminé de formule (I). Lesdites modifications induisent donc une modification/augmentation de l'activité « méthionine synthase » de ladite souche. On peut alors accélérer la production de la souche produisant de la méthionine de façon autonome à partir d'une source de carbone simple et de composé de formule (II).

Un autre objet de l'invention est donc un procédé de criblage d'une souche bactérienne initiale, éventuellement génétiquement modifiée, possédant un gène codant pour une enzyme cystathionine-γ-synthase ou acylhomosérine sulfhydrylase, en vue d'obtenir une souche bactérienne génétiquement modifiée produisant un acide aminé de formule (I), en particulier de la L-méthionine, et présentant une modification dans le gène de ladite enzyme, induisant une modification de l'activité « méthionine synthase » en présence d'un composé soufré de formule (II), présentant l'étape consistant à soumettre ladite souche bactérienne à une pression de sélection en présence du composé de formule (II), afin de diriger une évolution du gène codant pour ladite enzyme cystathionine-γ-synthase ou acylhomosérine sulfhydrylase, dans ladite souche bactérienne, ladite évolution consistant en une mutation pour leur permettre de réaliser de manière préférentielle la conversion directe du substrat de formule générale (III) en acide aminé de formule générale (I) ou en homocystéine de formule générale (IV).

Ladite souche bactérienne initiale présente éventuellement une inactivation et/ou une suractivation, notamment par insertion d'un promoteur fort constitutif, d'au moins un gène endogène.

L'invention se rapporte également à une souche bactérienne présentant une modification dans le gène de l'enzyme cystathionine-γ-synthase et/ou dans le gène de l'enzyme acylhomosérine sulfhydrylase, induisant une augmentation de l'activité « méthionine synthase » de ladite enzyme en présence du composé de formule (II), en particulier de méthyl-mercaptan. Une telle souche peut également présenter au moins une autre modification génétique, (inactivation, mutation ou surexpression d'un gène endogène), telle que mentionnée ci-dessus.

La souche selon l'invention est de préférence susceptible d'être obtenue par un procédé selon l'invention, et en particulier est obtenue par le procédé selon l'invention.

L'invention se rapporte également à un procédé de préparation d'un acide 2-amino-4(alkylmercapto)butyrique de formule (I) définie ci-dessus, dans lequel on cultive un microorganisme à activité « méthionine synthase » modifiée telle que défini précédemment, en présence d'un composé soufré de formule générale (II) dans un milieu approprié, ledit milieu approprié comprenant une source de carbone simple telle que définie précédemment. De préférence, ledit acide aminé de formule (I) est la méthionine, plus préférentiellement L-méthionine, et ledit composé soufré de formule (II) est le méthyl-mercaptan ou l'H2S.

Selon l'invention, les termes « culture » et « fermentation » sont employés indifféremment pour désigner la croissance de la bactérie sur un milieu de culture approprié comprenant une source de carbone simple.

La définition des conditions de culture des microorganismes selon l'invention (fermentation) est à là portée de l'homme du'métier. On fermente notamment les bactéries à une température comprise entre 20°C et 55°C, de préférence entre 25°C et 40°C, plus particulièrement d'environ 30°C pour *C. glutamicum* et d'environ 37°C pour *E. coli.*

La fermentation est généralement conduite en fermenteurs avec un milieu minéral de culture de composition connu défini et adapté en fonction des bactéries utilisées, contenant au moins une source de carbone simple ainsi que du composé soufré de formule (II).

En particulier, le milieu minéral de culture pour *E. coli* pourra ainsi être de composition identique ou similaire à un milieu M9 (Anderson, 1946, Proc. Natl. Acad. Sci. USA 32:120-128), un milieu M63 (Miller, 1992; A Short Course in Bacterial Genetics: A Laboratory Manual and Handbook for Escherichia coli and Related Bacteria, Cold Spring Harbor Laboratory Press, Cold Spring Harbor, New York) ou un milieu tel que défini par Schaefer et al. (1999, Anal. Biochem. 270 : 88-96).

De manière analogue, le milieu minéral de culture pour *C. glutamicum* pourra ainsi être de composition identique ou similaire au milieu BMCG (Liebl et al., 1989, Appl. Microbiol. Biotechnol. 32: 205-210) à un milieu tel que défini par Riedel et al. (2001, J. Mol. Microbiol. Biotechnol. 3: 573-583).

Les milieux peuvent être supplémentés pour compenser les auxotrophies ; ils contiennent une concentration en carbone simple adaptée en fonction du mode de culture et de production, ainsi que du composé soufré de formule (II) en concentration adaptée en fonction de l'évolution de la souche et du mode de production retenu.

Après fermentation, l'acide aminé de formule (I) est récupéré selon les méthodes usuelles, et le cas échéant, purifié.

Les techniques de récupération puis de purification des acides aminés de formule (I) dans les milieux de culture sont bien connues de l'homme du métier.

La présente invention concerne également un procédé de préparation d'un acide 2-amino-4(alkylmercapto)butyrique de formule (I) défini précédemment, dans lequel on fait réagir substrat dérivé de formule générale (III)

R"-O-(CH₂)₂-CHNH₂-COOH (III)

dans laquelle R" représente un radical acyle, de préférence choisi parmi le radical succinyle ou le radical acétyle,
avec une enzyme à activité « méthionine synthase » modifiée telle que définie précédemment, dans un milieu réactionnel approprié comprenant un composé soufré de formule générale (II) défini précédemment.

Le milieu réactionnel approprié est un milieu usuel de réaction enzymatique, bien connu de l'homme du métier, en particulier un milieu aqueux dans lequel les substrats et l'enzyme sont en solution ou en suspension. Les conditions de mise en oeuvre de la réaction sont bien connues de l'homme du métier, afin notamment d'éviter une dénaturation substantielle de l'enzyme.

Selon un mode particulier de réalisation de l'invention, l'enzyme à activité « méthionine synthase » modifiée est présente dans une bactérie inactivée ou dans un extrait cellulaire.

Selon un autre mode particulier de réalisation de l'invention, l'enzyme à activité « méthionine synthase » modifiée est une enzyme purifiée.

### D.II. Voie de biosynthèse de la cystéine

La présente invention se rapporte également au domaine de la bioconversion et d'obtention d'acides aminés par fermentation de microorganismes. Elle se rapporte à une méthode de criblage et d'évolution dirigée permettant d'identifier une souche de microorganisme, éventuellement génétiquement modifié, possédant une enzyme à activité « cystéine synthase modifiée », en particulier une O-acyl-L-homosérine sulfhydrolase modifiée, ladite souche produisant de la L-cystéine ou un acide aminé dérivé par métabolisme d'une source de carbone simple. L'invention concerne également la souche de microorganisme et un procédé de préparation de la L-cystéine, ou acide aminé dérivé, par culture de ladite souche de microorganisme.

La cystéine peut-être produite en utilisant des moyens très variés et des sources différentes. Ainsi Sun-Orient Chemical Co., Ltd., extrait la L-cystine à partir des cheveux hydrolysés en présence d'HCl. La L-cystine est alors convertie en L-cysteine monohydrochloride en utilisant un procédé d'électrolyse.

La DL-cystéine monohydrochloride peut être produite par le procédé de Strecker (réaction de la L-cystine en présence d'ammonium, d'HCN et de mercaptaldehyde).

La réaction de Bucherer-Berg, dans laquelle sont mis en présence du chloroacetaldehyde, de l'HCN, du bicarbonate d'ammonium et du sulfide de sodium, permet aussi de produire de la L-cystéine. La L-cystéine étant alors cristallisée sous sa forme monohydrochloride par réaction dans une solution d'acide chlorhydrique.

La production de la cystéine par voie enzymatique ou par bioconversion en utilisant la tryptophane synthase pour catalyser la réaction entre une alanine substituée en position bêta et un sulfide a été décrite dans les demandes de brevet GB 2 174 390 et EP 0 272 365. De même, la production de cystéine par fermentation de microorganismes a été décrite dans les demandes de brevet EP 0 885 962, WO 01/27 307 et WO 97/15 673 qui décrivent respectivement une optimisation de l'excrétion de la cysteine synthétisée par les microorganismes, la surexpression du gène CysB pour optimiser la production d'H2S, et une serine acétyl transferase insensible à la rétro-inhibition Par la cystéine.

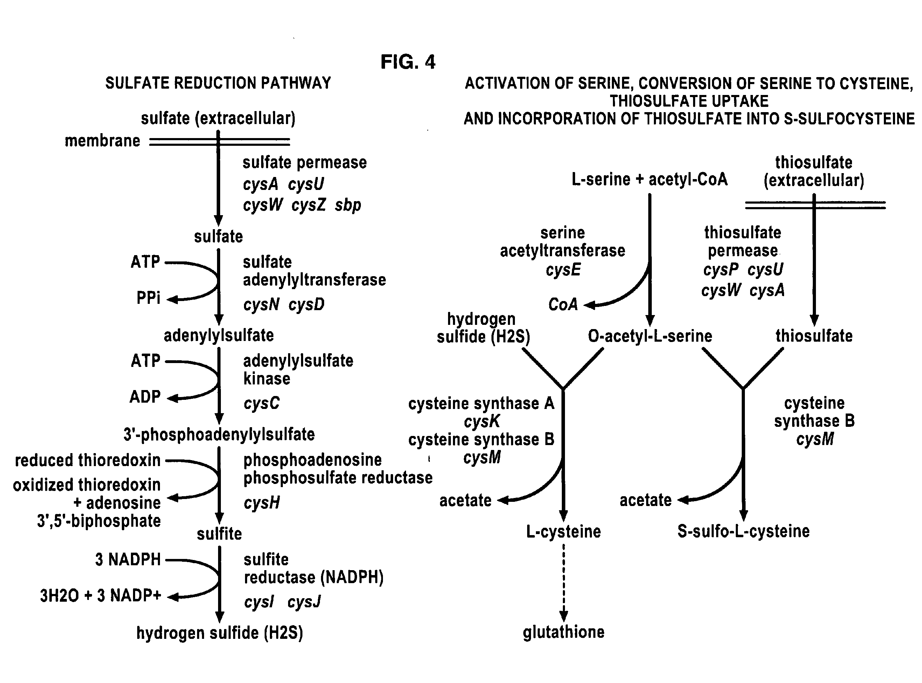

L'application de l'invention peut aussi se faire par exemple sur la voie de biosynthèse de la cystéine (figure 4) et permet d'obtenir des souches de microorganismes évolués, en particulier des bactéries, notamment *E. coli* et les corynébactéries, produisant de l'acide 2-amino-4-(alkylmercapto)propionique de formule générale (V)

R-S-CH₂-CHNH₂-COOH (V)

dans laquelle R est défini précédemment pour le composé de formule générale (I)
par métabolisme d'une source de carbone simple et d'une source de soufre comprenant un composé de formule générale (II) :

R'-SH (II)

définie précédemment,
lesdites souches présentant au moins un gène codant pour une enzyme mutée à activité « cystéine synthase évoluée ».

Par sel physiologiquement acceptable, on entend selon l'invention les sels du composé de formule générale (I) qui n'affectent pas le métabolisme et la capacité de croissance de la souche de microorganisme selon l'invention, notamment les sels de métaux alcalins comme le sodium.

Selon un mode préférentiel de réalisation de l'invention, R représente un radical alkyle linéaire ou ramifié comprenant de 1 à 4 atomes de carbone, en particulier choisi parmi les radicaux méthyle, éthyle, n-propyle, i-propyle, n-butyle, i-butyle ou t-butyle. De manière plus préférentielle, R représente le radical méthyle.

L'acide 2-amino-4-(alkylmercapto)propionique de formule générale (V) obtenu est de préférence la L-cystéine.

Par enzyme à activité « cystéine synthase évoluée », on entend selon l'invention toute enzyme mutée impliquée dans la biosynthèse de l'acide aminé de formule générale (V), en particulier de la L-cystéine dont l'activité essentielle consiste à effectuer la conversion directe d'une acétylsérine, de préférence de la O-acétyl-L-sérine en acide aminé de formule générale (V) en présence de composé soufré de formule générale (II), l'activité essentielle de l'enzyme initiale non mutée n'étant pas une activité « cystéine synthase ». Les enzymes naturellement impliquées dans la biosynthèse de la cystéine par conversion directe de la O-actéyl-L-sérine (acétylsérine) en L-cystéine en présence de sulfure d'hydrogène (H2S), comme les cystéine synthases A ou B, codées par les gènes *cys*K *ou cys*M*,* respectivement, sont donc exclues de cette définition.

De manière préférentielle, l'enzyme « initiale » non mutée est une enzyme catalysant une réaction de sulfhydrylation en présence d'H2S, de préférence une enzyme à activité O-acyl-L-homosérine sulfhydrolase.

De manière avantageuse, les enzymes « initiales » à activité O-acyl-L-homosérine sulfhydrylases sont choisies parmi les O-acyl-L-homosérine sulfhydrylases correspondant au PFAM référence PF01053 et au COG référence COG2873.

Elles sont de préférence choisies parmi les acylhomosérine sulfhydrylases suivantes :
NP_785969 O-acetylhomoserine (thiol)-lyase, Lactobacillus plantarum WCFS1
AAN68137 O-acetylhomoserine sulfhydrylase, Pseudomonas putida KT2440
NP_599886 O-acetylhomoserine sulfhydrylase, Corynebacterium glutamicum ATCC 13032
NP_712243 acetylhomoserine sulfhydrylase, Leptospira interrogans serovar lai str. 56601
BAC46370 O-succinylhomoserine sulfhydrylase, Bradyrhizobium japonicum USDA110
AAO57279 O-succinylhomoserine sulfhydrylase, Pseudomonas syringae pv. tomato str. DC3000
NP_284520 O-succinylhomoserine sulfhydrolase [Neisseria meningitidis Z2491
AAA83435 O-succinylhomoserine sulfhydrylase (P. aeruginosa)

L'O-acyl-L-homosérine sulfhydrolase est plus préférentiellement choisie parmi les O-acyl-L-homosérine sulfhydrolase codées par le gène metY de Corynebacterium, en particulier le gène *met*Y de *C*. *glutamicum* (Genbank AF220150), et les enzymes homologues présentant la même activité O-acyl-L-homosérine sulfhydrolase, et au moins 80% d'homologie de séquence avec l'O-acyl-L-homosérine sulfhydrolase codée par le gène *met*Y de *C. glutamicum* (Genbank AF220150), préférentiellement au moins 85 % d'homologie, plus préférentiellement au moins 90 % d'homologie.

L'invention est basée notamment sur le fait que l'on peut obtenir, de façon dirigée, une modification de la spécificité de substrat de l'enzyme acyl-homosérine sulfhydrylase afin qu'elle utilise préférentiellement l'acetylsérine. En conséquence l'invention est basée sur le fait que l'on peut modifier de façon dirigée la spécificité de substrat de l'enzyme non modifiée afin d'évoluer d'une activité acylhomoserine sulfhydrolase à une activité cystéine synthase.

Selon un mode préférentiel de réalisation de l'invention, les souches de microorganismes non modifiés ne possèdent pas naturellement d'activité O-acyl-L-homosérine sulfhydrolase ou ne possédant pas de gène homologue au gène *metY* codant pour cette enzyme.

Les souches modifiées selon l'invention sont génétiquement modifiées par inactivation, mutation et/ou suractivation d'au moins un gène endogène dans le but de permettre l'évolution d'une nouvelle voie métabolique. En particulier, les souches de microorganismes selon l'invention sont génétiquement modifiées afin de supprimer les gènes *cys*K et/ou *cys*M codant les protéines portant respectivement les activités enzymatiques cystéine synthase A, cystéine synthase B. De manière préférentielle, les gènes *cys*K et *cys*M sont supprimés.

Le gène *cys*K (figure 4) code la cystéine synthaseA (GenBank NP_416909) et le gènes *cys*M code la cystéine synthase B (GenBank NP_416916). L'inactivation de ces gènes revient à supprimer les voies de biosynthèse de la cystéine et donc à rendre la souche auxotrophe pour la cystéine.

On introduit alors dans les bactéries modifiées un gène codant pour une enzyme catalysant une réaction de sulfhydrylation en présence d'H2S, telle que définie précédemment, à l'exclusion des enzymes dont l'activité principale est une activité cystéine synthase (aussi dénommée O-acetylsérine sulfhydrolase), en particulier un gène codant pour une O-acyl-L-homosérine sulfhydrolase, comme le gène *met*Y de *Corynebacterium,* en particulier le gène *met*Y de *C. glutamicum* (Genbank AF220150).

Le gène codant pour une enzyme catalysant une réaction de sulfhydrylation en présence d'H2S peut être introduit dans la bactérie à modifier selon les techniques usuelles à la disposition de l'homme du métier, soit par intégration directe dans le génome, soit porté par un plasmide réplicatif.

La souche ainsi modifiée est de préférence sélectionnée et améliorée par un procédé de criblage et d'évolution, qui est aussi un objet de l'invention et qui permet de faire évoluer l'activité acyl-homosérine sulfhydrylase en une activité cystéine synthase afin de restaurer la production de cystéine.

La transformation de l'activité acyl-homosérine sulfhydrylase en activité « cystéine synthase évoluée » sera réputée acquise lorsque la souche bactérienne génétiquement modifiée puis évoluée (E) aura une vitesse de croissance au moins similaire à celle de la souche modifiée (M) initiale lorsque cultivée dans un milieu minimum en présence de glucose pour seule source de carbone. Dans un mode particulier on considérera la transformation de l'activité acyl-homosérine sulfhydrylase en activité « cystéine synthase évoluée » sera réputée acquise lorsque l'activité cystéine synthase portée par la protéine O-acyl-L-homosérine sulfhydrolase modifiée sera améliorée de 10% par rapport à son activité initiale. Dans certains cas, on peut observer cette amélioration par l'augmentation du taux de croissance de la bactérie (E) par rapport à celui de la bactérie (M). Enfin cette transformation de l'activité acyl-homosérine sulfhydrylase en activité « cystéine synthase évoluée » sera réputée acquise lorsque la souche (E) produira au moins autant de cystéine que la souche (M) dans des conditions de culture équivalentes, ne contenant pas de cystéine initialement.

Les souches selon l'invention peuvent également être génétiquement modifiées par inactivation, mutation et/ou suractivation d'au moins un gène endogène, la modification étant effectuée préalablement ou non à la mise en oeuvre de l'évolution de la souche modifiée. En particulier, les souches de microorganismes selon l'invention sont génétiquement modifiées afin de supprimer les gènes *cys*K et/ou *cys*M et/ou *met*B*,* codant les protéines portant respectivement les activités enzymatiques cystéine synthase A, cystéine synthase B et cystathionine gamma synthase. De manière préférentielle, les gènes *cys*K et *cys*M sont supprimés.

Le gène *cys*K code la cysteine synthaseA (GenBank NP_416909) et le gènes *cys*M code la cysteine synthase B (GenBank NP_416916). L'inactivation de ces gènes revient à supprimer les voies de biosynthèse de la cystéine et donc à rendre la souche auxotrophe pour la cystéine. Ceci permet de sélectionner les souches qui ont modifié l'activité acyl-homoserine sylfhydrylase en activité cystéine synthase afin de restaurer la production de cystéine.

On peut le cas échéant supprimer le gène codant l'activité cystathionine gamma-lyase.

Dans un mode de réalisation, ladite souche bactérienne peut comprendre également une modification de l'activité sérine O-acyltransférase porté par le gène *cys*E (figure 4) afin de lui conférer une insensibilité à la rétro-inhibition par la cystéine.

Dans un autre mode de réalisation, ladite souche bactérienne peut comprendre également une surexpression du gène *cys*B afin de déréguler la voie d'assimilation du soufre en H2S.

Il peut-être également avantageux pour la production d'acides aminés de formule (V), de préférence de la L-cysteine, de surexprimer le gène codant l'acetyl-CoA synthetase, comme le gène *acs* (EC 6.2.1.1), ayant le numéro d'accession AE000480, en même temps que le gène codant pour une enzyme à activité « cystéine synthase améliorée»défini précédemment (figure 4).

Il peut également être avantageux d'atténuer, voire de supprimer les gènes codant pour une acétate kinase et/ou pour une phosphotransacetylase , notamment les gènes *ack* et *pta* ayant respectivement les numéros d'accession AE000318 et AE000319, et codant respectivement une acétate kinase (EC 2.7.2.1) et une phosphotransacetylase (EC 2.3.1.8). Cette atténuation/suppression est de préférence combinée à une surexpression du gène codant l'acetyl-CoA synthetase.

Toutes ces modifications mentionnées ci-dessus peuvent être effectuées sur la souche modifiée préalablement au processus d'évolution du gène *met*Y ou sur la souche évoluée, c'est à dire postérieurement à l'évolution du gène *met*Y*,* à l'exception des délétions *cys*K et/ou *cys*M qui doivent être obligatoirement réalisées avant le processus de culture et d'évolution de la souche modifiée.

L'invention concerne donc également un procédé de préparation d'une souche bactérienne à activité «cystéine synthase évoluée » telle que définie précédemment, ledit procédé comprenant une étape consistant à cultiver dans un milieu de culture approprié comprenant une source de carbone simple et une souche bactérienne comprenant une enzyme catalysant une réaction de sulfhydrylation en présence d'H2S dont l'activité essentielle n'est pas une activité « cystéine synthase » telle que définie précédemment, afin de diriger une évolution du gène codant pour ladite enzyme dans ladite souche bactérienne, vers un gène codant pour une activité « cystéine synthase évoluée ».

De manière préférentielle et afin d'accélérer la sélection et l'évolution dirigée du gène codant pour l'enzyme catalysant une réaction de sulfhydrylation en présence d'H2S, on peut effectuer les opérations suivantes :

### a. Coupler la biosynthèse de la cystéine à la croissance du microorganisme de telle sorte que la production de cystéine soit nécessaire à une bonne croissance du microorganisme

Pour cette raison on peut faire le choix de déléter les gènes *cys*K*, cys*M et éventuellement *met*B et éventuellement le gène codant une activité cystationine gamma lyase (e.g. gène *yrh*B chez *B. subtilis),* afin de supprimer toutes voies de synthèse naturelle -de cystéine. Ce faisant la souche devient auxotrophe pour la cystéine.

Le microorganisme, pour vivre en milieu minimum contenant une source de carbone simple, doit donc optimiser l'activité cystéine synthase de l'O-acyl-L-homoserine sulfhydrylase, afin de rétablir la voie de synthèse de la cystéine à partir de d'acetylserine et d'H2S. Lorsque la délétion *met*B est réalisée il peut être nécessaire de supplémenter le milieu en méthionine.

### b. Supprimer les régulations, notamment les rétro-inhibitions soit au niveau des enzymes, soit au niveau des gènes afin que la voie de biosynthèse principale soit potentialisée

On peut ainsi surexprimer le gène *cys*B codant une protéine activatrice de la voie d'assimilation du soufre (WO01/27307), permettant ainsi l'optimisation de la production en H2S. Par ailleurs, il a été montré que la serine acetyl-transferase, codée par le gène *cys*E*,* était rétro-inhibée par la cysteine. Il est donc souhaitable de remplacer cette enzyme par une enzyme insensible à la rétro-inhibition (WO 97/15673 ; WO 02/061106) ou bien de surexprimer l'enzyme (WO 02/29029).

L'invention se rapporte également à un procédé de préparation de cystéine, dans lequel on cultive un microorganisme à activité « cystéine synthase évoluée » tel que défini précédemment dans un milieu de culture approprié, ledit milieu approprié comprenant une source de carbone simple telle que définie précédemment.

La définition des conditions de fermentation est du ressort de l'homme du métier. La fermentation est conduite en fermenteurs avec un milieu minéral de culture de composition connu défini et adapté en fonction des bactéries utilisées, contenant au moins une source de carbone simple.

Les milieux peuvent être supplémentés pour compenser les auxotrophies autres que celle provoquée par la délétion des gènes *cys*K et/ou *cys*M*;* ils contiennent une concentration en carbone simple adaptée en fonction du mode de culture et de production, ainsi que du composé soufré de formule (II) en concentration adaptée en fonction de l'évolution de la souche et du mode de production retenu.

Après fermentation, la cystéine est récupérée selon les méthodes usuelles, et le cas échéant, purifiée.

Les techniques de récupération puis de purification de la cystéine dans les milieux de culture sont bien connues de l'homme du métier.

La présente invention concerne aussi un procédé de préparation d'un acide aminé de formule générale (V) tel que défini prédédemment, caractérisé en ce que l'on fait réagir de l'acétylsérine avec une enzyme à activité « cystéine synthase évoluée » telle que définie précédemment, dans un milieu réactionnel approprié comprenant un composé soufré de formule générale (II) défini précédemment.

Le milieu réactionnel approprié est un milieu usuel de réaction enzymatique, bien connu de l'homme du métier, en particulier un milieu aqueux dans lequel les substrats et l'enzyme sont en solution ou en suspension. Les conditions de mise en oeuvre de la réaction sont bien connues de l'homme du métier, afin notamment d'éviter une dénaturation substantielle de l'enzyme.

Selon un mode particulier de réalisation de l'invention, l'enzyme à activité « cystéine synthase évoluée » est présente dans une bactérie inactivée ou dans un extrait cellulaire.

### D.III. Evolution des voies NADPH dépendantes.

Selon un autre mode préférentiel de réalisation de l'invention, on peut choisir de faire évoluer une voie métabolique utilisant le NADPH. Pour cela, la souche bactérienne initiale doit être modifiée de telle sorte que la vitesse de production du NADPH soit supérieure à sa vitesse d'oxydation en NADP+ (aussi écrit NADP) ce qui empêchera la croissance de la bactérie sur le milieux défini choisi pour mettre en oeuvre le procédé.

La présente invention concerne des souches de microorganismes modifiées pour permettre l'évolution de voies métaboliques consommatrices de NADPH. Les souches selon l'invention sont utilisables dans des procédés de biotransformation consommatrices de NADPH.

La présente invention concerne également un procédé de préparation de molécules par biotransformation comprenant la culture dans un milieu approprié d'une souche selon l'invention, ladite souche comprenant également les éléments génétiques nécessaires à la préparation de ladite molécule.

Les procédés de biotransformation ont été développés pour permettre la production de molécules en grande quantité à des coûts faibles, tout en permettant également la valorisation de différents sous-produits industriels ou de l'agriculture.

Pour produire des molécules d'intérêt par biotransformation *in vivo* on distinguera deux grandes approches :
- d'une part la fermentation qui permet la production de molécules par un microorganisme à partir d'une source de carbone simple (e.g. W00102547 qui décrit la production de lysine par fermentation de C. *glutamicum* en présence de glucose),
- d'autre part la bioconversion par un microorganisme d'un substrat donné différent de la source de carbone simple, qui est utilisée seulement pour produire la biomasse nécessaire, pour la production d'une molécule d'intérêt (*e.g.* WO0012745 qui décrit la production de dérivés R-pipéridine, WO0068397 qui décrit la production de Tagatose).

L'amélioration d'un procédé de biotransformation peut porter sur différents facteurs comme la température, l'oxygénation, la composition du milieu, le procédé de récupération, etc. On peut aussi envisager de modifier le microorganisme de telle sorte que la production de la molécule d'intérêt et/ou son excrétion soit augmentée.

Dans le cadre d'une fermentation on s'attachera par exemple à optimiser la voie de biosynthèse, par exemple en modifiant la régulation des gènes ou en modifiant le gène afin de modifier les caractéristiques enzymatiques ou encore en optimisant la régénération des co-substrats.

Dans le cadre de la bioconversion on s'attachera à améliorer les caractéristiques cinétiques des enzymes, à réduire la formation de co-produits et à optimiser la régénération de co-substrats impliqués dans la ou les étapes de bioconversion.

Parmi les co-substrats impliqués dans les biotransformations, le NADPH prend une part importante notamment pour la production des acides aminés (e.g. arginine, proline, isoleucine, méthionine, lysine), de vitamine (*e.g.* panthoténate, phylloquinone, tocophérol), de molécules aromatiques (e.g. WO9401564), ou d'autres molécules à haute valeur ajoutée.

La présente invention concerne des souches de microorganismes modifiés pour permettre l'évolution des enzymes ou de voies métaboliques consommatrices de NADPH.

Pour la création de tels microorganismes, les inventeurs ont opté pour des modifications qui augmentent la vitesse de production du NADPH et diminue sa vitesse d'oxydation en NADP+, lesdits microorganismes modifiés étant ensuite employés pour faire évoluer des enzymes ou des voies métaboliques consommatrices de NADPH. Ces bactéries pouvant aussi être judicieusement utilisées dans des procédés de production par biotransformation, de molécules issues de voies de synthèse NADPH dépendantes.

L'optimisation du NADPH est décrite ci-après pour *E. coli.* Le même principe peut être appliqué de manière similaire à tous les microorganismes cultivés en conditions aérobies.

Les souches modifiées selon l'invention comprennent une délétion du gène *udh*A et/ou du gène *qor.* Selon un mode préférentiel de réalisation de l'invention, les gènes *udhA* et *qor* sont tous deux délétés.

Selon un mode particulier de réalisation de l'invention, la souche modifiée selon l'invention comprend également une délétion d'un gène choisi parmi *pgi* ou *pfk*A et/ou *pfk*B*.*

Les gènes ci-dessus sont bien connus de l'homme du métier et décrits dans la littérature scientifique, notamment pour *E. coli:*

### Gènes et références dans E. coli :

*udh*A *:* X66026 soluble pyridine transhydrogenase;
*qor :* L02312 quinone oxidoreductase;
*pgi :* X15196 phosphoglucose isomerase (EC 5.3.1.9);
*pfk*A : X02519 phosphofructokinase-1;
*pfk*B: K02500 phosphofructokinase-2.

La présente invention a également pour objet un microorganisme évolué, modifié pour la production de NADPH telle que définie ci-dessus et ci-après, lequel comprend également, un ou plusieurs gènes codant des enzymes NADPH dépendantes, impliquées dans la biotransformation d'une molécule d'intérêt, que l'on souhaite faire évoluer, ainsi qu'un ou plusieurs gènes marqueurs de sélection.

Ces gènes peuvent être natifs de la souche modifiée selon l'invention ou encore introduits dans la souche optimisée selon l'invention par transformation avec un vecteur approprié, soit par intégration dans le génome du microorganisme ou encore par un vecteur réplicatif, ledit vecteur approprié portant un ou plusieurs gènes codant pour lesdites enzymes impliqués dans la bioconversion de ladite molécule d'intérêt ou lesdits marqueurs de sélection.

Ces gènes comprennent une séquence d'acide nucléique codant pour une enzyme impliquée dans la biotransformation de la molécule d'intérêt et/ou pour un marqueur de sélection, la séquence codante étant fusionnées à des séquences promotrices efficaces dans la cellule procaryote et/ou eucaryote choisie pour la biotransformation. Le vecteur (ou plasmide) peut-être un vecteur navette entre *E. coli* et un autre microorganisme.

La présente invention concerne également un procédé de préparation des souches modifiées selon l'invention telle que définies ci-dessus et ci-après, dans lequel on délète les gènes *udhA* et/ou *qor,* et le cas échéant les gènes *pgi* ou *pfk*A et/ou *pfk*B.

Selon un mode particulier de réalisation de l'invention, le procédé de préparation de souches selon l'invention comprend également la transformation des souches modifiées avec au moins un vecteur approprié comprenant un ou plusieurs gènes codant une ou plusieurs enzymes consommatrices de NADPH et impliquées dans la biotransformation d'une molécule d'intérêt, ainsi qu'un ou plusieurs gènes marqueurs de sélection. Le vecteur permet soit la réplication des gènes soit leur intégration dans le chromosome de la bactérie modifiée. La transformation de la souche par le vecteur défini ci-dessus peut être réalisée sur la souche modifiée ou préalablement à la modification de la souche selon l'invention.

La souche modifiée pour la production de NADPH est obtenue par biologie moléculaire.

Un autre aspect de l'invention concerne l'utilisation de ces souches modifiées selon l'invention pour faire évoluer des enzymes NADPH dépendantes permettant ainsi d'améliorer leurs caractéristiques cinétiques, d'élargir ou de réduire leur spécificité de substrat, et permettant *in fine* de créer une nouvelle voie métabolique et/ou d'améliorer les rendements de biotransformation. Un autre aspect de l'invention concerne l'utilisation de la souche modifiée ou de la souche évoluée pour réaliser des biotransformations consommatrices de NADPH avec des rendements de biotransformations supérieurs à ceux obtenus dans une souche non modifiée et/ou non-évoluée.

L'invention concerne aussi un procédé de production d'une molécule d'intérêt ayant une biosynthèse consommatrice de NADPH caractérisé en ce qu'il comprend les étapes suivantes :
a) mise en culture des microorganismes évolués selon l'invention dans un milieu de culture approprié favorisant leur croissance et comprenant les substances nécessaires pour la réalisation de la biotransformation par fermentation ou bioconversion, à l'exception du NADPH, et
b) extraction de la molécule d'intérêt du milieu et le cas échéant purification.

De manière préférentielle, la molécule d'intérêt est choisie parmi la cystéine, la méthionine, le 3-hydroxypropionate, l'hydrocortisone, le xylitol et le glycérol.

Pour une réaction de bioconversion le procédé comprend aussi l'ajout du substrat à convertir dans le milieu de culture approprié.

Le milieu de culture cité à l'étape b) du procédé selon l'invention défini ci-dessus comprend au moins un hydrate de carbone assimilable choisi parmi différents sucres assimilables, tels le glucose, le galactose, le saccharose, le lactose, ou les mélasses, ou les sous-produits de ces sucres. Une source de carbone simple tout particulièrement préférée est le glucose. Une autre source de carbone simple préférée est le saccharose. Le milieu de culture peut en outre contenir une ou plusieurs substances (e.g. acides aminés, vitamines, sels minéraux, ...) favorisant la production de la molécule d'intérêt.

Les exemples ci-après permettent d'illustrer l'invention, sans toutefois chercher à en limiter la portée.

### E. Description des figures

Figure 1 : synthèse de la méthionine à partir de l'homosérine, chez les bactéries.
Figure 2 : Schéma de synthèse de la méthionine selon l'invention appliqué dans *E. coli ;* une stratégie équivalente est transposable chez de nombreux microorganismes dont *C. glutamicum.* Les stratégies *met*B*** ou *met*Y**** nécessitent l'utilisation d'une souche initialement au moins Δ(*met*E) tandis que les stratégies *met*B**** ou *met*Y*** nécessitent l'utilisation d'une souche initialement au moins Δ*(met*C*).*

### Légende :

MetA : homosérine succinyltransferase ; pourra être remplacé par une isoforme insensible à la rétro-inhibition par la méthionine ou par une isoforme homoserine acetyltransferase éventuellement insensible à la rétro-inhibition par la méthionine.
MetB : cystathionine γ-synthase
MetB* : cystathionine γ-synthase évoluée en « méthionine synthase »
MetB** : cystathionine γ-synthase évoluée en homocysteine synthase
MetY* : O-acetyl-homosérine (de *C. glutamicum*) évoluée en homocysteine synthase
MetY** : O-acetyl-homosérine (de *C. glutamicum)* évoluée en « méthionine synthase »

La voie centrale représente la voie naturelle de synthèse de la méthionine chez *E. coli.* Les autres voies indiquées correspondent aux procédés selon l'invention.

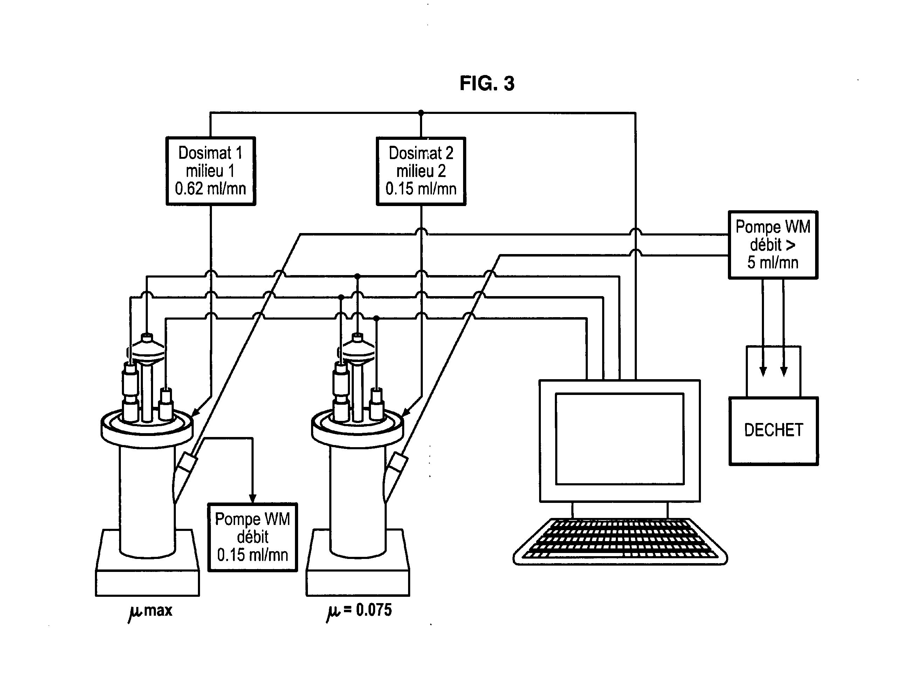

Figure 3 : représentation d'un mécanisme de fermentation continue pour la sélection dirigée des souches selon l'invention. Nous utilisons par exemple la technologie « Fedbatch-Pro » de la société DASGIP. Il s'agit d'un système modulaire contrôlé par ordinateur permettant la fermentation en parallèle de microorganismes en ayant un control de l'alimentation en milieu, en pH et pO2.
Figure 4 : synthèse de la cystéine à partir de la sérine, chez les bactéries.

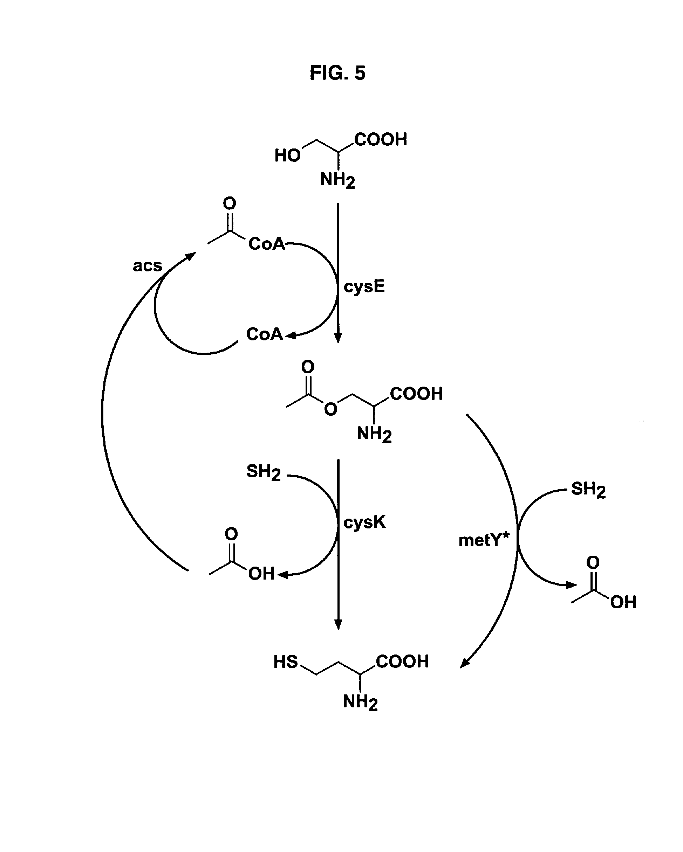

Figure 5 : stratégie pour obtenir une synthèse de cystéine à partir d'O-acetyl-L-sérine. La flèche rouge correspond à l'activité cystéine synthase portée par l'enzyme codée par le gène *met*Y évolué selon l'invention *(met*Y*^{*}).*

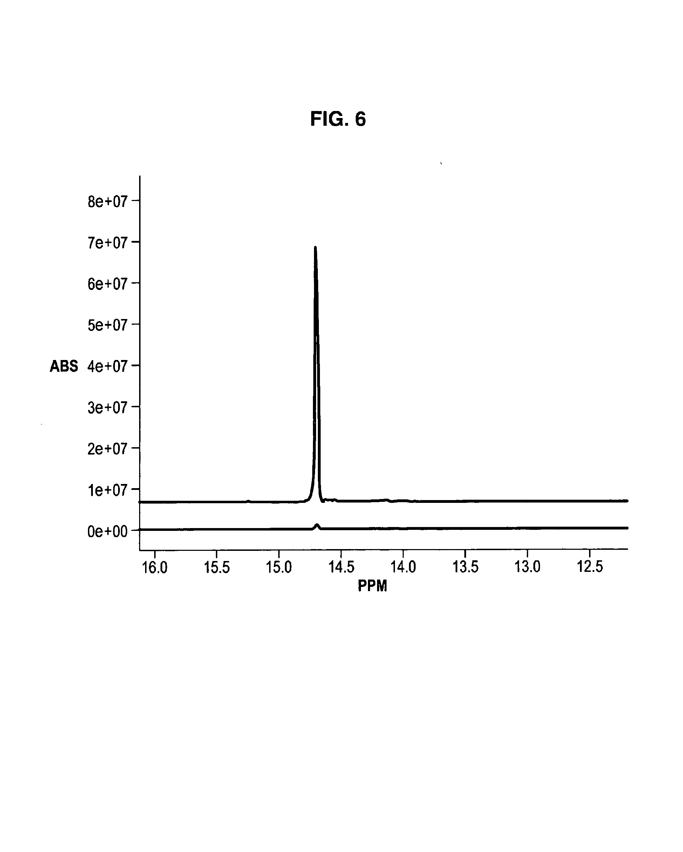

Figure 6 : Comparaison de spectres de ¹³C-RMN, correspondant au carbone 5 de la méthionine, obtenus par HSQC sur un hydrolysat de la souche sauvage (haut) ou de la souche K1a-F optimisée (bas). On observe que le carbone 5 de la souche K1a-F n'est pas marqué au carbone 13 ce qui confirme qu'il provient du méthylmercaptide de sodium
Figure 7: Alignement de séquences non modifiées de cystathionine-γ-synthases. Alignement réalisé avec l'algorithme MULTALIN (http://prodes.toulouse.inra.fr/multalin/cgi-bin/multalin.pl)

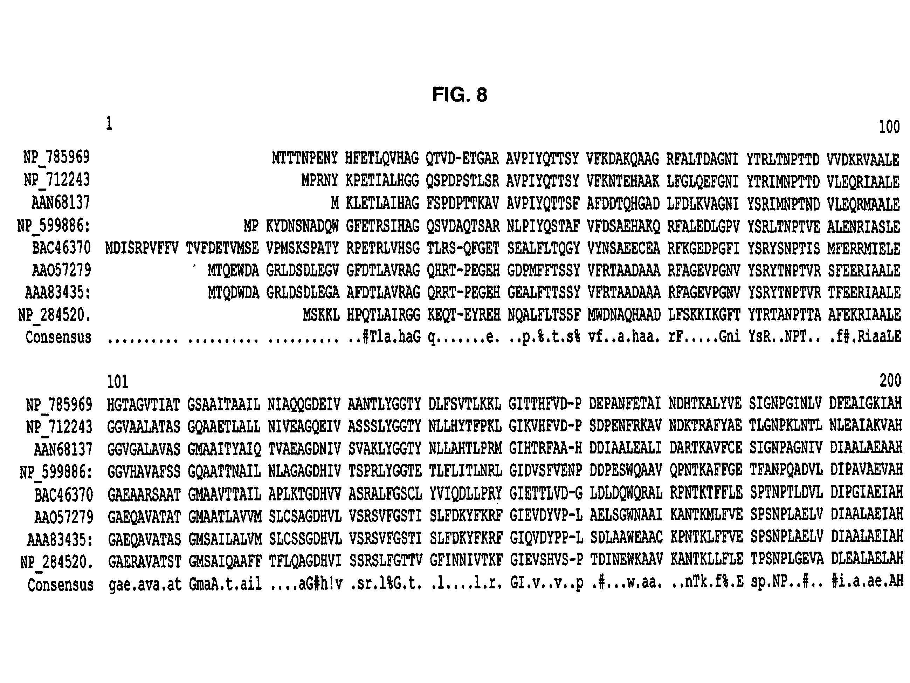

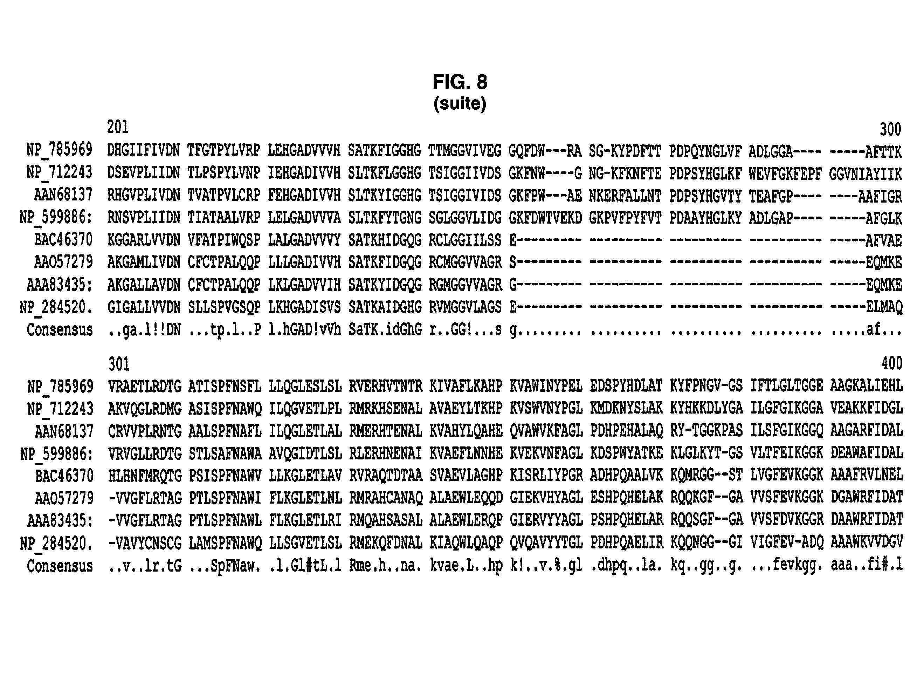

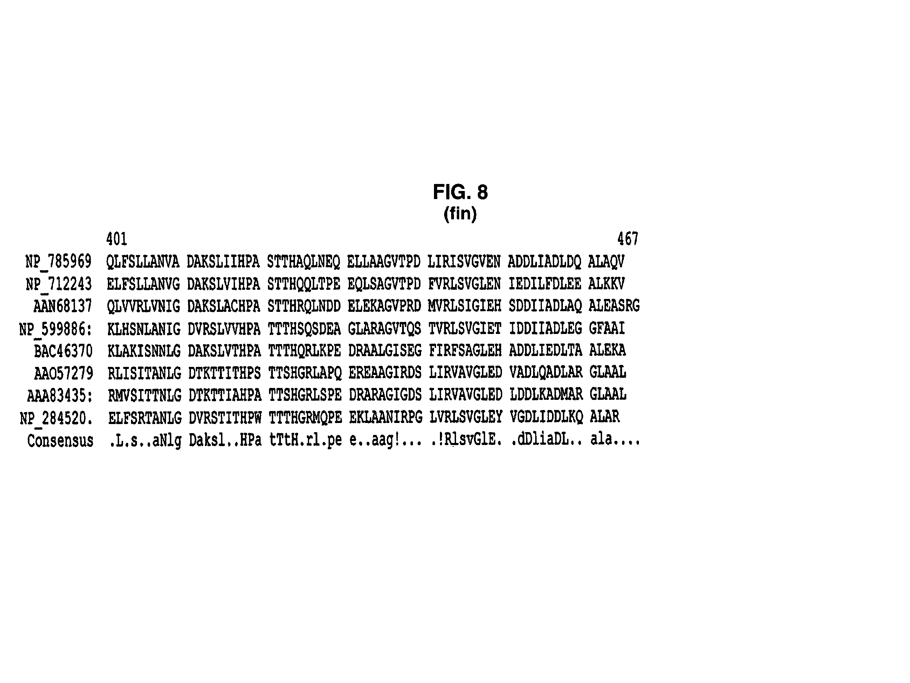

Figure 8 : Alignement de séquences non modifiées de acylhomosérine sulfhydrylases. Alignement réalisé avec l'algorithme MULTALIN.

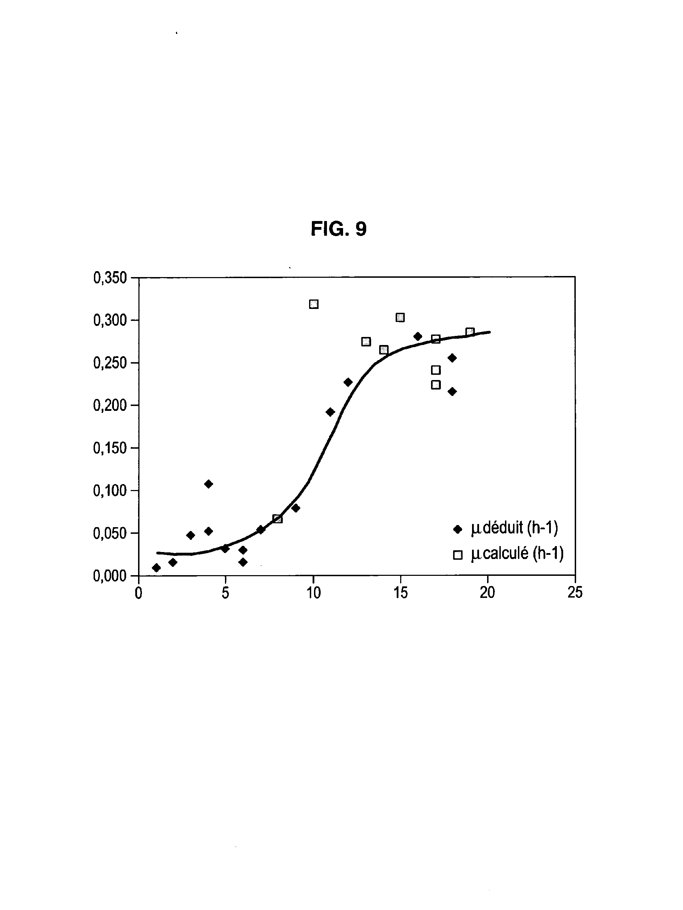

Figure 9: Evolution du taux de croissance de la souche initiale *E. coli* Δ(*met*E) lors du processus de sélection dirigée en batch. Abscisse : repiquage N° ; Flèche : Population K144 ; µ déduit : valeurs obtenues à partir de 2-3 valeurs de DO ; µ calculé : valeurs obtenues avec plus de 4 valeurs de DO.

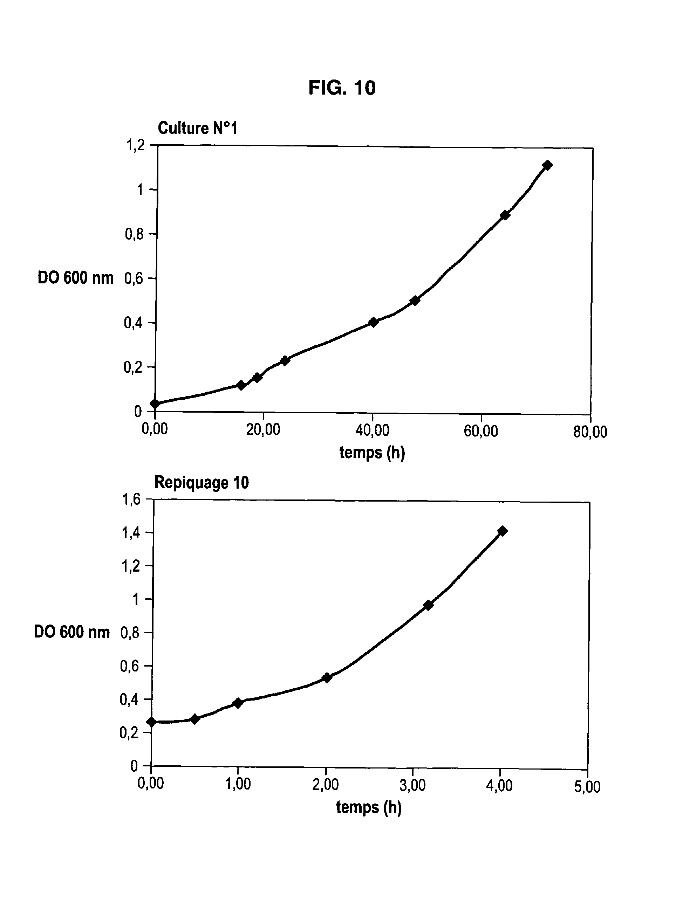

Figure 10 : Cinétique de croissance de la population E. *coli* Δ*met*C lors de son ensemencement initial (culture N°1) et lors de son dixième repiquage (Repiquage 10).

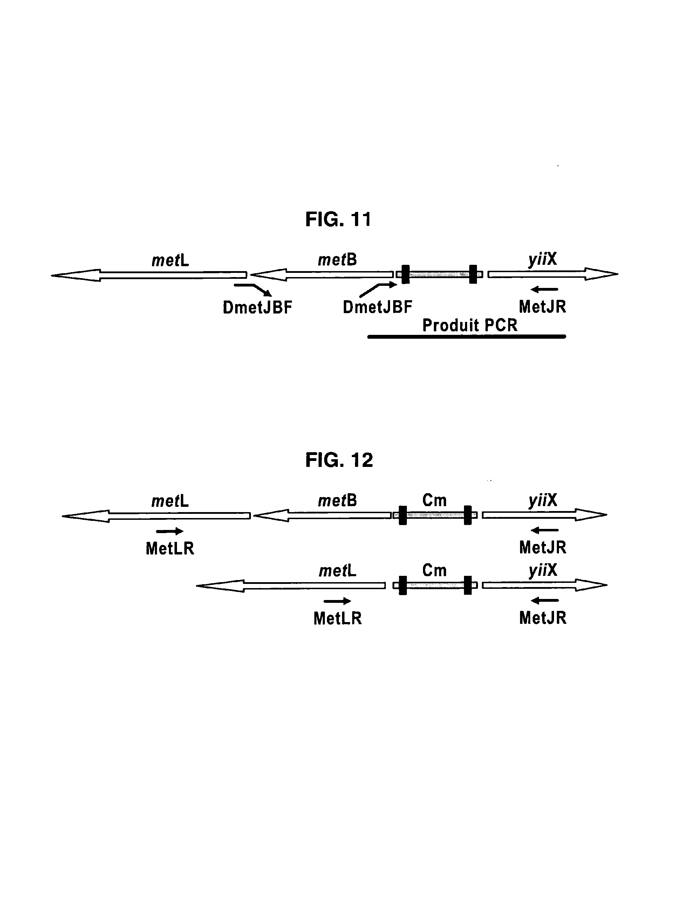

Figure 11: Réalisation de la PCR sur la souche modifiée *E. coli* Δ*met*J ::Cm en utilisant les amorces DmetJBF et MetJR. L'extrémité 5' de l'amorce DmetJBF est aussi capable de s'hybrider à une séquence située dans la partie 3' du gène *met*L*.*
Figure 12: Souches obtenues à l'issue de la recombinaison homologue avec le fragment amplifié par PCR (voir Figure 11); il est possible d'avoir deux évènements différents de recombinaison homologue, chacun ayant la même probabilité de se produire. Dans le premier cas, on recrée une souche *E*. *coli* Δ*met*J ::Cm alors que dans le second cas, on crée la souche *E*. *coli* [Δ*met*J *,* Δ*met*J ::Cm] en replaçant le promoteur de l'opéron *met*BLF devant *met*L*.*

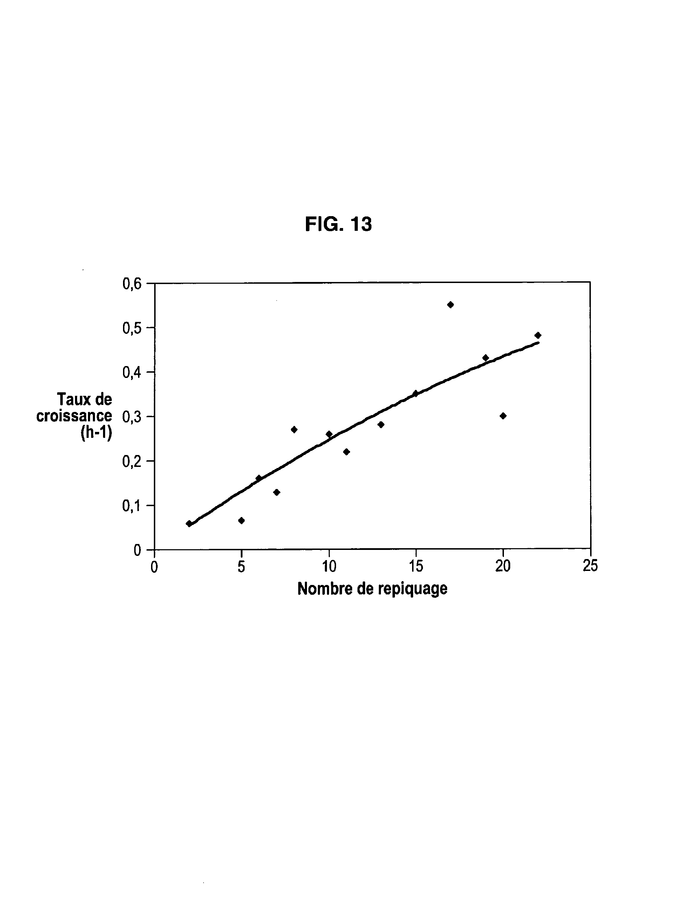

Figure 13: Evolution du taux de croissance de la souche *E. coli* [Δ*(met*BJ*, met*C*)* pTrc-*met*Y] sur un milieu défini (MML8), contenant du glucose pour seule source de carbone.

### F. Exemples

### F.I. Voie de Biosynthèse de la méthionine.

Une première application (Exemple F.I.1.) de l'invention au génie métabolique de la voie de biosynthèse de la méthionine comprend les étapes suivantes :
a) Délétion du gène *met*E dans la souche initiale *d'E.coli ;* la souche modifiée obtenue est donc auxotrophe à la méthionine. La souche initiale est capable de croître sur un milieu minimum (MM) dépourvu de méthionine, S-adénosylméthionine, homocystéine, cystathionine alors que la souche modifiée a perdu cette capacité.
b) Culture de la souche ci-avant modifiée sur le même milieu minimum (MM) auquel on ajoute le methylmercaptide de sodium (co-substrat) afin de faire évoluer une activité enzymatique endogène en activité méthionine-synthase afin de compenser l'activité enzymatique initialement délété (MetE).
c) Sélection d'une souche évoluée ayant une nouvelle activité méthionine-synthase en présence de methylmercaptide de sodium. La souche étant alors caractérisée.
d) Isolement du gène évolué codant la protéine ayant une activité enzymatique évoluée, en l'occurrence la cystathionine-γ-synthase ayant une activité méthionine-synthase améliorée.

Une deuxième application (Exemple F.I.2.) de l'invention, au génie métabolique de la voie de biosynthèse de la méthionine, comprend les étapes suivantes :
a) Délétion du gène *met*C dans la souche initiale *d'E.coli ;* la souche modifiée obtenue est donc auxotrophe à la méthionine. La souche initiale est capable de croître sur un milieu minimum (MM) dépourvu de méthionine, S-adénosylméthionine, homocystéine, cystathionine alors que la souche modifiée a perdu cette capacité.
b) Culture de la souche ci-avant modifiée sur le même milieu minimum (MM) en absence de tout co-substrat afin de faire évoluer une activité enzymatique endogène en activité homocystéine-synthase afin de compenser l'activité enzymatique initialement délété (MetC).

Une troisième application (Exemple F.I.3.) de l'invention à l'évolution de la voie de biosynthèse de la méthionine comprend les étapes suivantes :
a) Délétion des gènes *met*C*, met*B*, met*J dans la souche initiale d*'E.coli ;* la souche modifiée obtenue est donc auxotrophe à la méthionine. La souche initiale est capable de croître sur un milieu minimum (MM) dépourvu de méthionine, S-adénosylméthionine, homocystéine, cystathionine alors que la souche modifiée a perdu cette capacité.
al) Introduction du gène *met*Y*,* un gène hétérologue issu de *C. glutamicum.* Ce gène étant destiné à évoluer d'une activité acetylhomosérine sulfhydrylase en une activité méthionine-synthase.
b) Culture de la souche modifiée *E. coli* [*met*Y Δ*(met*B*, met*C*, met*J)] sur le même milieu minimum (MM) auquel on ajoute le methylmercaptide de sodium (co-substrat) afin de faire évoluer une activité enzymatique endogène en activité méthionine-synthase afin de compenser les activités enzymatiques initialement délétées (MetB, MetC).
c) Sélection d'une souche évoluée ayant une nouvelle activité méthionine-synthase en présence de methylmercaptide de sodium.

### Exemple F.I.1 Obtention d'une souche évoluée ayant une activité méthionine-synthase en présence de methylmercaptide de sodium.

### a) Construction de la souche modifiée E. coli Δ(metE)

L'inactivation du gène *met*E est réalisée en insérant une cassette de résistance à l'antibiotique chloramphénicol tout en délétant la majeure partie du gène concerné. La technique utilisée est décrite par Datsenko, K.A. ; Wanner, B.L. (2000) One-step inactivation of chromosomal genes in Escherichia coli K-12 using PCR products. Proc. Natl. Acad. Sci. USA 97 : 6640-6645.

Deux oligonucléotides sont utilisés pour réaliser la stratégie:
- DmetER de 100 bases (SEQ ID NO 1): 
taccccccgacgcaagttctgcgccgcctgcaccatgttcgccagtgccgcgcgggtttctggccagccgcgc
gttttcagCATATGAATATCCTCCTTAG
 avec :
   une région (caractères minuscules) homologue à la séquence (4012903 à 4012824) du gène *met*E (séquence 4010643 à 4012904, séquence de référence sur le site http://genolist.pasteur.fr/Colibri/)
   une région (caractères majuscules) pour l'amplification de la cassette de résistance au chloramphénicol du plasmide pKD3 (Datsenko, K.A. ; Wanner, B.L. (2000) One-step inactivation of chromosomal genes in Escherichia coli K-12 using PCR products. Proc. Natl. Acad. Sci. USA 97 : 6640-6645)
- DmetEF de 100 bases (SEQ ID NO 2): 
tgacaatattgaatcacaccctcggtttccctcgcgttggcctgcgtcgcgagctgaaaaagcgcaagaaagt
tattggTGTAGGCTGGAGCTGCTTCG
 avec :
   une région (caractères minuscules) homologue à la séquence (4010644 à 4010723) du gène *met*E
   une région (caractères majuscules) pour l'amplification de la cassette de résistance au chloramphénicol portée par le plasmide pKD3

Les oligonucléotides DmetER et DmetEF sont utilisés pour amplifier la cassette de résistance au chloramphénicol à partir du plasmide pKD3. Le produit PCR obtenu est alors introduit par électroporation dans la souche MG1655 (pKD46) dans laquelle l'enzyme Red recombinase exprimée permet la recombinaison homologue. Les transformants résistants à l'antibiotique sont alors sélectionnés et l'insertion de la cassette de résistance est vérifiée par une analyse PCR avec les oligonucléotides metER et metEF.
MetER (SEQ ID NO 3) : ggtttaagcagtatggtgggaagaagtcgc (homologue à la séquence de 4012978 à 4012949)
MetEF (SEQ ID NO 4) : cccggggatgaataaacttgccgccttccc (homologue à la séquence de 4010567 à 4010596)

La cassette de résistance au chloramphénicol peut ensuite être éliminée. Le plasmide pCP20 porteur de la recombinase FLP agissant au niveau des sites FRT de la cassette de résistance au chloramphénicol, est alors introduit dans les souches recombinantes par électroporation. Après une série de culture à 42°C, la perte de la cassette de résistance à l'antibiotique est vérifiée par une analyse PCR avec les mêmes oligonucléotides que ceux utilisés précédemment.

### b) Culture et évolution de la souche modifiée Δ(metE) en présence de methylmercaptide de sodium pour co-substrat

Pour optimiser *E. coli* pour la production de méthionine à partir du méthyl-mercaptan, on effectue une sélection dirigée en flacons.

La souche *E. coli* rendue auxotrophe pour la méthionine en inactivant le gène *met*E (voir ci-avant) ne peut donc croître qu'en fabriquant sa propre méthionine, par l'utilisation du méthyl-mercaptan.

La mise en oeuvre de cette technique permet la sélection d'une souche de *Escherichia coli* dont la cystathionine-γ-synthase (EC 4.2.99.9) a développé une activité « méthionine-synthase » modifiée en présence de méthyl-mercaptan.

La sélection dirigée est conduite en flacon en verre hermétiquement fermé contenant 50 ml de milieu minéral (Schaefer et al., 1999, Anal. Biochem. 270 : 88-96) en présence de 33 mM glucose, et du chloramphénicol à une concentration finale de 25 mg/1.

Les milieux de culture sont ensemencés avec la souche *E. coli* K12 Δ*met*E à DO₆₀₀ₙₘ définie. On ensemence avec une population de bactéries suffisamment importante pour que certaines bactéries possèdent potentiellement des mutations spontanées intéressantes dans le gène *met*B*,* permettant d'assimiler le méthyl-mercaptan. Cette population a été obtenue par culture de la souche auxotrophe en méthionine sur milieu minimum supplémenté en méthionine.

Trois flacons reçoivent alors 100 µl d'une solution à 400 mg/1 de sodium mercaptide, tandis qu'un quatrième flacon ne reçoit pas de sodium mercaptide. Les cultures sont réalisées sous agitation, à 37°C, pendant 6 jours, puis la DO₆₀₀ₙₘ est mesurée. Les résultats sont résumés dans le Tableau N°2 ci-dessous.

**Tableau 2. Mesure de la densité optique de milieux de cultures pour E. coli en présence (flacons 1-3) ou absence (flacon témoin) de sodium mercaptide.**

| | Flacon 1 | Flacon 2 | Flacon 3 | Flacon Témoin |
|---|---|---|---|---|
| DO₆₀₀ₙₘ à T=0 | 0.34 | 0.34 | 0.34 | 0.34 |
| DO₆₀₀ₙₘ à T=6 jours | 0.23 | 1.14 | 0.79 | 0.32 |

Ces résultats montrent que les flacons 2 et 3 ont permis de multiplier une souche capable d'utiliser le méthyl-mercaptan pour produire la méthionine nécessaire à sa croissance (augmentation de la densité optique).

L'activité « méthionine synthase » améliorée observée provient d'une modification dans le gène de la cystathionine y-synthase de la souche *E. coli* K12 Δ*met*E*,* contenue dans les flacons 2 et 3.

La population bactérienne du Flacon 2 peut alors être utilisée pour améliorer davantage l'activité « méthionine synthase » en présence de méthyl-mercaptan, en utilisant un procédé de criblage et amélioration par fermentation en étage, ou en recommençant le procédé en flacon tel qu'il vient d'être décrit.

### c) Criblage et amélioration par fermentation en étage

Pour optimiser *E. coli* pour la production de méthionine à partir du méthyl-mercaptan, on effectue une sélection.

La mise en oeuvre de cette technique permet la sélection d'une souche de *Escherichia coli* dont la cystathionine-γ-synthase (EC 4.2.99.9) a développé une activité « méthionine-synthase » améliorée en présence de méthyl-mercaptan.

Alternativement, on peut utiliser une souche obtenue selon l'exemple 2.

La sélection dirigée est conduite dans un système en continu en étage (Figure 3).

Le premier fermenteur produit les bactéries à une vitesse proche du taux de croissance maximum. Les bactéries passent en continu de ce fermenteur dans un second fermenteur caractérisé par un taux de dilution plus faible et un milieu avec le crible de sélection (ici, le méthyl-mercaptan).

La pression de sélection, imposée à la bactérie dans le second fermenteur, est établie par la concentration en méthyl-mercaptan. Des cycles successifs de sélection permettent d'appliquer aux bactéries des cribles de plus en plus fort par des concentrations croissantes en méthyl-mercaptan.

Pour chaque concentration, la souche sélectionnée dans le second fermenteur est celle qui a évolué pour métaboliser la totalité du méthyl-mercaptan (méthyl-mercaptan résiduel nul dans le fermenteur).

Dans ce cas, on recommence la sélection en utilisant le fermenteur n°2 comme fermenteur de croissance et le fermenteur n°1 comme fermenteur de crible, présentant du méthyl-mercaptan de concentration plus forte qu'à l'étape précédente.

On effectue différents cycles de sélection pour obtenir une souche fermentant le méthyl-mercaptan avec une vitesse élevée. L'analyse de cette souche permet de définir les mutations dans le gène de la cystathionine-γ-synthase.

### d) Sélection de la souche évoluée en présence de methylmercaptide de sodium.

La population d'*E*. *coli* Δ(*met*E) issue du flacon 2 subi des repiquages successifs en flacons, permettant d'obtenir la population Kla-F.

La nouvelle population obtenue Kla-F est mise en culture dans un milieu minimum (Schaefer et al., 1999, Anal. Biochem. 270 : 88-96) contenant 2,5 g.l⁻¹ de glucose entièrement marqué au carbone 13 et du méthylmercaptide de sodium (200 ppm) non enrichi en carbone 13. Cette population est auxotrophe pour la méthionine en l'absence de méthylmercaptide de sodium.

Après la culture, les cellules sont récupérées, lavées puis hydrolysées par HCI 6N pendant 24 heures à 107°C. Une analyse par RMN bidimensionnelle est alors réalisée (HSQC). Cette analyse permet d'étudier le carbone 5 de la méthionine, qui provient, soit de la L-cystéine, produite à partir du glucose présent dans la solution (voie classique), soit du méthylmercaptide de sodium lorsque la nouvelle voie métabolique selon l'invention est utilisée.

L'expérience est conduite de manière similaire avec la souche sauvage *E. coli* K12 (produisant la méthionine à partir du glucose), en absence de méthylmercaptide de sodium.

La figure 6 montre deux spectres 1D, issus de deux acquisitions distinctes, superposés pour une meilleure lecture. Ces spectres 1D sont extraits de spectres RMN à deux dimension type HSQC (corrélation entre protons et carbone 13). Les spectres RMN à deux dimensions ont été obtenus sur un hydrolysat acide des bactéries.

L'échantillon analysé est un hydrolysat total ; cependant du fait de la sensibilité de la RMN et des temps d'acquisition utilisés, on détecte essentiellement les acides aminés, les sucres, les bases et le glycérol. Chaque carbone (couplé à un proton) de chaque acide aminé donne une résonance magnétique nucléaire.

Le carbone 5 de la méthionine (c'est à dire le groupe méthyl terminal) présente un déplacement chimique d'environ 14,7 ppm. La figure 6 présente la zone de déplacement chimique centrée autour de 14,7 ppm pour les deux souches.

On remarque que dans le cas du spectre supérieur, le signal du carbone 5 est fort, indiquant que le carbone 5 est marqué au carbone 13. En conséquence, ce carbone 5 provient du glucose marqué introduit comme substrat dans le milieu de culture.

On remarque par contre que le même signal est très faible dans le spectre inférieur (souche Kla-F). Cela signifie que le carbone 5 n'est pratiquement pas marqué. Pourtant les autres carbones de la molécule sont fortement marqués (résultats non présentés). Le carbone 5 non marqué ne provient donc pas du glucose mais du méthyl-mercaptan.

On peut donc conclure que la souche Kla-F produit de la méthionine à partir de succinyl-L-homosérine et de méthylmercaptide de sodium.

La population Kla-F subit 14 nouveaux cycles de repiquage successifs en flacon. On obtient ainsi la population K144 (figure 9) que l'on étale alors sur milieu minimum gélosé contenant du glucose pour seule source de carbone. Les boites inoculées sont placées en condition aerobie dans une jarre anaérobie dans laquelle est introduit un tube contenant du méthylmercaptide de sodium dissout dans de l'eau, la jarre est alors placée dans un incubateur à 37°C. La température d'ébullition du methylmercaptide de sodium étant de 5°C, l'atmosphère de la jarre anaérobie s²enrichie en methylmercaptan. Après 4 jours, les clones apparaissent sur les boites ; ils correspondent aux bactéries capables de produire de la méthionine en présence de méthylmercaptan. Dix clones sont isolés, dont le clone K176. Le clone K176 est multiplié en culture liquide et des stocks glycérols sont réalisés portant le numéro K183.

Pour le clone K183 et la souche *E. coli* K12 Δ(*met*E) initiale, la séquence des gènes *met*J et *met*B (SEQ ID N°5) a été déterminée. La séquence obtenue pour *met*B évoluée, appelée *met*B*^{*}* (SEQ ID N°7) permet d'observer la présence d'une alanine en position 325 (SEQ ID N°8) en remplacement d'un glutamate (SEQ ID N°6). Le gène *met*J ne présente aucune mutation. Cette souche a été déposée à la CNCM le 2 avril 2003, sous le numéro 1-3005.

### • Caractérisation du clone K183

Le clone K183 est cultivé en flacon, en milieu minimum avec glucose et methylmercaptide de sodium pour seule source de carbone. En parallèle, une culture est réalisée dans des conditions identiques avec la souche *E. coli* K12 sauvage. On observe que la consommation de glucose par g de biomasse est deux fois plus importante que pour une souche de *E*. *coli* sauvage (MBM01). La surconsommation est probablement due en partie à la production d'acétate.

**Tableau 3. Comparaison du rendement de biomasse entre la souche E. coli sauvage et le clone évolué K183 :**

| **Souches** | **Rendement Biomasse** | **Rendement Acétate** |
|---|---|---|
| MBM01 | 0.45 | <0.002 |
| K183 | 0.24 | 0.36 |

| | | |
|---|---|---|
| Rendement Biomasse exprimé en g_{(biomasse)}/g_{(glucose)}. Rendement Acétate exprimé en g_{(acetate)}/g_{(glucose)}. | | |

L'analyse des métabolites intracellulaires et extracellulaire de ces deux cultures montre notamment :

En intracellulaire, une augmentation de l'alanine, du pyruvate, du ketobutyrate et de 2 ketoisocaproate et une diminution de la concentration en tryptophane, norvaline, norleucine, leucine,et methionine.

En extracellulaire, une accumulation de glutamate, d'isoleucine, thréonine, valine et 2-ketoisocaproate et une diminution de pyruvate, norleucine, tryptophane.

### • Caractérisation de l'activité spécifique « méthionine synthase » des souches MBM01 et K183 en présence de méthylmercaptan.

Afin de montrer l'amélioration de l'activité méthionine synthase dans la souche K183 par rapport à la souche sauvage (MBM01), des réactions enzymatiques sont réalisées en utilisant des extraits acellulaires préparés à partir de cultures des souches K183 et MBM01 réalisées sur milieu riche (milieu BH1, commercialisé par DIFCO, avec 2,5 g/L de glucose) en absence de méthylmercaptan. Les extraits protéiques sont désalés sur PD10 et réservés sur glace.

### Conditions réactionnelles et traitement de l'échantillon

- Préparer sur la glace une solution de sodium methanethiolate diluée 10 fois (100 µl de solution 3M plus 900 µl d'eau mQ)
- Préparer sur la glace les mélanges réactionnels constitués de 20µL de tampon phosphate 500 mM pH 6.5, 10µl Pyridoxal phosphate 2,5 mM, 16 µL O-succinylhomosérine 25 mM, 10 µL Sodium methanethiolate 0,3 M, 24 µL eau milliQ.
- Placer les tubes à 37°C (thermomixer sous la hotte) et ajouter l'extrait protéique (20 µl) pour démarrer la réaction.
- Pour arrêter la réaction (0 à 30 minutes), placer les tubes sur glace et ajouter 400 µl d'acétone à -20°C.
- placer les tubes à -20°C pendant 30 minutes
- puis ouvrir les tubes sous la hotte pendant 10 minutes pour évaporer le méthanethiol et l'acétone (les maintenir sur glace)
- centrifuger 5 minutes à 10000 g , transvaser le surnageant (∼100 µL) dans des tubes éppendorf et diluer pour un volume final de 1 mL.

### Mesure de l'activité méthionine synthase en détectant la quantité de succinate libéré du succinylhomosérine :

- Dix µl de l'échantillon ci-dessus obtenu sont analysés par chromatographie ionique en utilisant un Appareil Dionex DX-500 équipé d'une précolonne AG-11 1 2 mm et d'une colonne AS-11 2 mm, d'un suppresseur ASRS Ultra, d'une boucle injection 10 µl. Un gradient est alors appliqué : 0 - 7 min 0,5 mM KOH ; 7 min injection ; 7 - 9,5 min 0,5 mM KOH ; 9,5 - 13 min 0,5 - 5 mM KOH ; 13 - 25 min 5 - 38,3 mM KOH.

### Mesure de l'activité méthionine synthase en détectant la quantité de méthionine synthétisée en présence de methylmercaptan

L'analyse est réalisée par GC-MS ce qui nécessite de silyler les échantillons préalablement à l'injection. Pour cela chaque échantillon reçoit un standard interne (serine13C) qui permet de valider la qualité de la silylation. Les échantillons sont ensuite lyophilisés pendant la nuit.

La dérivation est réalisée en appliquant le protocole suivant :

Ajouter 400 µl de la solution d'hydroxylamine (0,250g +/- 0,002 g dissous dans 10 ml de Pyridine) à l'aide d'une pipette automatique de 1 ml et fermer correctement les tubes. Mélanger à l'aide d'un Vortex pendant 2 fois 10 secondes. Concentrer le milieu réactionnel au fond du tube par centrifugation (maxi 1 minute à 5000 g) et laisser réagir 1 heure 30 à 30°C. Ouvrir les tubes et ajouter 1000 µl de solution de BSTFA à l'aide d'une pipette automatique de 1 ml et compléter avec 100µl de Pyridine (pipette automatique de 200 µl). Refermer les tubes, vortexer pendant 10 secondes et mettre à incuber respectivement pendant 60 minutes à 60°C dans le cas des dérivés TBDMS et 30 minutes à 70°C pour le BSTFA. Si nécessaire filtrer les échantillons sur filtre à usage unique avec membrane PTFE de 0,22 µm ou centrifuger à 5000 g pendant 5 minutes. Transférer dans un flacon de 1,5 ml, sertir et injecter en CPG.

Les analyses ont été réalisées avec l'appareil Agilent technologies GC6890/MSD5973 équipé d'une colonne apolaire (HP-5MS, Bios Analytique). Le gaz vecteur est l'hélium à débit constant de 1 ml.min-1. L'injection de 1 µl d'échantillon a lieu en mode splitless avec un débit de purge de 50 ml.min-1 pendant 0,85 min. Le profil de température est le suivant : la température initiale de 90°C est maintenue pendant 2 minutes puis augmente jusqu'à 320 °C avec une pente de 10 °C.min-1. Cette température est maintenue pendant 6 minutes. La détection se fait par spectrométrie de masse avec ionisation par impact électronique en mode balayage dans une gamme variant de m/z = 40 à 550 amu. Le délai de passage de solvant est réglé à 3,10 minutes.

Dans ces conditions, une activité « méthionine synthase » a pu être dosée dans les échantillons incubés avec le méthanethiol, via la quantification d'une part, du succinate par chromatographie ionique et d'autre part, de la méthionine par GC-MS. Les résultats sont reportés dans le Tableau 4 ci-dessous.

**Tableau 4 : Activité méthionine synthase en présence de méthylmercaptan d'extraits des souches MBM01 et K183**

| **Souche** | **Concentration en protéines** | **Activité spécifique** **(mUI/mg protéines)** | |
|---|---|---|---|
| | | **Dosage Succinate** | **Dosage Méthionine** |
| **MBM01** | 3,43 | 0,30 | 0,23 |
| **K183** | 3,62 | 1,40 | 1,72 |

On observe donc que l'activité méthionine synthase en présence de methylmercaptan est renforcée dans la souche évoluée par rapport à la souche sauvage confirmant que la cystathionine γ-synthase mutée (E325A) a une activité méthionine synthase modifiée.

### e) isolement du gène évolué et caractérisation cinétique de l'enzyme METB^{*} ayant une activité méthionine-synthase évoluée

Afin de déterminer les paramètres cinétiques des activités méthionine-synthase et cystathionine-γ-synthase portées par METB et METB^{*}, les gènes metB et metB* ont été clonés dans un vecteur de surexpression pTopo (Invitrogene) en utilisant la stratégie suivante :
- Amplification du gène *met*B ou *met*B*^{*}* avec les oligonucléotides metJ / metLR et la polymérase thermorésistante Pwo.
- Ligation du produit PCR au plasmide pTOPO 4-PCR blunt, et introduction du plasmide ainsi formé, pTopo.metB ou pTopo.metB* dans DH5a et sélection des clones Ap'.
- Vérification par digestion enzymatique de la configuration du plasmide pTopo.metB ou pTopo.metB* après extraction.
- Introduction du plasmide vérifié pTopo.metB dans la souche MG1655(OmetBJ, Omette). Vérification par digestion enzymatique, comme précédemment du plasmide introduit. Vérification par PCR de la souche MG1655(Δ*metBJ,* Δ*met*E) avec les oligonuc1éotides metJR / metLR pour la délétion metJ, metER / metEF pour la délétion metE : souche MINS33.

Les cultures sont alors réalisées sur milieu riche et des extraits protéiques sont réalisés. Les activités enzymatiques méthionine-synthase et cystathionine-γ-synthase sont ensuite déterminées en utilisant respectivement le methylmercaptide de sodium et la cystéine comme co-substrat réactionnel. ,

Les caractéristiques cinétiques de l'activité méthionine synthase sont présentées dans le tableau N°5 tandis que les caractéristiques cinétiques de l'activité cystathionine-y-synthase sont présentées dans le tableau N°6.

**Tableau 5. Caractéristiques cinétiques apparentes de l'activité méthionine-synthase portée par les enzymes METB et METB*.**

| | Km | Vmax |
|---|---|---|
| pTOPOmetB | 277 mM | 13,9 mUl/mg protéines |
| PTOPOmetB* | 6 mM | 5,6 mUl/mg protéines |

On observe que la mutation A325E permet de diminuer de plus de 45 fois le Km de l'enzyme pour le methylmercaptan alors que le Vmax n'est lui diminué que d'un facteur 2 .

**Tableau 6. Caractéristiques cinétiques apparentes de l'activité cystathionine-y-synthase portée par les enzymes METB et METB^{*}.**

| | Km | Vmax |
|---|---|---|
| pTOPOmetB | 7,5 mM | 39840 mUl/mg protéines |
| PTOPOmetB* | 0,6 mM | 2889 mUl/mg protéines |

On observe que la mutation A325E permet de diminuer d'environ 13 fois l'activité cystathionine-y-symthase et le Km de l'enzyme pour la cystéine.

### Exemple F.I.2. : Evolution d'une activité homocystéine synthase à partir d'une cystathionine-γ-synthase

### • a Construction des souches MG1655 (ΔmetC::Cm) et MG1655 (ΔmetC)

Pour inactiver le gène *met*C la stratégie de recombinaison homologue décrite par Datsenko & Wanner (2000) est utilisée. Cette stratégie permet l'insertion d'une cassette de résistance à l'antibiotique chloramphénicol tout en délétant la majeure partie du gène concerné. Pour cela, nous avons utilisé deux oligonucléotides :
Pour *met*C *:*
   - DmetCR de 100 bases (SEQ ID NO 13):
      ccggcgtccagatcggcaatcagatcgtcgacatcttccagaccaatatgcaggcgaatcaaggtcccgctaa aatcgatCATATGAATATCCTCCTTAG
      avec
      une région (caractères minuscules) homologue à la séquence (3151419 à 3151359) du gène *met*C (séquence 3150251 à 3151438, séquence de référence sur le site http://genolist.pasteur.fr/Colibri/)
      une région (caractères majuscules) pour l'amplification de la cassette de résistance au chloramphénicol (séquence de référence dans la publication Datsenko, K.A. et Wanner, B.L., 2000, PNAS, 97 : 6640-6645)
- DmetCF de 100 bases (SEQ ID NO 14) :
   cggacaaaaagcttgatactcaactggtgaatgcaggacgcagcaaaaaatacactctcggcgcggtaaata gcgtgattTGTAGGCTGGAGCTGCTTCG
   avec
   une région (caractères minuscules) homologue à la séquence (3150255 à 3150334) du gène *met*C
   une région (caractères majuscules) pour l'amplification de la cassette de résistance au chloramphénicol.

Les oligonucléotides DmetCR et DmetCF sont utilisés pour amplifier la cassette de résistance au chloramphénicol à partir du plasmide pKD3. Le produit PCR obtenu est ensuite introduit par électroporation dans la souche MG1655 (pKD46) dans laquelle l'enzyme Red recombinase exprimée permet la recombinaison homologue. Les transformants résistants à l'antibiotique sont alors sélectionnés et l'insertion de la cassette de résistance est vérifiée par une analyse PCR avec les oligonucléotides metCR et metCF définis précédemment. La souche retenue est nommée MG1655 (Δ*met*C::Cm).

MetCR (SEQ ID NO 11) : cgtccgggacgccttgatcccggacgcaac (homologue à la séquence de 3151522 à 3151493)

MetCF (SEQ ID NO 12) : gcgtttacgcagtaaaaaagtcaccagcacgc (homologue à la séquence de 3150118 à 3150149)

La cassette de résistance au chloramphénicol peut ensuite être éliminée. Le plasmide pCP20 porteur de la recombinase FLP agissant au niveau des sites FRT de la cassette de résistance au chloramphénicol, est alors introduit dans les souches recombinantes par électroporation. Après une série de culture à 42°C, la perte de la cassette de résistance à l'antibiotique est vérifiée par une analyse PCR avec les mêmes oligonucléotides que ceux utilisés précédemment. La souche retenue est nommée MG1655 (Δ*met*C)*.*

La construction de la souche Δ*met*C est décrite dans l'exemple F.I.2. Dans un mode particulier de l'invention la souche *E. coli* Δ*met*C est mise en culture en flacon (voir exemple F.I.1) contenant un milieu minimum avec glucose comme seule source de carbone. Le milieu ne contient ni methylmercaptan, ni H2S. Des repiquages sont réalisés et les taux de croissances sont déterminés pour chaque repiquage. On observe une très nette amélioration du taux de croissance de la souche Δ(*met*C) sur milieu minimum suggérant que l'activité homocystéine synthase portée par la cystathionine γ-synthase s'est fortement améliorée en présence d'H2S endogène (voire figure 10).

| **Cycles de repiquage N°** | **µ mesuré (h⁻¹)** |
|---|---|
| 1 | 0,05 |
| 3 | 0,37 |
| 5 | 0,39 |
| 10 | 0,44 |
| 12 | 0,44 |

### Exemple F.I.3. : Evolution d'une activité méthionine synthase à partir d'une activité acétylhomosérine sulfhydrylase.

### Construction de la souche MG1655 (ΔmetB-ΔmetJ)

Pour déléter les gènes *met*B et *met*J*,* et conserver le promoteur de l'opéron metBL, nous avons inséré une cassette de résistance à l'antibiotique chloramphénicol tout en délétant la majeure partie des gènes concernés et en maintenant le promoteur de metBL. Pour cela, nous avons utilisé deux oligonucléotides.

Pour *met*BJ *:*
- MetJR de 30 bases (SEQ ID NO 9):
   ggtacagaaaccagcaggctgaggatcagc
   homologue à la séquence (4125431 à 4125460) en aval du gène *met*J (séquence 4125975 à 4125581, séquence de référence sur le site http://genolist.pasteur.fr/Colibri/)
- DmetJBF de 100 bases (SEQ ID N0 10) : 
tatgcagctgacgacctttcgcccctgcctgcgcaatcacactcatttttaccccttgtttgcagcccggaagcca
ttttcaggcaccagagtaaacatt
 avec
   une partie homologue (caractères majuscule) à la séquence (4126217 à 4126197) entre les gènes *met*J et *met*B (séquence 4126252 à 4127412) contenant la région promotrice de l'opéron *met*BLF*.*
   une partie homologue (caractères minuscule) à la séquence (4127460 à 4127380) correspondant au début du gène *met*L (séquence 4127415 à 4129847) et à la fin du gène *met*B*.*

Ces deux oligonucléotides ont été utilisés pour-amplifier la région concernée sur l'ADN chromosomique de MG1655 Δ*(met*J ::Cm) ; voir figure 11.

Le produit PCR obtenu est alors introduit par électroporation dans la souche MG1655 (pKD46) dans laquelle l'enzyme Red recombinase exprimée permet la recombinaison homologue. Les transformants résistants à l'antibiotique sont alors sélectionnés et la délétion de gène *metB* par recombinaison homologue est vérifiée par une analyse PCR avec les oligonucléotides MetJR et MetLR, définis précédemment.

La souche souhaitée est la souche MG1655 (Δ*met*B*-*Δ*met*J ::Cm) où les gènes *met*J et *met*B ont été éliminés et le promoteur de l'opéron *met*BLF replacé devant *met*L (voir figure 12).

La cassette de résistance au chloramphénicol peut ensuite être éliminée. Le plasmide pCP20 porteur de la recombinase FLP agissant au niveau des sites FRT de la cassette de résistance au chloramphénicol, est alors introduit dans les souches recombinantes par électroporation. Après une série de culture à 42°C, la perte de la cassette de résistance à l'antibiotique est vérifiée par une analyse PCR avec les mêmes oligonucléotides que ceux utilisés précédemment (MetLR et MetJR).

### Construction des souches MG1655 Δ(metC ::Cm, metJB) et MG1655 Δ(metC, metJB)

Pour déléter le gène *met*C (séquence 3150251 à 3151438, séquence de référence sur le site http://genolist.pasteur.fr/Colibri/) de la souche MG1655 (Δ*metB-*Δ*met*J)*,* nous avons utilisé la technique de transduction avec le phage P1. Le protocole suivi est réalisé en deux étapes avec la préparation du lysat de phage sur la souche MG1655 Δ(*met*C::Cm) puis transduction dans la souche MG1655 Δ(*met*B-Δ*met*J)*.*

La construction de la souche Δ(*met*C::Cm) est décrite dans l'exemple F.I.2.

### Préparation du lvsat de phage P1 :

- Ensemencement par 100µl d'une culture de nuit de la souche MG1655 (Δ*met*C::Cm) de 10 ml de LB + Cm 30µg/ml + glucose 0,2% + CaCl₂ 5 mM
- Incubation 30 min à 37°C sous agitation
- Ajout de 100 µl de lysat de phage P1 préparé sur la souche sauvage MG1655 (environ 1.10⁹ phage/ml)
- Agitation à 37°C pendant 3 heures jusqu'à la lyse totale des cellules
- Ajout de 200 µl de chloroforme et vortex
- Centrifugation 10 min à 4500g pour éliminer les débris cellulaires
- Transfert du surnageant dans un tube stérile et ajout de 200 µl de chloroforme
- Conservation du lysat à 4°C

### Transduction

- Centrifugation 10 min à 1500g de 5 ml d'une culture de nuit de la souche MG1655 (Δ*metB*-Δ*metJ*) en milieu LB
- Suspension du culot cellulaire dans 2,5 ml de MgS0₄ 10 mM, CaCl₂ 5 mM
- Tubes témoins : 100 µl cellules
   100 µl phages P1 de la souche MG1655 (Δ*met*C*::*Cm)
- Tube test: 100 µl de cellules + 100 µl de phages P1 de la souche MG1655 (Δ*met*C::Cm)
- Incubation 30 min à 30°C sans agitation
- Ajout de 100 µl de citrate de sodium 1 M dans chaque tube puis vortex
- Ajout de 1 ml de LB
- Incubation 1 heure à 37°C sous agitation
- Etalement sur des boîtes LB + Cm 30 µg/ml après centrifugation 3 min à 7000 rpm des tubes.
- Incubation à 37°C jusqu'au lendemain

### Vérification de la souche

Les transformants résistants à l'antibiotique sont alors sélectionnés et l'insertion de la région contenant (*met*C::Cm) est vérifiée par une analyse PCR avec les oligonucléotides MetCR et MetCF d'une part et MetJR et MetLR d'autre part afin de vérifier également la souche délétée des gènes *met*B et *met*J*.* La souche retenue est dénommée MG1655 Δ(*metC* ::Cm, *met*JB)*.*

MetCR (SEQ ID NO 11) : cgtccgggacgccttgatcccggacgcaac (homologue à la séquence de 3151522 à 3151493)

MetCF (SEQ ID NO 12) : gcgtttacgcagtaaaaaagtcaccagcacgc (homologue à la séquence de 3150118 à 3150149)

Comme précédemment, la cassette de résistance au chloramphénicol peut ensuite être éliminée. Le plasmide pCP20 porteur de la recombinase FLP agissant au niveau des sites FRT de la cassette de résistance au chloramphénicol, est alors introduit dans les souches recombinantes par électroporation. Après une série de culture à 42°C, la perte de la cassette de résistance à l'antibiotique est vérifiée par une analyse PCR avec les mêmes oligonucléotides que ceux utilisés précédemment (MetCR et MetCF d'une part et MetJR et Met LR d'autre part). La souche retenue est dénommée MG1655 Δ(*met*C, *met*JB)*.*

### Introduction du plasmide pTrc99A-metY et évolution de la souche

Dans un mode de réalisation particulier, on construit un plasmide permettant l'expression du gène *metY* de *C. glutamicum.* Ce gène est amplifié par PCR à partir d'ADN chromosomique de *C. glutamicum* puis introduit dans un plasmide pTrc99A. On pourra choisir d'amplifier par PCR le gène *metY* et éventuellement son promoteur naturel. Dans un mode de réalisation préféré, on clone le gène *metY* sous le contrôle d'un promoteur permettant une expression dans *E*. *coli.* Le vecteurs utilisés est un pTrC99A (Pharmacia), mais on pourrait aussi utiliser un vecteur choisi parmi pUC, pBluescript, pCR-Script, pTopo ...

La souche *Escherichia coli* Δ(*met*C*,* meJB) précédemment obtenue est transformée avec le plasmide pTrc-*met*Y de *C. glutamicum.* La transformation de la souche est réalisée par éléctroporation.

La souche obtenue est ensuite inoculée dans un Erlen Meyer (DO₆₀₀ₙₘ ∼ 0.4 - 0.5) contenant 10% de son volume en milieu minimum avec glucose pour seule source de carbone. La faible activité succinylhomoserine sulfhydrylase initialement portée par l'enzyme MetY limite la croissance (µ ∼0.06 h⁻¹) de la population bactérienne sur milieu minimum (MML8) du fait d'une limitation en méthionine synthétisée. Des repiquages sont réalisés lorsque la DO₆₀₀ₙₘ dans la fiole atteint environ 2. La sélection est ainsi menée pendant 22 cycles de cultures. On observe une forte amélioration du taux de croissance pendant cette phase d'évolution-sélection (Figure 13). Compte tenu de l'expérience acquise, il est vraisemblable que l'amélioration de croissance observée corresponde à une évolution du gène *met*Y de telle sorte que l'activité O-acetyl-homosérine sulfhydrylase soit modifiée en une activité O-succinyl-homosérine sulfhydrylase permettant de produire de l'homocysteine à partir d'O-succinylhomosérine et d'H2S ; ces deux substrats étant produits par la bactérie.

Afin d'optimiser le processus d'évolution de *met*Y, une démarche similaire peut-être réalisée en utilisant d'autres mutants d*'Escherichia coli,* on pourra notamment utiliser le mutant Δ(*met*C*, met*B)*.*

### Exemple F.I.4.: Procédé de culture Fed-Batch pour la production et la purification de méthionine.

### Préculture :

La préculture est réalisée pendant une nuit en fiole de 500 ml contenant 50 ml de milieu minimum type M9 modifié, complété avec 2.5 g/1 de glucose. Les cellules sont récupérées par centrifugation et reprises dans 5 ml de milieu minimum type M9 modifié.

### Culture en fermenteur.

La culture est réalisée dans un fermenteur de volume utile de 300ml de type Fedbatch-pro DASGIP.

Le fermenteur est rempli avec 145 ml de milieu minimum type M9 modifié et inoculer avec les 5 ml de préculture. Soit une DO600nm d'inoculation comprise entre 0.5 et 1.2.

La température de la culture est maintenue entre 30 et 37°c et le pH est ajusté en permanence à une valeur comprise entre 6.5 et 8. Il est partiellement régulé par l'ajout d'une solution CH3SNa. Une solution de soude 2N peut le cas échéant compléter la régulation. L'agitation est maintenue à entre 200 et 400 rpm pendant la phase de batch et est augmentée jusqu'à 1000 rpm en fin de fed-batch. La teneur en 02 dissout est maintenue entre 30% et 40% de saturation en utilisant un contrôleur de gaz. Dés que la OD 600nm à une valeur comprise entre 2.5 et 3 nous commençons le fed-batch par ajout du milieu de fed à un débit initial compris entre 0.3 et 0.5 ml/h avec une augmentation progressive jusqu'à des débits compris entre 2.5 et 3.5 ml/h. après ce stade le débit est maintenu constant pendant un temps compris entre 24h et 32h . Le milieu de fed est constitué sur la base d'un milieu M9 modifié complémenté par une concentration en glucose comprise entre 300 et 500g/l de glucose. Dans le même temps nous complémentons le milieu avec une solution de CH3SNa (solution entre 1 et 5M) afin de permettre la croissance de la bactérie tout en régulant le pH. Dés que la concentration cellulaire atteint une concentration comprise entre 20 et 50 g/l nous remplaçons le milieu de fed par un milieu minimum type M9 limité en phosphore. La solution de méthyl-mercaptan est remplacée par une injection directe de CH3SH sous forme gazeux dans le fermenteur. Le débit du gaz est adapté au débit de la solution de fed dans des rapports molaires avec le substrat carboné de 1 à 3. Dés que la concentration cellulaire est comprise entre 50 et 80 g/1 la fermentation est arrêtée. Le mout de fermentation est ajusté à un pH compris entre 7.5 et 9 par une solution de NaOH puis chauffé à entre 60 et 80°c . Le mout est ensuite filtré sur des modules UF. La température du jus est maintenue entre 60 et 80°C, puis le jus est concentré avant passage sur charbon pour décoloration (en colonne ou en batch). Le jus décoloré est de nouveau filtré pour retirer les dernières particules avant d'être acidifié par HCI concentré jusqu'à un pH inférieur à 2.28 (pK1 de la méthionine). Les cristaux methionine.HCI ainsi formés sont récupérés par filtration, puis l'HCI est éliminé par évaporation pour purifier la L-Méthionine.

### Dépôt de matériel biologique

La Souche K183 a été déposé le 02 Avril 2003 à la Collection Nationale de Cultures de Microorganismes (CNCM), 25 rue du Docteur Roux, 75724 Paris Cedex 15, France, selon les dispositions du Traité de Budapest, sous le Numéro d'ordre I-3005.

### F. II. Evolution de la voie de biosynthèse de la cystéine

Une application (Exemple F.II.1.) de l'invention au génie métabolique de la voie de biosynthèse de la cystéine comprend les étapes suivantes :
a) Délétion des gènes *cys*K*, cys*M dans la souche initiale d*'E.coli ;* la souche modifiée obtenue est donc auxotrophe à la cystéine. La souche initiale est capable de croître sur un milieu minimum (MM) dépourvu de méthionine, S-adénosylméthionine, homocystéine, cystathionine, cystéine alors que la souche modifiée a perdu cette capacité.
al) Introduction du gène *met*Y*,* un gène hétérologue issu de *C. glutamicum.* Ce gène étant destiné à évoluer d'une activité acetylhomosérine sulfhydrylase en une activité cystéine-synthase.
b) Culture de la souche modifiée *E. coli* [*met*Y Δ(*cys*k*, cys*M)] sur le même milieu minimum (MM) en absence de tout co-substrat afin de faire évoluer MetY en une activité cystéine-synthase afin de compenser les activités enzymatiques initialement délétées (CysK, CysM).
c) S'élection d'une souche évoluée ayant une nouvelle activité cystéine-synthase en présence d'H2S endogène ; vérification de la nouvelle voie de synthèse.

### Exemple F.II.1 : Evolution d'une activité acetylhomoserine sulfhydrylase en activité cysteine synthase.

### a) construction de la souche E. coli Δ(cysK, cysM).

L'inactivation des gènes ***cys*K et *cys*M** est réalisée en insérant une cassette de résistance à un antibiotique (chloramphénicol et kanamycine respectivement) tout en délétant la majeure partie des gènes concernés. La technique utilisée est décrite par Datsenko, K.A. ; Wanner, B.L. (2000) One-step inactivation of chromosomal genes in Escherichia coli K-12 using PCR prodücts. Proc. Natl. Acad. Sci. USA 97 : 6640-6645. Pour chaque construction un couple d'oligonucléotide a été synthétisé :

### Pour cysK:

DcysKR de 100 bases (SEQ ID NO 15) : 
tgttgcaattctttctcagtgaagagatcggcaaacaatgcggtgcttaaataacgctcacccgatgatggtagaataacC
ATATGAATATCCTCCTTAG
avec
- une région (caractères minuscules) homologue à la séquence (2531396 à 2531317) du gène *cys*k (séquence de référence sur le site http://genolist.pasteur.fr/Colibri/)
- une région (caractères majuscules) pour l'amplification de la cassette de résistance au chloramphénicol (séquence de référence dans la publication Datsenko, K.A. et Wanner, B.L., 2000, PNAS, 97 : 6640-6645) DcysKF de 100 bases (SEQ ID NO 16) : 
agtaagattttgaagataactcgctgactatcggtcacacgccgctggttcgcctgaatcgcatcggtaacggacgcatT
GTAGGCTGGAGCTGCTTCG
avec :
- une région (caractères minuscules) homologue à la séquence (2530432 à 2530511) du gène *cysK*
- une région (caractères majuscules) pour l'amplification de la cassette de résistance au chloramphénicol.

### Pour cysM :

DcysMR de 100 bases (SEQ ID NO 17): 
cctgccccctggctaaaatgctcttccccaaacaccccggtagaaaggtagcgatcgccacgatcgcagatgatcgcca
cCATATGAATATCCTCCTTAG
 avec :
- une région (caractères minuscules) homologue à la séquence (2536699 à 2536778) du gène *cys*M (séquence de référence sur le site http://genolist.pasteur.fr/Colibri/)
- une région (caractères majuscules) pour l'amplification de la cassette de résistance à la kanamycine

### DcysMF de 100 bases (SEQ ID NO 18):

agtacattagaacaaacaataggcaatacgcctctggtgaagttgcagcgaatggggccggataacggcagtgaagtgt
gTGTAGGCTGGAGCTGCTTCG

avec :
- une région (caractères minuscules) homologue à la séquence (2537600 à 2537521) du gène *cys*M
- une région (caractères majuscules) pour l'amplification de la cassette de résistance à la kanamycine

Les oligonucléotides DcysKR et DcysKF d'une part et DcysMR et DcysMF d'autre part, sont utilisés pour amplifier respectivement la cassette de résistance au chloramphénicol et à la kanamycine à partir des plasmides pKD3 et pKD4. Le produit PCR obtenu est alors introduit par électroporation dans la souche MG1655 (pKD46) dans laquelle l'enzyme Red recombinase exprimée permet la recombinaison homologue. Les transformants résistants à chacun des antibiotiques sont alors sélectionnés et l'insertion de la cassette de résistance est vérifiée par une analyse PCR avec les oligonucléotides cysKR et cysKF d'une part et cysMR et cysMF d'autre part.
cyKR (SEQ ID NO 19) : tttttaacagacgcgacgcacgaagagcgc (homologue à la séquence de 2531698 à 2531669)
cysKF (SEQ ID NO 20) : ggcgcgacggcgatgtgggtcgattgctat (homologue à la séquence de 2530188 à 2530217)
cysMR (SEQ ID NO 21) : ggggtgacggtcaggactcaccaatacttc (homologue à la séquence de 2536430 à 2536459)
cysMF (SEQ ID NO 22) : gcgcgcatcgctggccgctgggctacacac (homologue à la séquence de 2538071 à 2538042)

Les cassettes de résistance au chloramphénicol et à la kanamycine peuvent ensuite être éliminées. Pour cela, Le plasmide pCP20 porteur de la recombinase FLP agissant au niveau des sites FRT de la cassette de résistance au chloramphénicol ou à la kanamycine, est introduit dans les souches recombinantes par électroporation. Après une série de cultures à 42°C, la perte de la cassette de résistance à l'antibiotique est vérifiée par une analyse PCR avec les mêmes oligonucléotides que ceux utilisés précédemment.

### a1) introduction du gène metY dans la souche précédente

Le plasmide pTopometY a été construit par insertion du gène *metY* dans le vecteur Zero Blunt TOPO PCR cloning kit (PCR4 TOPO vector, Invitrogen). Pour cela, le gène *met*Y a été amplifié par PCR avec la polymérase Pwo à partir de l'ADN chromosomique de la souche *Corynebacterium glutamicum* ATCC13032 en utilisant les oligonucléotides suivants :
MetYR (SEQ ID NO 23) :
   ttagagctgttgacaattaatcatccggctcgtataatgtgtggaataaaaactcttaaggacctccaaatgcc

Promoteur de type TRC (pTRC-O) en gras et noir, RBS du gène *metY* en gras et souligné, codon d'initiation du gène *met*Y en gras et italiques.

MetYF (SEQ ID NO 24) :
gctctgtctagtctagtttgcattctcacg

Séquence choisie en aval du terminateur de transcription du gène *met*Y*.*

Le produit PCR obtenu est ensuite directement cloné dans le vecteur Topo pour donner le plasmide pTopometY. Le vecteur Topo porte une origine de réplication pour *E. coli,* un gène de résistance à l'ampicilline et un gène de résistance à la kanamycine.

Le plasmide pTopometY est alors introduit dans la souche *E. coli* DH5α pour vérification de la construction. Le séquençage du gène *met*Y du plasmide pTopometY avec les oligonucléotides universels M13 reverse et M13 forward sera ensuite réalisé pour confirmer la construction.

Le plasmide est introduit dans la souche *E. coli* Δ(*cys*K*, cys*M) par électroporation.

### c) Culture de la souche modifiée afin de faire évoluer le gène metY codant l'activité acetyl-homoserine sulfhydrylase vers une activité cysteine synthase.

La sélection dirigée de la souche précédente contenant le gène metY peut-être réalisée en flacons ou en Erlen-Meyer. La mise en oeuvre de cette technique permet la sélection d'une souche de *Escherichia coli* dont l'enzyme acetyl-homosérine sulfhydrolase a évolué en une activité « cystéine synthase ». La sélection dirigée est conduite en Erlen-Meyer contenant 50 ml de milieu minéral (Schaefer et al., 1999, Anal. Biochem. 270 : 88-96) en présence de 33 mM glucose, de chloramphénicol à une concentration finale de 25 mg/1 et de kanamycine à une concentration de 25 mg/ml

Les milieux de culture sont ensemencés avec la souche *E. coli* K12 [Δ (*cys*K*, cys*M) pTopometY] à DO₆₀₀ₙₘ définie. On ensemence avec une population de bactéries suffisamment importante pour que certaines bactéries possèdent potentiellement des mutations spontanées intéressantes dans le gène *met*Y*,* permettant d'assimiler l'O-acétyl serine. Cette population a été obtenue par culture de la souche auxotrophe en cystéine sur milieu minimum supplémenté en cystéine. Une culture témoin est ensemencée avec la souche *E. coli* K12 (Δ *cys*k*, cys*M)*.*

Les cultures sont réalisées sous agitation, à 37°C, pendant 6 jours, puis la DO₆₀₀ₙₘ est mesurée. La culture témoin ne présente pratiquement pas d'évolution de DO alors que quelques autres cultures ont vu leur DO évoluer de manière significative. Il est probable que l'activité « cystéine synthase évoluée » se soit développée dans les populations contenues dans ces Erlen-Meyer. La ou les mutations sont vraisemblablement intervenue dans le gène metY puisque c'est la seule différence entre ces souches et la souche témoin.

La population bactérienne de ces cultures positives peut alors être utilisée pour améliorer davantage l'activité cystéine synthase en recommençant le procédé en flacon tel qu'il vient d'être décrit.

### c) sélection des clones

La population évoluée est étalée alors sur milieu minimum gélosé contenant du glucose pour seule source de carbone. Les boites inoculées sont placées en condition aérobie dans un incubateur à 37°C. Après 36 heures, les clones apparaissent sur les boites ; ils correspondent aux bactéries capables de produire de la cystéine à partir du glucose pour seule source de carbone. Trois clones sont isolés.

### c) contrôle de la voie de synthèse

La population d'*E*. *coli* K12 [Δ (cysK, *cys*M) pTopometY] évoluée est mise en culture dans un milieu minimum (Schaefer et al., 1999, Anal. Biochem. 270 : 88-96) contenant 2,5 g.1⁻¹ de glucose entièrement marqué au carbone 13. Après la culture, les cellules sont récupérées, lavées puis hydrolysées par HC1 6N pendant 24 heures à 107°C. Une analyse par RMN bidimensionnelle est alors réalisée (HSQC). Cette analyse permet d'étudier le flux des carbones 13 du glucose et confirmer que la synthèse de la cystéine se fait bien via la sérine et l'acetyl-sérine suggérant ainsi que l'enzyme codée par le gène *met*Y a effectivement évoluée en une cystéine synthase.

### F. III. Evolution d'enzymes NADPH dépendantes

Une application (Exemple F.III.1.) de l'invention au génie métabolique aux voies de bioconversion NADPH dépendantes comprend les étapes suivantes :
a1) Inactivation des gènes *udh*A et *pgi* dans la souche initiale d*'E.coli.*
a2) Inactivation des gènes *pfkA, pfkB et udhA* dans la souche initiale d*'E.coli.*

Les deux souches modifiées obtenues sont donc optimisées pour leur capacité de réduction du NADP. La souche initiale est capable de croître sur un milieu minimum (MM) alors que les souches modifiées ont une capacité à croitre, sur ce même milieu, fortement reduite.
b) introduction sur plasmide du gène *yue*D codant pour une benzil réductase de *Bacillus cereus* dans une de ces souches alterées.
c) Culture de la souche ci-avant modifiée sur milieu minimum (MM) auquel on ajoute le p-nitrobenzaldehyde (substrat) afin de faire évoluer l'activité enzymatique pour l'adapter à ce substrat faiblement utilisé.
d) Caractérisation de l'enzyme YueD évoluée

### F.III.1 : Construction des souches E. coli Δ(udhA, pgi) et E.coli Δ(pfkA, pfkB, udhA) et modification de l'activité enzymatique de la benzyl réductase de Bacillus cereus

### a1) construction de la souche modifiée E. coli Δ(udhA, pgi)

L'inactivation du gène *udhA* est réalisée en insérant une cassette de résistance à l'antibiotique kanamycine tout en délétant la majeure partie du gène concerné. La technique utilisée est décrite par Datsenko; K.A. ; Wanner, B.L. (2000) One-step inactivation of chromosomal genes in Escherichia coli K-12 using PCR products. Proc. Natl. Acad Sci. USA 97 : 6640-6645.

Deux oligonucléotides sont utilisés pour réaliser la stratégie:
- DudhAR de 100 bases (SEQ ID NO 25): 
Cccagaatctcttttgtttcccgatggaacaaaattttcagcgtgcccacgttcatgccgacgatttgtgcgcgtgcca
gTGTAGGCTGGAGCTGCTTCG
 avec :
   une région (caractères minuscules) homologue à la séquence (4157144 à 4157223) du gène *udhA* (séquence 4158303 à 4156969, séquence de référence sur le site http://genolist.pasteur.fr/Colibri/)
   une région (caractères majuscules) pour l'amplification de la cassette de résistance à la kanamycine du plasmide pKD4 (Datsenko, K.A.; Wanner, B.L. (2000) One-step inactivation of chromosomal genes in Escherichia coli K-12 using PCR products. Proc. Natl. Acad Sci. USA 97 : 6640-6645)
- DudhAF de 100 bases (SEQ ID NO 26): 
ggtgcgcgcgtcgcagttatcgagcgttatcaaaatgttggcggcggttgcacccactggggcaccatcccgt
cgaaagcCATATGAATATCCTCCTTAG
 avec:
   une région (caractères minuscules) homologue à la séquence (4158285 à 4158206) du gène *udhA*
   une région (caractères majuscules) pour l'amplification de la cassette de résistance à la kanamycine portée par le plasmide pKD4.

Les oligonucléotides DudhAR et DudhAF sont utilisés pour amplifier la cassette de résistance à la kanamycine à partir du plasmide pKD4. Le produit PCR obtenu est alors introduit par électroporation dans la souche MG1655 (pKD46) dans laquelle l'enzyme Red recombinase exprimée permet la recombinaison homologue. Les transformants résistants à l'antibiotique sont alors sélectionnés et l'insertion de la cassette de résistance est vérifiée par une analyse PCR avec les oligonucléotides UdhAR et UdhAF.

UdhAR (SEQ ID NO 27) : gcgggatcactttactgccagcgctggctg (homologue à la séquence de 4156772 à 4156801)

UdhAF (SEQ ID NO 28) : ggccgctcaggatatagccagataaatgac (homologue à la séquence de 4158475 à 4158446)

L'inactivation du *gène pgi* est réalisée en insérant une cassette de résistance à l'antibiotique chloramphénicol tout en délétant la majeure partie du gène concerné. La technique utilisée est décrite par Datsenko, K.A. ; Wanner, B.L. (2000) One-step inactivation of chromosomal genes in Escherichia coli K-12 using PCR products. Proc. Natl. Acad. Sci. USA 97 : 6640-6645.

Deux oligonucléotides sont utilisés pour réaliser la stratégie:
- DpgiR de 100 bases (SEQ ID NO 29): 
gcgccacgctttatagcggttaatcagaccattggtcgagctatcgtggctgctgatttctttatcatctttcagctc
tgCATATGAATATCCTCCTTAG
 avec :
   une région (caractères minuscules) homologue à la séquence (4232980 à 4232901) du gène *pgi* (séquence 4231337 à 4232986, séquence de référence sur le site http://genolist.pasteur.fr/Colibri/)
   une région (caractères majuscules) pour l'amplification de la cassette de résistance au chloramphénicol du plasmide pKD3 (Datsenko, K.A. ; Wanner, B.L. (2000) One-step inactivation of chromosomal genes in Escherichia coli K-12 using PCR products. Proc. Natl. Acad Sci. USA 97 : 6640-6645)
- DpgiF de 100 bases (SEQ ID NO 30): 
ccaacgcagaccgctgcctggcaggcactacagaaacacttcgatgaaatgaaagacgttacgatcgccgat
ctttttgcTGTAGGCTGGAGCTGCTTCG
 avec :
   une région (caractères minuscules) homologue à la séquence (4231352 à 4231432) du gène *pgi*
   une région (caractères majuscules) pour l'amplification de la cassette de résistance au chloramphénicol portée par le plasmide pKD3

Les oligonucléotides DpgiR et DpgiF sont utilisés pour amplifier la cassette de résistance au chloramphénicol à partir du plasmide pKD3. Le produit PCR obtenu est alors introduit par électroporation dans la souche MG 1655 *ΔudhA* (pKD46) dans laquelle l'enzyme Red recombinase exprimée permet la recombinaison homologue. Les transformants résistants à l'antibiotique sont alors sélectionnés et l'insertion de la cassette de résistance est vérifiée par une analyse PCR avec les oligonucléotides PgiR et PgiF.

PgiR (SEQ ID NO 31) : cggtatgatttccgttaaattacagacaag (homologue à la séquence de 4233220 à 4233191)

PgiF (SEQ ID NO 32) : gcggggcggttgtcaacgatggggtcatgc (homologue à la séquence de 4231138 à 4231167)

Les deux cassettes de résistance au chloramphénicol et à la kanamycine peuvent ensuite être éliminées. Le plasmide pCP20 porteur de la recombinase FLP agissant au niveau des sites FRT des cassettes de résistance, est alors introduit dans les souches recombinantes par électroporation. Après une série de culture à 42°C, la perte des cassettes de résistance à l'antibiotique est vérifiée par une analyse PCR avec les mêmes oligonucléotides que ceux utilisés précédemment.

### a2) construction de la souche modifiée E.coli Δ(pfkA, pfkB, udhA)

L'inactivation du gène *pfkA* est réalisée en insérant une cassette de résistance à l'antibiotique chloramphénicol tout en délétant la majeure partie du gène concerné. La technique utilisée est décrite par Datsenko, K.A. ; Wanner, B.L. (2000) One-step inactivation of chromosomal genes in Escherichia coli K-12 using PCR products. Proc. Natl. Acad Sci. USA 97 : 6640-6645.

Deux oligonucléotides sont utilisés pour réaliser la stratégie:
- DpfkAR de 100 bases (SEQ ID NO 33): 
Ttcgcgcagtccagccagtcacctttgaacggacgcttcatgttttcgatagcgtcgatgatgtcgtggtgaaccagc
tgCATATGAATATCCTCCTTAG
 avec :
   Une région (caractères minuscules) homologue à la séquence (4106081 à 4106002) du gène *pfkA* (séquence 4105132 à 4106094, séquence de référence sur le site http://genolist.pasteur.fr/Colibri/
   une région (caractères majuscules) pour l'amplification de la cassette de résistance au chloramphénicol du plasmide pKD3 (Datsenko, K.A. ; Wanner, B.L. (2000) One-step inactivation of chromosomal genes in Escherichia coli K-12 using PCR products. Proc. Natl. Acad. Sci. USA 97 : 6640-6645)
- DpfkAF de 100 bases (SEQ ID NO 34):

ggtgtgttgacaagcggcggtgatgcgccaggcatgaacgccgcaattcgcggggttgttcgttctgcgctga
cagaa ggTGTAGGCTGGAGCTGCTTCG avec :

   une région (caractères minuscules) homologue à la séquence (4105147 à 4105227) du gène *pfkA*
   une région (caractères majuscules) pour l'amplification de la cassette de résistance au chloramphénicol portée par le plasmide pKD3.

Les oligonucléotides DpfkAR et DpfkAF sont utilisés pour amplifier la cassette de résistance au chloramphénicol à partir du plasmide pKD3. Le produit PCR obtenu est alors introduit par électroporation dans la souche MG1655 (pKD46) dans laquelle l'enzyme Red recombinase exprimée permet la recombinaison homologue. Les transformants résistants à l'antibiotique sont alors sélectionnés et l'insertion de la cassette de résistance est vérifiée par une analyse PCR avec les oligonucléotides PfkAR et PfkAF.

PtkAR (SEQ ID NO 35) : ccctacgccccacttgttcatcgcccg (homologue à la séquence de 4106434 à 4106408)

PfkAF (SEQ ID NO 36) : cgcacgcggcagtcagggccgacccgc (homologue à la séquence de 4104751 à 4104777)

L'inactivation du gène *udhA* était decrite en exemple F.III.1 a1) et peut être effectuée de la même manière.

Les deux cassettes de résistance au chloramphénicol et à la kanamycine peuvent ensuite être éliminées. Le plasmide pCP20 porteur de la recombinase FLP agissant au niveau des sites FRT des cassettes de résistance, est alors introduit dans les souches recombinantes par électroporation. Après une série de culture à 42°C, la perte des cassettes de résistance à l'antibiotique est vérifiée par une analyse PCR avec les mêmes oligonucléotides que ceux utilisés précédemment.

L'inactivation du gène *pfkB* est réalisée en insérant une cassette de résistance à l'antibiotique chloramphénicol tout en délétant la majeure partie du gène concerné. La technique utilisée est décrite par Datsenko, K.A. ; Wanner, B.L. (2000) One-step inactivation of chromosomal genes in Escherichia coli K-12 using PCR products. Proc. Natl. Acad. Sci. USA 97 : 6640-6645.

Deux oligonucléotides sont utilisés pour réaliser la stratégie:
- DpfkBR de 100 bases (SEQ ID NO 37): 
gcgggaaaggtaagcgtaaatttttgcgtatcgtcatgggagcacagacgtgttccctgattgagtgtggctgcact
ccCATATGAATATCCTCCTTAG
 avec :
   Une région (caractères minuscules) homologue à la séquence (1805320 à 1805241) du gène *pfkB* (séquence 1804394 à 1805323, séquence de référence sur le site http://genolist.pasteur.fr/Colibri/)
   une région (caractères majuscules) pour l'amplification de la cassette de résistance à la chloramphénicol du plasmide pKD3 (Datsenko, K.A. ; Wanner, B.L. (2000) One-step inactivation of chromosomal genes in Escherichia coli K-12 using PCR products. Proc. Natl. Acad. Sci. USA 97 : 6640-6645)
- DpfkBF de 100 bases (SEQ ID NO 38):

gcgccctctctcgatagcgcaacaattaccccgcaaatttatcccgaaggaaaactgcgctgtaccgcaccgg
tgttcgTGTAGGCTGGAGCTGCTTCG avec :

   une région (caractères minuscules) homologue à la séquence (1804421 à 1804499) du gène *pfkB*
   une région (caractères majuscules) pour l'amplification de la cassette de résistance au chloramphénicol portée par le plasmide pKD3.

Les oligonucléotides DpfkBR et DpfkBF sont utilisés pour amplifier la cassette de résistance au chloramphénicol à partir du plasmide pKD3. Le produit PCR obtenu est alors introduit par électroporation dans la souche MG1655 Δ(*pfkA, udhA*)*.* (pKD46) dans laquelle l'enzyme Red recombinase exprimée permet la recombinaison homologue. Les transformants résistants à l'antibiotique sont alors sélectionnés et l'insertion de la cassette de résistance est vérifiée par une analyse PCR avec les oligonucléotides PfkBR et PfkBF.

PfkBR (SEQ ID NO 39): gccggttgcactttgggtaagccccg (homologue à la séquence de (1805657-1805632)

PfkBF (SEQ ID NO 40): tggcaggatcatccatgacagtaaaaacgg (homologue à la séquence de 1803996 à 1804025

La cassette de résistance au chloramphénicol peut ensuite être éliminée. Le plasmide pCP20 porteur de la recombinase FLP agissant au niveau des sites FRT des cassettes de résistance, est alors introduit dans la souche recombinante par électroporation. Après une série de culture à 42°C, la perte de la cassette de résistance à l'antibiotique est vérifiée par une analyse PCR avec les mêmes oligonucléotides que ceux utilisés précédemment.

### b) introduction du gène yueD codant Benzyl réductase de Bacillus cereus dans la souche Δ(pgi, udhA) ou également Δ(pfkA, pfkB, udhA)

Le gène *yueD* de *Bacillus cereus* est cloné dans le vecteur pTrC99A (Amersham-Pharmacia). Les oligonucléotides YueDR et YueDF sont utilisés pour amplifier le gène à partir de l'ADN génomique de *Bacillus cereus.* YueDR (SEQ ID NO 41): CGTGAATTC dans le milieu minimum du 1-phenyl-1,2-propanedione (co-substrat) et l'on observe que la souche modifiée est capable de croître avec un taux de croissance similaire ou légèrement inférieur par rapport à celui de la souche initiale (c'est à dire non modifiée) sur le même milieu. II est alors décidé d'ensemencer (OD 5) un chemostat en milieu minimum contenant du p-nitrobenzaldehyde (co-substrat) avec la souche modifiée. Dans ces conditions la souche présentera un taux de croissance similaire à la souche modifiée cultivée en milieu minimum en absence de co-substrat. Le chemostat est maintenu sur une période de 1 à 5 semaines en augmentant progressivement le taux de dilution. L'augmentation du taux de dilution pouvant se faire par palier ou de manière progressive. Des stocks glycérols, de la population contenue dans le chemostat, sont réalisés régulièrement.

### d) Caractérisation de l'enzyme YueD évoluée

Lorsque la population ne peut plus s'adapter aux taux de dilutions imposés, on considère que la sélection est terminée. Les colonies sont isolées de la population évoluée finale (si nécessaire on utilise l'un des derniers stocks glycérol réalisés) et les caractéristiques cinétiques de la benzil réductase évoluée sont évaluées, par comparaison avec la benzil réductase initiale, en utilisant les substrats 1-phenyl-1,2-propanedione et p-nitrobenzaldehyde. On observe une forte amélioration du k_{cat} de la benzil réductase évoluée pour le p-nitrobenzaldehyde tandis que le k_{cat} de cette même enzyme évoluée pour le 1-phenyl-1,2-propanedione diminue fortement. Le séquençage des clones évolués montre une accumulation de mutations ponctuelles, ce qui explique la modification de leur comportement quant à la spécificité de substrat.

### REFERENCES

Anderson, E.H. (1946), Growth requirements of virus-resistant mutants of Escherichia coli strain "B" Proc. Natl. Acad. Sci. USA 32:120-128
A Baudin, O Ozier-Kalogeropoulos, A Denouel, F Lacroute, and C Cullin (1993) A simple and efficient method for direct gene deletion in Saccharomyces cerevisiae, Nucl. Acids Res., 21: 3329-3330, 1993;
Brachmann CB, Davies A, Cost GJ, Caputo E, Li J, Hieter P, Boeke JD. (1998) Designer deletion strains derived from Saccharomyces cerevisiae S288C: a useful set of strains and plasmids for PCR-mediated gene disruption and other applications. Yeast. 14 :115-32.
Datsenko, K.A. ; Wanner, B.L. (2000) One-step inactivation of chromosomal genes in Escherichia coli K-12 using PCR products. Proc. Natl. Acad. Sci. USA 97 : 6640-6645
Miller, 1992 ; A Short Course in Bacterial Genetics: A Laboratory Manual and Handbook for Escherichia coli and Related Bacteria, Cold Spring Harbor Laboratory Press, Cold Spring Harbor, New York
Sambrook et al. (1989) Molecular cloning : a laboratory manual. 2nd Ed. Cold Spring Harbor Lab., Cold Spring Harbor, New York
Schaefer, U.; Boos, W.; Takors, R.; Weuster-Botz, D. (1999) Automated sampling device for monitoring intracellular metabolite dynamics., Anal. Biochem. 270 : 88-96 Wach,A., Brachat,A., Pohlmann,R., and Philippsen, P. (1994) New heterologous modules for classical or PCR-based gene disruptions in Saccharomyces cerevisiae. Yeast, 10, 1793-1808.

## Revendications

1. Procédé de préparation de microorganismes évolués, permettant une modification des voies métaboliques consommatrices de NADPH, **caractérisé en ce qu'**il comprend les étapes suivantes :
**a)** Modification génétique des cellules d'un microorganisme initial de telle sorte que la vitesse de production du NADPH soit supérieure à sa vitesse d'oxydation en NADP, ce microorganisme comprenant un ou plusieurs gènes codant pour des enzymes NADPH dépendantes impliquées dans la biotransformation d'une molécule d'intérêt,
**b)** Culture des microorganismes modifiés précédemment obtenus sur un milieu défini pour faire évoluer ce ou ces gènes codant pour des enzymes NADPH dépendantes impliquées dans la biotransformation d'une molécule d'intérêt,
**c)** Sélection des cellules de microorganismes modifiés capables de se développer sur le milieu défini.

2. Procédé selon la revendication 1, **caractérisé en ce que** la modification génétique de l'étape a) comprend la limitation de l'oxydation du NADPH en NADP.

3. Procédé selon l'une des revendications 1 ou 2, **caractérisé en ce que** au moins l'un des gènes *udhA* et/ou *qor* est délété.

4. Procédé selon l'une des revendications 1 à 3, **caractérisé en ce que** la modification génétique de l'étape a) permet de favoriser la réduction du NADP en NADPH.

5. Procédé selon l'une des revendications 1 à 4, **caractérisé en ce que** au moins l'un des gènes *pgi, pfkA* et *pfkB* est délété.

6. Procédé selon l'une des revendications 1 à 5, **caractérisé en ce que** le ou les gènes codant pour des enzymes NADPH dépendantes impliquées dans la biotransformation d'une molécule d'intérêt sont introduits dans les souches modifiées de l'étape a) avec un vecteur approprié.

7. Microorganisme évolué susceptible d'être obtenu par le procédé selon l'une des revendications 1 à 6.

8. Utilisation du microorganisme évolué selon la revendication 7 dans un procédé de biotransformation consommateur de NADPH.

9. Procédé de production d'une molécule d'intérêt ayant une biosynthèse consommatrice de NADPH **caractérisé en ce qu'**il comprend les étapes suivantes :
**a)** mise en culture des microorganismes évolués selon la revendication 7 dans un milieu de culture approprié favorisant leur croissance et comprenant les substances nécessaires pour la réalisation de la biotransformation par fermentation ou bioconversion, à l'exception du NADPH, et
**b)** extraction de la molécule d'intérêt du milieu et le cas échéant purification.

10. Procédé selon la revendication 9 dans lequel la molécule d'intérêt est choisie parmi la cystéine, la méthionine, le 3-hydroxypropionate, l'hydrocortisone, le xylitol et le glycérol.


SEQUENCE LISTING

<110>  Metabolic Explorer

<120>  Procédé de préparation de microorganismes évolués permettant la création
ou la modification de voies métaboliques

<130>  D20701/ 345773

<150>  FR 0301924

<151>  2003-02-18

<150>  FR 0305768

<151>  2003-05-14

<150>  FR 0305769

<151>  2003-05-14

<150>  FR 0313054

<151>  2003-11-06

<160>  42

<170>  PatentIn version 3.1

<210>  1

<211>  100

<212>  DNA

<213>  Artificial Sequence

<220>

<223>  DmetER

<400>  1
tacccccgac gcaagttctg cgccgcctgc accatgttcg ccagtgccgc gcgggtttct      60

ggccagccgc gcgttttcag catatgaata tcctccttag                            100

<210>  2

<211>  100

<212>  DNA

<213>  Artificial Sequence

<220>

<223>  DmetEF

<400>  2
tgacaatatt gaatcacacc ctcggtttcc ctcgcgttgg cctgcgtcgc gagctgaaaa     60

aagcgcaaga aagttattgg tgtaggctgg agctgcttcg                          100

<210>  3

<211>  30

<212>  DNA

<213>  Artificial Sequence

<220>

<223>  MetER

<400>  3
ggtttaagca gtatggtggg aagaagtcgc                                      30

<210>  4

<211>  30

<212>  DNA

<213>  Artificial Sequence

<220>

<223>  MetEF

<400>  4
cccggggatg aataaacttg ccgccttccc                                      30

<210>  5

<211>  1161

<212>  DNA

<213>  Escherichia coli

<400>  5

atgacgcgta aacaggccac catcgcagtg cgtagcgggt taaatgacga cgaacagtat 60

ggttgcgttg tcccaccgat ccatctttcc agcacctata actttaccgg atttaatgaa 120

ccgcgcgcgc atgattactc gcgtcgcggc aacccaacgc gcgatgtggt tcagcgtgcg 180

ctggcagaac tggaaggtgg tgctggtgca gtacttacta ataccggcat gtccgcgatt 240

cacctggtaa cgaccgtctt tttgaaacct ggcgatctgc tggttgcgcc gcacgactgc 300

tacggcggta gctatcgcct gttcgacagt ctggcgaaac gcggttgcta tcgcgtgttg 360

tttgttgatc aaggcgatga acaggcatta cgggcagcgc tggcagaaaa acccaaactg 420

gtactggtag aaagcccaag taatccattg ttacgcgtcg tggatattgc gaaaatctgc 480

catctggcaa gggaagtcgg ggcggtgagc gtggtggata acaccttctt aagcccggca 540

ttacaaaatc cgctggcatt aggtgccgat ctggtgttgc attcatgcac gaaatatctg 600

aacggtcact cagacgtagt ggccggcgtg gtgattgcta aagacccgga cgttgtcact 660

gaactggcct ggtgggcaaa caatattggc gtgacgggcg gcgcgtttga cagctatctg 720

ctgctacgtg ggttgcgaac gctggtgccg cgtatggagc tggcgcagcg caacgcgcag 780

gcgattgtga aatacctgca aacccagccg ttggtgaaaa aactgtatca cccgtcgttg 840

ccggaaaatc aggggcatga aattgccgcg cgccagcaaa aaggctttgg cgcaatgttg 900

agttttgaac tggatggcga tgagcagacg ctgcgtcgtt tcctgggcgg gctgtcgttg 960

tttacgctgg cggaatcatt agggggagtg gaaagtttaa tctctcacgc cgcaaccatg 1020

acacatgcag gcatggcacc agaagcgcgt gctgccgccg ggatctccga gacgctgctg 1080

cgtatctcca ccggtattga agatggcgaa gatttaattg ccgacctgga aaatggcttc 1140

cgggctgcaa acaaggggta a 1161

<210> 6

<211> 10

<212> PRT

<213> Escherichia coli

<400> 6

Met Glu Thr Thr His Arg Ala Arg Gly Leu
1               5                   10

<210> 7

<211> 1161

<212> DNA

<213> Escherichia coli

<400> 7

atgacgcgta aacaggccac catcgcagtg cgtagcgggt taaatgacga cgaacagtat 60
ggttgcgttg tcccaccgat ccatctttcc agcacctata actttaccgg atttaatgaa 120
ccgcgcgcgc atgattactc gcgtcgcggc aacccaacgc gcgatgtggt tcagcgtgcg 180
ctggcagaac tggaaggtgg tgctggtgca gtacttacta ataccggcat gtccgcgatt 240
cacctggtaa cgaccgtctt tttgaaacct ggcgatctgc tggttgcgcc gcacgactgc 300
tacggcggta gctatcgcct gttcgacagt ctggcgaaac gcggttgcta tcgcgtgttg 360
tttgttgatc aaggcgatga acaggcatta cgggcagcgc tggcagaaaa acccaaactg 420
gtactggtag aaagcccaag taatccattg ttacgcgtcg tggatattgc gaaaatctgc 480
catctggcaa gggaagtcgg ggcggtgagc gtggtggata acaccttctt aagcccggca 540
ttacaaaatc cgctggcatt aggtgccgat ctggtgttgc attcatgcac gaaatatctg 600
aacggtcact cagacgtagt ggccggcgtg gtgattgcta aagacccgga cgttgtcact 660
gaactggcct ggtgggcaaa caatattggc gtgacgggcg gcgcgtttga cagctatctg 720
ctgctacgtg ggttgcgaac gctggtgccg cgtatggagc tggcgcagcg caacgcgcag 780
gcgattgtga aatacctgca aacccagccg ttggtgaaaa aactgtatca cccgtcgttg 840
ccggaaaatc aggggcatga aattgccgcg cgccagcaaa aaggctttgg cgcaatgttg 900
agttttgaac tggatggcga tgagcagacg ctgcgtcgtt tcctgggcgg gctgtcgttg 960
tttacgctgg cggcatcatt agggggagtg gaaagtttaa tctctcacgc cgcaaccatg 1020
acacatgcag gcatggcacc agaagcgcgt gctgccgccg ggatctccga gacgctgctg 1080
cgtatctcca ccggtattga agatggcgaa gatttaattg ccgacctgga aaatggcttc 1140
cgggctgcaa acaaggggta a 1161

<210> 8
<211> 5
<212> PRT
<213> Escherichia coli

<400> 8
Met Glu Thr Thr His
1               5

<210> 9
<211> 30
<212> DNA
<213> Artificial Sequence

<220>

<223>  MetJR

<400>  9
ggtacagaaa ccagcaggct gaggatcagc                                        30

<210>  10

<211>  100

<212>  DNA

<213>  Artificial Sequence

<220>

<223>  DmetJBF

<400>  10
tatgcagctg acgacctttc gcccctgcct gcgcaatcac actcattttt accccttgtt      60

tgcagcccgg aagccatttt caggcaccag agtaaacatt                            100

<210>  11

<211>  30

<212>  DNA

<213>  Artificial Sequence

<220>

<223>  MetCR

<400>  11
cgtccgggac gccttgatcc cggacgcaac                                        30

<210>  12

<211>  32

<212>  DNA

<213>  Artificial Sequence

<220>

<223>  MetCF

<400>  12
gcgtttacgc agtaaaaaag tcaccagcac gc                                     32

<210>  13

<211>  100

<212>  DNA

<213>  Artificial Sequence

<220>

<223>  DmetCR

<400>  13
ccggcgtcca gatcggcaat cagatcgtcg acatcttcca gaccaatatg caggcgaatc     60

aaggtcccgc taaaatcgat catatgaata tcctccttag                           100

<210>  14

<211>  100

<212>  DNA

<213>  Artificial Sequence

<220>

<223>  DmetCF

<400>  14
cggacaaaaa gcttgatact caactggtga atgcaggacg cagcaaaaaa tacactctcg     60

gcgcggtaaa tagcgtgatt tgtaggctgg agctgcttcg                           100

<210>  15

<211>  100

<212>  DNA

<213>  Artificial Sequence

<220>

<223>  DcysKR

<400>  15
tgttgcaatt ctttctcagt gaagagatcg gcaaacaatg cggtgcttaa ataacgctca     60

cccgatgatg gtagaataac catatgaata tcctccttag                           100

<210>  16

<211>  100

<212>  DNA

<213>  Artificial Sequence

<220>

<223>  DcysKF

<400> 16
agtaagattt ttgaagataa ctcgctgact atcggtcaca cgccgctggt tcgcctgaat 60

cgcatcggta acggacgcat tgtaggctgg agctgcttcg 100

<210> 17

<211> 100

<212> DNA

<213> Artificial Sequence

<220>

<223> DcysMR

<400> 17
cccgccccct ggctaaaatg ctcttcccca aacaccccgg tagaaaggta gcgatcgcca 60

cgatcgcaga tgatcgccac catatgaata tcctccttag 100

<210> 18

<211> 100

<212> DNA

<213> Artificial Sequence

<220>

<223> DcysMF

<400> 18
agtacattag aacaaacaat aggcaatacg cctctggtga agttgcagcg aatggggccg 60

gataacggca gtgaagtgtg tgtaggctgg agctgcttcg 100

<210> 19

<211> 30

<212> DNA

<213> Artificial Sequence

<220>

<223> cysKR

<400> 19
tttttaacag acgcgacgca cgaagagcgc 30

<210> 20

<211> 30

<212>  DNA

<213>  Artificial Sequence

<220>

<223>  cysKR

<400>  20
ggcgcgacgg cgatgtgggt cgattgctat                                        30

<210>  21

<211>  30

<212>  DNA

<213>  Artificial Sequence

<220>

<223>  cysMR

<400>  21
ggggtgacgg tcaggactca ccaatacttc                                        30

<210>  22

<211>  30

<212>  DNA

<213>  Artificial Sequence

<220>

<223>  cysMF

<400>  22
gcgcgcatcg ctggccgctg ggctacacac                                        30

<210>  23

<211>  74

<212>  DNA

<213>  Artificial Sequence

<220>

<223>  MetYR

<400>  23
ttagagctgt tgacaattaa tcatccggct cgtataatgt gtggaataaa aactcttaag      60

gacctccaaa tgcc                                                    74

<210>  24
<211>  30
<212>  DNA
<213>  Artificial Sequence

<220>
<223>  MetYF

<400>  24
gctctgtcta gtctagtttg cattctcacg                                    30

<210>  25
<211>  100
<212>  DNA
<213>  Artificial Sequence

<220>
<223>  DudhAR

<400>  25
cccagaatct cttttgtttc ccgatggaac aaaattttca gcgtgcccac gttcatgccg    60
acgatttgtg cgcgtgccag tgtaggctgg agctgcttcg                         100

<210>  26
<211>  100
<212>  DNA
<213>  Artificial Sequence

<220>
<223>  DudhAF

<400>  26
ggtgcgcgcg tcgcagttat cgagcgttat caaaatgttg gcggcggttg cacccactgg    60
ggcaccatcc cgtcgaaagc catatgaata tcctccttag                         100

<210>  27
<211>  30
<212>  DNA
<213>  Artificial Sequence

<220>

<223>  UdhAR

<400>  27
gcgggatcac tttactgcca gcgctggctg                                    30

<210>  28

<211>  30

<212>  DNA

<213>  Artificial Sequence

<220>

<223>  UdhAF

<400>  28
ggccgctcag gatatagcca gataaatgac                                    30

<210>  29

<211>  100

<212>  DNA

<213>  Artificial Sequence

<220>

<223>  DpgiR

<400>  29
gcgccacgct ttatagcggt taatcagacc attggtcgag ctatcgtggc tgctgatttc      60

tttatcatct ttcagctctg catatgaata tcctccttag                          100

<210>  30

<211>  100

<212>  DNA

<213>  Artificial Sequence

<220>

<223>  DpgiF

<400>  30
ccaacgcaga ccgctgcctg gcaggcacta cagaaacact tcgatgaaat gaaagacgtt      60

acgatcgccg atctttttgc tgtaggctgg agctgcttcg                          100

<210>  31

<211>  30

<212>  DNA

<213>  Artificial Sequence

<220>

<223>  PgiR

<400>  31
cggtatgatt tccgttaaat tacagacaag                                        30

<210>  32

<211>  30

<212>  DNA

<213>  Artificial Sequence

<220>

<223>  PgiF

<400>  32
gcggggcggt tgtcaacgat ggggtcatgc                                        30

<210>  33

<211>  100

<212>  DNA

<213>  Artificial Sequence

<220>

<223>  DpfkAR

<400>  33
ttcgcgcagt ccagccagtc acctttgaac ggacgcttca tgttttcgat agcgtcgatg      60

atgtcgtggt gaaccagctg catatgaata tcctccttag                            100

<210>  34

<211>  100

<212>  DNA

<213>  Artificial Sequence

<220>

<223>  DpfkAF

<400>  34
ggtgtgttga caagcggcgg tgatgcgcca ggcatgaacg ccgcaattcg cggggttgtt     60

cgttctgcgc tgacagaagg tgtaggctgg agctgcttcg                           100

<210>  35

<211>  27

<212>  DNA

<213>  Artificial Sequence

<220>

<223>  PfkAR

<400>  35
ccctacgccc cacttgttca tcgcccg                                          27

<210>  36

<211>  27

<212>  DNA

<213>  Artificial Sequence

<220>

<223>  PfkAF

<400>  36
cgcacgcggc agtcagggcc gacccgc                                          27

<210>  37

<211>  100

<212>  DNA

<213>  Artificial Sequence

<220>

<223>  DpfkBR

<400>  37
gcgggaaagg taagcgtaaa ttttttgcgt atcgtcatgg gagcacagac gtgttccctg     60

attgagtgtg gctgcactcc catatgaata tcctccttag                           100

<210>  38

<211>  99

<212>  DNA

<213>  Artificial Sequence


<220>

<223>  DpfkBF

<400>  38
gcgccctctc tcgatagcgc aacaattacc ccgcaaattt atcccgaagg aaaactgcgc      60

tgtaccgcac cggtgttcgt gtaggctgga gctgcttcg                             99

<210>  39

<211>  26

<212>  DNA

<213>  Artificial Sequence


<220>

<223>  PfkBR

<400>  39
gccggttgca ctttgggtaa gccccg                                           26

<210>  40

<211>  30

<212>  DNA

<213>  Artificial Sequence


<220>

<223>  PfkBF

<400>  40
tggcaggatc atccatgaca gtaaaaacgg                                       30

<210>  41

<211>  29

<212>  DNA

<213>  Artificial Sequence


<220>

<223>  YveDR

<400>  41

cgtgaattct tattcatcaa ttctaataa 29

<210> 42

<211> 32

<212> DNA

<213> Artificial Sequence

<220>

<223> YueDF

<400> 42
acgttcatga gatacgttat cataacagga ac 32